(19) Europäisches Patentamt
European Patent Office
Office européen des brevets

(11) EP 3 919 618 A1

(12) **EUROPEAN PATENT APPLICATION**
published in accordance with Art. 153(4) EPC

(43) Date of publication:
**08.12.2021 Bulletin 2021/49**

(21) Application number: **19913370.3**

(22) Date of filing: **25.06.2019**

(51) Int Cl.:
***C12N 15/113*** *(2010.01)* ***A61K 48/00*** *(2006.01)*
***A61P 35/00*** *(2006.01)*

(86) International application number:
**PCT/CN2019/092720**

(87) International publication number:
**WO 2020/155534 (06.08.2020 Gazette 2020/32)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.01.2019 CN 201910092375**

(71) Applicant: **Ractigen Therapeutics**
**Nantong, Jiangsu 226400 (CN)**

(72) Inventors:
- **KANG, Tao**
**Nantong, Jiangsu 226400 (CN)**
- **LI, Longcheng**
**Nantong, Jiangsu 226400 (CN)**
- **KANG, Moorim**
**Nantong, Jiangsu 226400 (CN)**

(74) Representative: **Høiberg P/S**
**Adelgade 12**
**1304 Copenhagen K (DK)**

# (54) OLIGONUCLEOTIDE MOLECULE AND APPLICATION THEREOF IN TUMOR THERAPY

(57) The present invention relates to oligomeric nucleic acids and uses thereof for the treatment of tumors. The oligomeric nucleic acid for tumor treatment provided by the present application can be small activating nucleic acid molecules. A small activating nucleic acid molecule of the present invention can be a double-stranded or single-stranded RNA molecule targeting the promoter region of an LHPP gene comprising a first nucleic acid strand and a second nucleic acid strand. The double-stranded RNA molecule targeting the promoter region of the LHPP gene comprises two nucleic acid strands of 16 to 35 nucleotides in length, wherein one of the nucleic acid strands has at least 75% homology or complementarity to a target selected from the promoter region of the LHPP gene. The present invention also relates to pharmaceutical compositions comprising the small activating nucleic acids and optional pharmaceutically acceptable carriers, and methods for upregulating the expression of the LHPP gene in a cell and methods for treating diseases or conditions related to insufficient or decreased expression of LHPP gene by using the small activating nucleic acid molecules or the pharmaceutical compositions.

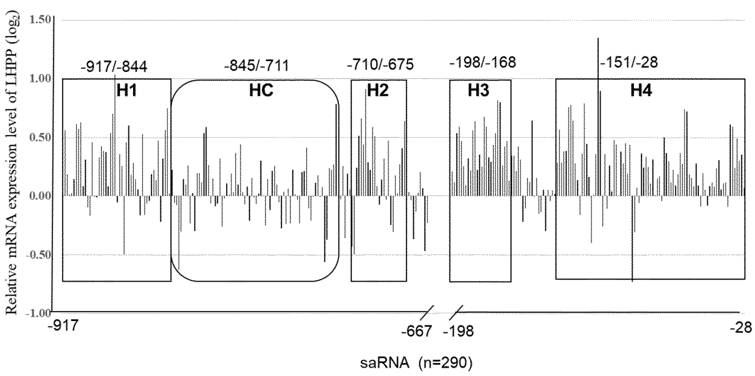


*FIG. 3*

EP 3 919 618 A1

SEQUENCE LISTING

<110> Ractigen Therapeutics

<120> OLIGONUCLEOTIDE MOLECULE AND APPLICATION THEREOF IN TUMOR THERAPY

<130> Docket No.: 065786.11037/5US1

<150> PCT/CN2019/092720
<151> 2019-06-25

<150> CN 201910092375.8
<151> 2019-01-30

<160> 508

<170> PatentIn version 3.5

<210> 1
<211> 21
<212> DNA
<213> Artificial Sequence

<220>
<223> Synthetic Construct

<220>
<221> misc_feature
<222> (20)..(21)
<223> deoxythymidine (dT) base

<400> 1
gcucuuuguc cgcugaucut t 21

<210> 2
<211> 21
<212> DNA
<213> Artificial Sequence

<220>
<223> Synthetic Construct

<220>
<221> misc_feature
<222> (20)..(21)
<223> deoxythymidine (dT) base

<400> 2
uguuggacca gaaguaaagt t 21

<210> 3
<211> 21
<212> DNA
<213> Artificial Sequence

<220>
<223> Synthetic Construct

<220>
<221> misc_feature
<222> (20)..(21)
<223> deoxythymidine (dT) base

<400> 3
agguccuaug cauccucaut t 21

<210> 4
<211> 21
<212> DNA
<213> Artificial Sequence

<220>
<223> Synthetic Construct

<220>
<221> misc_feature
<222> (20)..(21)
<223> deoxythymidine (dT) base

<400> 4
cucuuugucc gcugaucuct t 21

<210> 5
<211> 21
<212> DNA
<213> Artificial Sequence

<220>
<223> Synthetic Construct

<220>
<221> misc_feature
<222> (20)..(21)
<223> deoxythymidine (dT) base

<400> 5
aauuugccuu ugaccuuuct t 21

<210> 6
<211> 21
<212> DNA
<213> Artificial Sequence

<220>
<223> Synthetic Construct

<220>
<221> misc_feature
<222> (20)..(21)
<223> deoxythymidine (dT) base

<400> 6
auuugccuuu gaccuuucut t 21

<210> 7
<211> 21
<212> DNA
<213> Artificial Sequence

<220>
<223> Synthetic Construct

<220>
<221> misc_feature
<222> (20)..(21)
<223> deoxythymidine (dT) base

<400> 7
uuuccugcuc uuuguccgct t 21

<210> 8
<211> 21
<212> DNA
<213> Artificial Sequence

<220>
<223> Synthetic Construct

<220>
<221> misc_feature
<222> (20)..(21)
<223> deoxythymidine (dT) base

<400> 8
uucuucucag cccaggucct t 21

<210> 9
<211> 21
<212> DNA
<213> Artificial Sequence

<220>
<223> Synthetic Construct

<220>
<221> misc_feature
<222> (20)..(21)
<223> deoxythymidine (dT) base

<400> 9
ucugcuuuuc cugcucuuut t 21

<210> 10
<211> 21
<212> DNA
<213> Artificial Sequence

<220>
<223> Synthetic Construct

<220>
<221> misc_feature
<222> (20)..(21)
<223> deoxythymidine (dT) base

<400> 10
uucugcuuuu ccugcucuut t 21

<210> 11
<211> 21
<212> DNA
<213> Artificial Sequence

<220>
<223> Synthetic Construct

<220>
<221> misc_feature
<222> (20)..(21)
<223> deoxythymidine (dT) base

<400> 11
aagguuccga ggggccauut t 21

<210> 12
<211> 21
<212> DNA
<213> Artificial Sequence

<220>
<223> Synthetic Construct

<220>
<221> misc_feature
<222> (20)..(21)
<223> deoxythymidine (dT) base

<400> 12
aaaaggacac cguuucaggt t 21

<210> 13
<211> 21
<212> DNA
<213> Artificial Sequence

<220>
<223> Synthetic Construct

<220>
<221> misc_feature
<222> (20)..(21)
<223> deoxythymidine (dT) base

<400> 13
aaaggacacc guuucagggt t 21

<210> 14
<211> 21
<212> DNA
<213> Artificial Sequence

<220>
<223> Synthetic Construct

<220>
<221> misc_feature
<222> (20)..(21)
<223> deoxythymidine (dT) base

<400> 14
auguuggacc agaaguaaat t 21

<210> 15
<211> 21
<212> DNA
<213> Artificial Sequence

<220>
<223> Synthetic Construct

<220>
<221> misc_feature
<222> (20)..(21)
<223> deoxythymidine (dT) base

<400> 15
ucuucucagc ccagguccut t 21

<210> 16
<211> 21
<212> DNA
<213> Artificial Sequence

<220>
<223> Synthetic Construct

<220>
<221> misc_feature
<222> (20)..(21)
<223> deoxythymidine (dT) base

<400> 16
ugucucaauu ugccuuugat t 21

<210> 17
<211> 21
<212> DNA
<213> Artificial Sequence

<220>
<223> Synthetic Construct

<220>
<221> misc_feature
<222> (20)..(21)
<223> deoxythymidine (dT) base

<400> 17
ccagguccua ugcauccuct t 21

<210> 18
<211> 21
<212> DNA
<213> Artificial Sequence

<220>
<223> Synthetic Construct

<220>
<221> misc_feature
<222> (20)..(21)
<223> deoxythymidine (dT) base

<400> 18
cugcuuuucc ugcucuuugt t 21

<210> 19
<211> 21
<212> DNA
<213> Artificial Sequence

<220>
<223> Synthetic Construct

<220>
<221> misc_feature
<222> (20)..(21)
<223> deoxythymidine (dT) base

<400> 19
uucuuaggga cuuguuuuct t 21

<210> 20
<211> 21
<212> DNA
<213> Artificial Sequence

<220>
<223> Synthetic Construct

<220>
<221> misc_feature
<222> (20)..(21)
<223> deoxythymidine (dT) base

<400> 20
aucuaugcag ggcuguuaut t 21

<210> 21
<211> 21
<212> DNA
<213> Artificial Sequence

<220>
<223> Synthetic Construct

<220>
<221> misc_feature
<222> (20)..(21)
<223> deoxythymidine (dT) base

<400> 21
uucuugucuc aauuugccut t 21

<210> 22
<211> 21
<212> DNA
<213> Artificial Sequence

<220>
<223> Synthetic Construct

<220>
<221> misc_feature
<222> (20)..(21)
<223> deoxythymidine (dT) base

<400> 22
gaaagcucaa agcuauguut t 21

<210> 23
<211> 21
<212> DNA
<213> Artificial Sequence

<220>
<223> Synthetic Construct

<220>
<221> misc_feature
<222> (20)..(21)
<223> deoxythymidine (dT) base

<400> 23
uugaaagcuc aaagcuaugt t 21

<210> 24
<211> 21
<212> DNA
<213> Artificial Sequence

<220>
<223> Synthetic Construct

<220>
<221> misc_feature
<222> (20)..(21)
<223> deoxythymidine (dT) base

<400> 24
gguuugcacc cggucuucut t 21

<210> 25
<211> 21
<212> DNA
<213> Artificial Sequence

<220>
<223> Synthetic Construct

<220>
<221> misc_feature
<222> (20)..(21)
<223> deoxythymidine (dT) base

<400> 25
accagaagua aaguguucut t 21

<210> 26
<211> 21
<212> DNA
<213> Artificial Sequence

<220>
<223> Synthetic Construct

<220>
<221> misc_feature
<222> (20)..(21)
<223> deoxythymidine (dT) base

<400> 26
guuugcaccc ggucuucuut t 21

<210> 27
<211> 21
<212> DNA
<213> Artificial Sequence

<220>
<223> Synthetic Construct

<220>
<221> misc_feature
<222> (20)..(21)
<223> deoxythymidine (dT) base

<400> 27
gucucaauuu gccuuugact t 21

<210> 28
<211> 21
<212> DNA
<213> Artificial Sequence

<220>
<223> Synthetic Construct

<220>
<221> misc_feature
<222> (20)..(21)
<223> deoxythymidine (dT) base

<400> 28
aggugccuuc uugucucaat t 21

<210> 29
<211> 21
<212> DNA
<213> Artificial Sequence

<220>
<223> Synthetic Construct

<220>
<221> misc_feature
<222> (20)..(21)
<223> deoxythymidine (dT) base

<400> 29
uugaggaaac uccucccuut t 21

<210> 30
<211> 21
<212> DNA
<213> Artificial Sequence

<220>
<223> Synthetic Construct

<220>
<221> misc_feature
<222> (20)..(21)
<223> deoxythymidine (dT) base

<400> 30
uccuaugcau ccucaucuat t 21

<210> 31
<211> 21
<212> DNA
<213> Artificial Sequence

<220>
<223> Synthetic Construct

<220>
<221> misc_feature
<222> (20)..(21)
<223> deoxythymidine (dT) base

<400> 31
ugaaagcuca aagcuaugut t 21

<210> 32
<211> 21
<212> DNA
<213> Artificial Sequence

<220>
<223> Synthetic Construct

<220>
<221> misc_feature
<222> (20)..(21)
<223> deoxythymidine (dT) base

<400> 32
uguuuucugc uuuuccugct t 21

<210> 33
<211> 21
<212> DNA
<213> Artificial Sequence

<220>
<223> Synthetic Construct

<220>
<221> misc_feature
<222> (20)..(21)
<223> deoxythymidine (dT) base

<400> 33
cucuucuuug aaagcucaat t 21

<210> 34
<211> 21
<212> DNA
<213> Artificial Sequence

<220>
<223> Synthetic Construct

<220>
<221> misc_feature
<222> (20)..(21)
<223> deoxythymidine (dT) base

<400> 34
aaagguuccg aggggccaut t 21

<210> 35
<211> 21
<212> DNA
<213> Artificial Sequence

<220>
<223> Synthetic Construct

<220>
<221> misc_feature
<222> (20)..(21)
<223> deoxythymidine (dT) base

<400> 35
cuucuugucu caauuugcct t 21

<210> 36
<211> 21
<212> DNA
<213> Artificial Sequence

<220>
<223> Synthetic Construct

<220>
<221> misc_feature
<222> (20)..(21)
<223> deoxythymidine (dT) base

<400> 36
auugaggaaa cuccucccut t 21

<210> 37
<211> 21
<212> DNA
<213> Artificial Sequence

<220>
<223> Synthetic Construct

<220>
<221> misc_feature
<222> (20)..(21)
<223> deoxythymidine (dT) base

<400> 37
uauguuggac cagaaguaat t 21

<210> 38
<211> 21
<212> DNA
<213> Artificial Sequence

<220>
<223> Synthetic Construct

<220>
<221> misc_feature
<222> (20)..(21)
<223> deoxythymidine (dT) base

<400> 38
caggugccuu cuugucucat t 21

<210> 39
<211> 21
<212> DNA
<213> Artificial Sequence

<220>
<223> Synthetic Construct

<220>
<221> misc_feature
<222> (20)..(21)
<223> deoxythymidine (dT) base

<400> 39
caauuugccu uugaccuuut t 21

<210> 40
<211> 21
<212> DNA
<213> Artificial Sequence

<220>
<223> Synthetic Construct

<220>
<221> misc_feature
<222> (20)..(21)
<223> deoxythymidine (dT) base

<400> 40
guaaaguguu cucguuucut t 21

<210> 41
<211> 21
<212> DNA
<213> Artificial Sequence

<220>
<223> Synthetic Construct

<220>
<221> misc_feature
<222> (20)..(21)
<223> deoxythymidine (dT) base

<400> 41
cccagguccu augcauccut t 21

<210> 42
<211> 21
<212> DNA
<213> Artificial Sequence

<220>
<223> Synthetic Construct

<220>
<221> misc_feature
<222> (20)..(21)
<223> deoxythymidine (dT) base

<400> 42
ccuaugcauc cucaucuaut t 21

<210> 43
<211> 21
<212> DNA
<213> Artificial Sequence

<220>
<223> Synthetic Construct

<220>
<221> misc_feature
<222> (20)..(21)
<223> deoxythymidine (dT) base

<400> 43
aagcuuccga aaaggacact t 21

<210> 44
<211> 21
<212> DNA
<213> Artificial Sequence

<220>
<223> Synthetic Construct

<220>
<221> misc_feature
<222> (20)..(21)
<223> deoxythymidine (dT) base

<400> 44
uugcacccgg ucuucuugct t 21

<210> 45
<211> 21
<212> DNA
<213> Artificial Sequence

<220>
<223> Synthetic Construct

<220>
<221> misc_feature
<222> (20)..(21)
<223> deoxythymidine (dT) base

<400> 45
guccgcugau cuccugggat t 21

<210> 46
<211> 21
<212> DNA
<213> Artificial Sequence

<220>
<223> Synthetic Construct

<220>
<221> misc_feature
<222> (20)..(21)
<223> deoxythymidine (dT) base

<400> 46
uugaaagguu ccgaggggct t 21

<210> 47
<211> 21
<212> DNA
<213> Artificial Sequence

<220>
<223> Synthetic Construct

<220>
<221> misc_feature
<222> (20)..(21)
<223> deoxythymine (dT) base

<400> 47
cauccucauc uaugcagggt t 21

<210> 48
<211> 21
<212> DNA
<213> Artificial Sequence

<220>
<223> Synthetic Construct

<220>
<221> misc_feature
<222> (20)..(21)
<223> deoxythymine (dT) base

<400> 48
ggugccuucu ugucucaaut t 21

<210> 49
<211> 21
<212> DNA
<213> Artificial Sequence

<220>
<223> Synthetic Construct

<220>
<221> misc_feature
<222> (20)..(21)
<223> deoxythymine (dT) base

<400> 49
ugccuuugac cuuucuuagt t 21

<210> 50
<211> 21
<212> DNA
<213> Artificial Sequence

<220>
<223> Synthetic Construct

<220>
<221> misc_feature
<222> (20)..(21)
<223> deoxythymine (dT) base

<400> 50
cucaaagcua uguuggacct t 21

<210> 51
<211> 21
<212> DNA
<213> Artificial Sequence

<220>
<223> Synthetic Construct

<220>
<221> misc_feature
<222> (20)..(21)
<223> deoxythymine (dT) base

<400> 51
gaccagaagu aaaguguuct t 21

<210> 52
<211> 21
<212> DNA
<213> Artificial Sequence

<220>
<223> Synthetic Construct

<220>
<221> misc_feature
<222> (20)..(21)
<223> deoxythymine (dT) base

<400> 52
cugaucuccu gggaagaaat t 21

<210> 53
<211> 21
<212> DNA
<213> Artificial Sequence

<220>
<223> Synthetic Construct

<220>
<221> misc_feature
<222> (20)..(21)
<223> deoxythymine (dT) base

<400> 53
uuccugcucu uuguccgcut t 21

<210> 54
<211> 21
<212> DNA
<213> Artificial Sequence

<220>
<223> Synthetic Construct

<220>
<221> misc_feature
<222> (20)..(21)
<223> deoxythymine (dT) base

<400> 54
cagguccuau gcauccucat t 21

<210> 55
<211> 21
<212> DNA
<213> Artificial Sequence

<220>
<223> Synthetic Construct

<220>
<221> misc_feature
<222> (20)..(21)
<223> Synthetic Construct

<400> 55
uccgcugauc uccugggaat t 21

<210> 56
<211> 21
<212> DNA
<213> Artificial Sequence

<220>
<223> Synthetic Construct

<220>
<221> misc_feature
<222> (20)..(21)
<223> deoxythymine (dT) base

<400> 56
uagcaucgag ugguauuuut t 21

<210> 57
<211> 21
<212> DNA
<213> Artificial Sequence

<220>
<223> Synthetic Construct

<220>
<221> misc_feature
<222> (20)..(21)
<223> deoxythymine (dT) base

<400> 57
gaucuccugg gaagaaagct t 21

<210> 58
<211> 21
<212> DNA
<213> Artificial Sequence

<220>
<223> Synthetic Construct

<220>
<221> misc_feature
<222> (20)..(21)
<223> deoxythymine (dT) base

<400> 58
ucaauuugcc uuugaccuut t 21

<210> 59
<211> 21
<212> DNA
<213> Artificial Sequence

<220>
<223> Synthetic Construct

<220>
<221> misc_feature
<222> (20)..(21)
<223> deoxythymine (dT) base

<400> 59
aagcuauguu ggaccagaat t 21

<210> 60
<211> 21
<212> DNA
<213> Artificial Sequence

<220>
<223> Synthetic Construct

<220>
<221> misc_feature
<222> (20)..(21)
<223> deoxythymine (dT) base

<400> 60
uugccuuuga ccuuucuuat t 21

<210> 61
<211> 21
<212> DNA
<213> Artificial Sequence

<220>
<223> Synthetic Construct

<220>
<221> misc_feature
<222> (20)..(21)
<223> deoxythymine (dT) base

<400> 61
uugaccuuuc uuagggacut t 21

<210> 62
<211> 21
<212> DNA
<213> Artificial Sequence

<220>
<223> Synthetic Construct

<220>
<221> misc_feature
<222> (20)..(21)
<223> deoxythymine (dT) base

<400> 62
ugauaguccc guuucuuct t 21

<210> 63
<211> 21
<212> DNA
<213> Artificial Sequence

<220>
<223> deoxythymine (dT) base

<220>
<221> misc_feature
<222> (20)..(21)
<223> deoxythymine (dT) base

<400> 63
caucuaugca gggcuguuat t 21

<210> 64
<211> 21
<212> DNA
<213> Artificial Sequence

<220>
<223> Synthetic Construct

<220>
<221> misc_feature
<222> (20)..(21)
<223> deoxythymine (dT) base

<400> 64
ucucuucuuu gaaagcucat t 21

<210> 65
<211> 21
<212> DNA
<213> Artificial Sequence

<220>
<223> Synthetic Construct

<220>
<221> misc_feature
<222> (20)..(21)
<223> deoxythymine (dT) base

<400> 65
agcuauguug gaccagaagt t 21

<210> 66
<211> 21
<212> DNA
<213> Artificial Sequence

<220>
<223> Synthetic Construct

<220>
<221> misc_feature
<222> (20)..(21)
<223> deoxythymine (dT) base

<400> 66
uuucugcuuu uccugcucut t 21

<210> 67
<211> 21
<212> DNA
<213> Artificial Sequence

<220>
<223> Synthetic Construct

<220>
<221> misc_feature
<222> (20)..(21)
<223> deoxythymine (dT) base

<400> 67
uuuucugcuu uuccugcuct t 21

<210> 68
<211> 21
<212> DNA
<213> Artificial Sequence

<220>
<223> Synthetic Construct

<220>
<221> misc_feature
<222> (20)..(21)
<223> deoxythymine (dT) base

<400> 68
cgcugaucuc cugggaagat t 21

<210> 69
<211> 21
<212> DNA
<213> Artificial Sequence

<220>
<223> Synthetic Construct

<220>
<221> misc_feature
<222> (20)..(21)
<223> deoxythymine (dT) base

<400> 69
gcuauguugg accagaagut t 21

<210> 70
<211> 21
<212> DNA
<213> Artificial Sequence

<220>
<223> Synthetic Construct

<220>
<221> misc_feature
<222> (20)..(21)
<223> deoxythymine (dT) base

<400> 70
uguagcaucg agugguauut t 21

<210> 71
<211> 21
<212> DNA
<213> Artificial Sequence

<220>
<223> Synthetic Construct

<220>
<221> misc_feature
<222> (20)..(21)
<223> deoythymine (dT) base

<400> 71
cgaaaaggac accguuucat t 21

<210> 72
<211> 21
<212> DNA
<213> Artificial Sequence

<220>
<223> Synthetic Construct

<220>
<221> misc_feature
<222> (20)..(21)
<223> deoxythymine (dT) base

<400> 72
agcuuccgaa aaggacacct t 21

<210> 73
<211> 21
<212> DNA
<213> Artificial Sequence

<220>
<223> Synthetic Construct

<220>
<221> misc_feature
<222> (20)..(21)
<223> deoxythymine (dT) base

<400> 73
ccugggaaga aagcuuccgt t 21

<210> 74
<211> 21
<212> DNA
<213> Artificial Sequence

<220>
<223> Synthetic Construct

<220>
<221> misc_feature
<222> (20)..(21)
<223> deoxythymine (dT) base

<400> 74
uuuuccugcu cuuuguccgt t 21

<210> 75
<211> 21
<212> DNA
<213> Artificial Sequence

<220>
<223> Synthetic Construct

<220>
<221> misc_feature
<222> (20)..(21)
<223> deoxythymine (dT) base

<400> 75
ugcucuuugu ccgcugauct t 21

<210> 76
<211> 21
<212> DNA
<213> Artificial Sequence

<220>
<223> Synthetic Construct

<220>
<221> misc_feature
<222> (20)..(21)
<223> deoxythymine (dT) base

<400> 76
uuggaccaga aguaaagugt t 21

<210> 77
<211> 21
<212> DNA
<213> Artificial Sequence

<220>
<223> Synthetic Construct

<220>
<221> misc_feature
<222> (20)..(21)
<223> deoxythymine (dT) base

<400> 77
cuuuguccgc ugaucuccut t 21

<210> 78
<211> 21
<212> DNA
<213> Artificial Sequence

<220>
<223> Synthetic Construct

<220>
<221> misc_feature
<222> (20)..(21)
<223> deoxythymine (dT) base

<400> 78
cuugucucaa uuugccuuut t 21

<210> 79
<211> 21
<212> DNA
<213> Artificial Sequence

<220>
<223> Synthetic Construct

<220>
<221> misc_feature
<222> (20)..(21)
<223> deoxythymine (dT) base

<400> 79
cacccggucu ucuugcccut t 21

<210> 80
<211> 21
<212> DNA
<213> Artificial Sequence

<220>
<223> Synthetic Construct

<220>
<221> misc_feature
<222> (20)..(21)
<223> deoxythymine (dT) base

<400> 80
cuuugaccuu ucuuagggat t 21

<210> 81
<211> 21
<212> DNA
<213> Artificial Sequence

<220>
<223> Synthetic Construct

<220>
<221> misc_feature
<222> (20)..(21)
<223> deoxythymine (dT) base

<400> 81
ccuuugaccu uucuuagggt t 21

<210> 82
<211> 21
<212> DNA
<213> Artificial Sequence

<220>
<223> Synthetic Construct

<220>
<221> misc_feature
<222> (20)..(21)
<223> deoxythymine (dT) base

<400> 82
ugggaagaaa gcuuccgaat t 21

<210> 83
<211> 21
<212> DNA
<213> Artificial Sequence

<220>
<223> Synthetic Construct

<220>
<221> misc_feature
<222> (20)..(21)
<223> deoxythymine (dT) base

<400> 83
ucuaugcagg gcuguuauct t 21

<210>  84
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  84
aaagcuaugu uggaccagat t    21

<210>  85
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  85
ucucaauuug ccuuugacct t    21

<210>  86
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  86
ugcauccuca ucuaugcagt t    21

<210>  87
<211>  21
<212>  DNA
<213>  Artificial Sequence

<220>
<223>  Synthetic Construct

<220>
<221> misc_feature
<222> (20)..(21)
<223> deoxythymine (dT) base

<400> 87
gaaagguucc gaggggccat t 21

<210> 88
<211> 21
<212> DNA
<213> Artificial Sequence

<220>
<223> Synthetic Construct

<220>
<221> misc_feature
<222> (20)..(21)
<223> deoxythymine (dT) base

<400> 88
gccuucuugu cucaauuugt t 21

<210> 89
<211> 21
<212> DNA
<213> Artificial Sequence

<220>
<223> Synthetic Construct

<220>
<221> misc_feature
<222> (20)..(21)
<223> deoxythymine (dT) base

<400> 89
acuccucccu uuuaauauct t 21

<210> 90
<211> 21
<212> DNA
<213> Artificial Sequence

<220>
<223> Synthetic Construct

<220>
<221> misc_feature
<222> (20)..(21)
<223> deoxythymine (dT) base

<400> 90
gaagaaagcu uccgaaaagt t 21

<210> 91
<211> 21
<212> DNA
<213> Artificial Sequence

<220>
<223> Synthetic Construct

<220>
<221> misc_feature
<222> (20)..(21)
<223> deoxythymine (dT) base

<400> 91
ugaccuuucu uagggacuut t 21

<210> 92
<211> 21
<212> DNA
<213> Artificial Sequence

<220>
<223> Synthetic Construct

<220>
<221> misc_feature
<222> (20)..(21)
<223> deoxythymine (dT) base

<400> 92
aagcucaaag cuauguuggt t 21

<210> 93
<211> 21
<212> DNA
<213> Artificial Sequence

<220>
<223> Synthetic Construct

<220>
<221> misc_feature
<222> (20)..(21)
<223> deoxythymine (dT) base

<400> 93
caguuccggg uuugcaccct t 21

<210> 94
<211> 21
<212> DNA
<213> Artificial Sequence

<220>
<223> Synthetic Construct

<220>
<221> misc_feature
<222> (20)..(21)
<223> deoxythymine (dT) base

<400> 94
ugcacccggu cuucuugcct t 21

<210> 95
<211> 21
<212> DNA
<213> Artificial Sequence

<220>
<223> Synthetic Construct

<220>
<221> misc_feature
<222> (20)..(21)
<223> deoxythymine (dT) base

<400> 95
ccuccugggu uuaacgcaut t 21

<210> 96
<211> 21
<212> DNA
<213> Artificial Sequence

<220>
<223> Synthetic Construct

<220>
<221> misc_feature
<222> (20)..(21)
<223> deoxythymine (dT) base

<400> 96
gcuuccgaaa aggacaccgt t 21

<210> 97
<211> 21
<212> DNA
<213> Artificial Sequence

<220>
<223> Synthetic Construct

<220>
<221> misc_feature
<222> (20)..(21)
<223> deoxythymine (dT) base

<400> 97
cucaauuugc cuuugaccut t 21

<210> 98
<211> 21
<212> DNA
<213> Artificial Sequence

<220>
<223> Synthetic Construct

<220>
<221> misc_feature
<222> (20)..(21)
<223> deoxythymine (dT) base

<400> 98
gguccuaugc auccucauct t 21

<210> 99
<211> 21
<212> DNA
<213> Artificial Sequence

<220>
<223> Synthetic Construct

<220>
<221> misc_feature
<222> (20)..(21)
<223> deoxythymine (dT) base

<400> 99
guuucugcu uuuccugcut t 21

<210> 100
<211> 21
<212> DNA
<213> Artificial Sequence

<220>
<223> Synthetic Construct

<220>
<221> misc_feature
<222> (20)..(21)
<223> deoxythymine (dT) base

<400> 100
uuguuuucug cuuuuccugt t 21

<210> 101
<211> 21
<212> DNA
<213> Artificial Sequence

<220>
<223> Synthetic Construct

<220>
<221> misc_feature
<222> (20)..(21)
<223> deoxythymine (dT) base

<400> 101
cagaaguaaa guguucucgt t 21

<210> 102
<211> 21
<212> DNA
<213> Artificial Sequence

<220>
<223> Synthetic Construct

<220>
<221> misc_feature
<222> (20)..(21)
<223> deoxythymine (dT) base

<400> 102
ugcuuuuccu gcucuuugut t 21

<210> 103
<211> 21
<212> DNA
<213> Artificial Sequence

<220>
<223> Synthetic Construct

<220>
<221> misc_feature
<222> (20)..(21)
<223> deoxythymine (dT) base

<400> 103
gcugaucucc ugggaagaat t 21

<210> 104
<211> 21
<212> DNA
<213> Artificial Sequence

<220>
<223> Synthetic Construct

<220>
<221> misc_feature
<222> (20)..(21)
<223> deoxythymine (dT) base

<400> 104
acaccguuuc aggggcgagt t 21

<210> 105
<211> 21
<212> DNA
<213> Artificial Sequence

<220>
<223> Artificial Sequence

<220>
<221> misc_feature
<222> (20)..(21)
<223> deoxythymine (dT) base

<400> 105
gaaaaggaca ccguuucagt t 21

<210> 106
<211> 21
<212> DNA
<213> Artificial Sequence

<220>
<223> Synthtetic Construct

<220>
<221> misc_feature
<222> (20)..(21)
<223> deoxythymine (dT) base

<400> 106
uuucuucuca gcccagguct t 21

<210> 107
<211> 21
<212> DNA
<213> Artificial Sequence

<220>
<223> Synthetic Construct

<220>
<221> misc_feature
<222> (20)..(21)
<223> deoxythymine (dT) base

<400> 107
ucaucuaugc agggcuguut t 21

<210> 108
<211> 21
<212> DNA
<213> Artificial Sequence

<220>
<223> Synthetic Construct

<220>
<221> misc_feature
<222> (20)..(21)
<223> deoxythymine (dT) base

<400> 108
ugaggaaacu ccucccuuut t 21

<210> 109
<211> 21
<212> DNA
<213> Artificial Sequence

<220>
<223> Synthetic Construct

<220>
<221> misc_feature
<222> (20)..(21)
<223> deoxythymine (dT) base

<400> 109
aggggccauu gaggaaacut t 21

<210> 110
<211> 21
<212> DNA
<213> Artificial Sequence

<220>
<223> Synthetic Construct

<220>
<221> misc_feature
<222> (20)..(21)
<223> deoxythymine (dT) base

<400> 110
uuugccuuug accuuucuut t 21

<210> 111
<211> 21
<212> DNA
<213> Artificial Sequence

<220>
<223> Synthetic Construct

<220>
<221> misc_feature
<222> (20)..(21)
<223> deoxythymine (dT) base

<400> 111
uuguccgcug aucuccuggt t 21

<210> 112
<211> 21
<212> DNA
<213> Artificial Sequence

<220>
<223> Synthetic Construct

<220>
<221> misc_feature
<222> (20)..(21)
<223> deoxythymine (dT) base

<400> 112
uucucagccc agguccuaut t 21

<210> 113
<211> 21
<212> DNA
<213> Artificial Sequence

<220>
<223> Synthetic Construct

<220>
<221> misc_feature
<222> (20)..(21)
<223> deoxythymine (dT) base

<400> 113
gugccuucuu gucucaauut t 21

<210> 114
<211> 21
<212> DNA
<213> Artificial Sequence

<220>
<223> Synthetic Construct

<220>
<221> misc_feature
<222> (20)..(21)
<223> deoxythymidine (dT) base

<400> 114
ggguuuaacg cauugugaut t 21

<210> 115
<211> 21
<212> DNA
<213> Artificial Sequence

<220>
<223> Synthetic Construct

<220>
<221> misc_feature
<222> (20)..(21)
<223> deoxythymine (dT) base

<400> 115
uggaccagaa guaaagugut t 21

<210> 116
<211> 21
<212> DNA
<213> Artificial Sequence

<220>
<223> Synthetic Construct

<220>
<221> misc_feature
<222> (20)..(21)
<223> deoxythymine (dT) base

<400> 116
uugucucaau uugccuuugt t 21

<210> 117
<211> 21
<212> DNA
<213> Artificial Sequence

<220>
<223> Synthetic Construct

<220>
<221> misc_feature
<222> (20)..(21)
<223> deoxythymine (dT) base

<400> 117
gggaagaaag cuuccgaaat t 21

<210> 118
<211> 21
<212> DNA
<213> Artificial Sequence

<220>
<223> Synthetic Construct

<220>
<221> misc_feature
<222> (20)..(21)
<223> deoxythymine (dT) base

<400> 118
gcccaggucc uaugcaucct t 21

<210> 119
<211> 21
<212> DNA
<213> Artificial Sequence

<220>
<223> Synthetic Construct

<220>
<221> misc_feature
<222> (20)..(21)
<223> deoxythymine (dT) base

<400> 119
uuugcacccg gucuucuugt t 21

<210> 120
<211> 21
<212> DNA
<213> Artificial Sequence

<220>
<223> Synthetic Construct

<220>
<221> misc_feature
<222> (20)..(21)
<223> Synthetic Construct

<400> 120
cugggaagaa agcuuccgat t 21

<210> 121
<211> 21
<212> DNA
<213> Artificial Sequence

<220>
<223> Synthetic Construct

<220>
<221> misc_feature
<222> (20)..(21)
<223> Synthetic Construct

<400> 121
ccaguuccgg guuugcacct t 21

<210> 122
<211> 21
<212> DNA
<213> Artificial Sequence

<220>
<223> Synthetic Construct

<220>
<221> misc_feature
<222> (20)..(21)
<223> deoxythymine (dT) base

<400> 122
guuuucuucu cagcccaggt t 21

<210> 123
<211> 21
<212> DNA
<213> Artificial Sequence

<220>
<223> Synthetic Construct

<220>
<221> misc_feature
<222> (20)..(21)
<223> deoxythymine (dT) base

<400> 123
ucuugucuca auuugccuut t 21

<210> 124
<211> 21
<212> DNA
<213> Artificial Sequence

<220>
<223> Synthetic Construct

<220>
<221> misc_feature
<222> (20)..(21)
<223> deoxythymine (dT) base

<400> 124
guccuaugca uccucaucut t 21

<210> 125
<211> 21
<212> DNA
<213> Artificial Sequence

<220>
<223> Synthetic Construct

<220>
<221> misc_feature
<222> (20)..(21)
<223> deoxythymine (dT) base

<400> 125
uuccgaaaag gacaccguut t 21

<210> 126
<211> 21
<212> DNA
<213> Artificial Sequence

<220>
<223> Synthetic Construct

<220>
<221> misc_feature
<222> (20)..(21)
<223> deoxythymine (dT) base

<400> 126
uuuucuucuc agcccaggut t 21

<210> 127
<211> 21
<212> DNA
<213> Artificial Sequence

<220>
<223> Synthetic Construct

<220>
<221> misc_feature
<222> (20)..(21)
<223> deoxythymine (dT) base

<400> 127
gcauugugau agucccguut t 21

<210> 128
<211> 21
<212> DNA
<213> Artificial Sequence

<220>
<223> Synthetic Construct

<220>
<221> misc_feature
<222> (20)..(21)
<223> deoxythymine (dT) base

<400> 128
acuugaaagg uuccgagggt t 21

<210> 129
<211> 21
<212> DNA
<213> Artificial Sequence

<220>
<223> Synthetic Construct

<220>
<221> misc_feature
<222> (20)..(21)
<223> deoxythymine (dT) base

<400> 129
gguuccgagg ggccauugat t 21

<210> 130
<211> 21
<212> DNA
<213> Artificial Sequence

<220>
<223> Synthetic Construct

<220>
<221> misc_feature
<222> (20)..(21)
<223> deoxythymine (dT) base

<400> 130
ccuucuuguc ucaauuugct t 21

<210> 131
<211> 21
<212> DNA
<213> Artificial Sequence

<220>
<223> Synthetic Construct

<220>
<221> misc_feature
<222> (20)..(21)
<223> deoxythymine (dT) base

<400> 131
uggguuuaac gcauugugat t 21

<210> 132
<211> 21
<212> DNA
<213> Artificial Sequence

<220>
<223> Synthetic Construct

<220>
<221> misc_feature
<222> (20)..(21)
<223> deoxythymine (dT) base

<400> 132
uuugaccuuu cuuagggact t 21

<210> 133
<211> 21
<212> DNA
<213> Artificial Sequence

<220>
<223> Synthetic Construct

<220>
<221> misc_feature
<222> (20)..(21)
<223> deoxythymine (dT) base

<400> 133
gugauagucc cguuuucuut t 21

<210> 134
<211> 21
<212> DNA
<213> Artificial Sequence

<220>
<223> Synthetic Construct

<220>
<221> misc_feature
<222> (20)..(21)
<223> deoxythymine (dT) base

<400> 134
uacaggugcc uucuugucut t 21

<210> 135
<211> 21
<212> DNA
<213> Artificial Sequence

<220>
<223> deoxythymine (dT) base

<400> 135
ugugauaguc ccguuuucut t 21

<210> 136
<211> 21
<212> DNA
<213> Artificial Sequence

<220>
<223> Synthetic Construct

<220>
<221> misc_feature
<222> (20)..(21)
<223> deoxythymine (dT) base

<400> 136

cagggcuguu aucugcauat t 21

<210> 137
<211> 21
<212> DNA
<213> Artificial Sequence

<220>
<223> Synthetic Construct

<220>
<221> misc_feature
<222> (20)..(21)
<223> deoxythymine (dT) base

<400> 137
ccguuucagg ggcgagugat t 21

<210> 138
<211> 21
<212> DNA
<213> Artificial Sequence

<220>
<223> Synthetic Construct

<220>
<221> misc_feature
<222> (20)..(21)
<223> deoxythymine (dT) base

<400> 138
gccauugagg aaacuccuct t 21

<210> 139
<211> 21
<212> DNA
<213> Artificial Sequence

<220>
<223> Synthetic Construct

<220>
<221> misc_feature
<222> (20)..(21)
<223> deoxythymine (dT) base

<400> 139
ccauugagga aacuccucct t 21

<210> 140
<211> 21
<212> DNA
<213> Artificial Sequence

<220>

<223> Synthetic Construct

<220>
<221> misc_feature
<222> (20)..(21)
<223> deoxythymine (dT) base

<400> 140
cucagcccag guccuaugct t 21

<210> 141
<211> 21
<212> DNA
<213> Artificial Sequence

<220>
<223> Synthetic Construct

<220>
<221> misc_feature
<222> (20)..(21)
<223> Synthetic Construct

<220>
<221> misc_feature
<222> (20)..(21)
<223> deoxythymine (dT) base

<400> 141
ugaucuccug ggaagaaagt t 21

<210> 142
<211> 21
<212> DNA
<213> Artificial Sequence

<220>
<223> Synthetic Construct

<220>
<221> misc_feature
<222> (20)..(21)
<223> deoxythymine (dT) base

<400> 142
aauguagcau cgagugguat t 21

<210> 143
<211> 21
<212> DNA
<213> Artificial Sequence

<220>
<223> Synthetic Construct

<220>

<221> misc_feature
<222> (20)..(21)
<223> deoxythymine (dT) base

<400> 143
cuucuuugaa agcucaaagt t 21

<210> 144
<211> 21
<212> DNA
<213> Artificial Sequence

<220>
<223> Synthetic Construct

<220>
<221> misc_feature
<222> (20)..(21)
<223> deoxythymine (dT) base

<400> 144
aaggacaccg uuucaggggt t 21

<210> 145
<211> 21
<212> DNA
<213> Artificial Sequence

<220>
<223> Synthetic Construct

<220>
<221> misc_feature
<222> (20)..(21)
<223> deoxythymine (dT) base

<400> 145
ccgaaaagga caccguuuct t 21

<210> 146
<211> 21
<212> DNA
<213> Artificial Sequence

<220>
<223> Synthetic Construct

<220>
<221> misc_feature
<222> (20)..(21)
<223> deoxythymine (dT) base

<400> 146
aacuugaaag guuccgaggt t 21

<210> 147

<211> 21
<212> DNA
<213> Artificial Sequence

<220>
<223> deoxythymine (dT) base

<400> 147
ccagaaguaa aguguucuct t 21

<210> 148
<211> 21
<212> DNA
<213> Artificial Sequence

<220>
<223> Synthetic Construct

<220>
<221> misc_feature
<222> (20)..(21)
<223> deoxythymine (dT) base

<400> 148
gucccguuuu cuucucagct t 21

<210> 149
<211> 21
<212> DNA
<213> Artificial Sequence

<220>
<223> Synthetic Construct

<220>
<221> misc_feature
<222> (20)..(21)
<223> deoxythymine (dT) base

<400> 149
ccucaucuau gcagggcugt t 21

<210> 150
<211> 21
<212> DNA
<213> Artificial Sequence

<220>
<223> Synthetic Construct

<220>
<221> misc_feature
<222> (20)..(21)
<223> deoxythymine (dT) base

<400> 150
gaaagcuucc gaaaaggact t 21

<210> 151
<211> 21
<212> DNA
<213> Artificial Sequence

<220>
<223> Synthetic Construct

<220>
<221> misc_feature
<222> (20)..(21)
<223> deoxythymine (dT) base

<400> 151
uccugcucuu uguccgcugt t 21

<210> 152
<211> 21
<212> DNA
<213> Artificial Sequence

<220>
<223> Synthetic Construct

<220>
<221> misc_feature
<222> (20)..(21)
<223> deoxythymine (dT) base

<400> 152
ccuuucuuag ggacuuguut t 21

<210> 153
<211> 21
<212> DNA
<213> Artificial Sequence

<220>
<223> deoxythymine (dT) base

<220>
<221> misc_feature
<222> (20)..(21)
<223> deoxythymine (dT) base

<400> 153
cuuagggacu uguuuucugt t 21

<210> 154
<211> 21
<212> DNA
<213> Artificial Sequence

<220>
<223> Synthetic Construct

<220>
<221> misc_feature
<222> (20)..(21)
<223> deoxythymine (dT) base

<400> 154
aaaaccuccu ggguuuaact t 21

<210> 155
<211> 21
<212> DNA
<213> Artificial Sequence

<220>
<223> Synthetic Construct

<220>
<221> misc_feature
<222> (20)..(21)
<223> Synthetic Construct

<220>
<221> misc_feature
<222> (20)..(21)
<223> deoxythymine (dT) base

<400> 155
aggacaccgu uucaggggct t 21

<210> 156
<211> 21
<212> DNA
<213> Artificial Sequence

<220>
<223> Synthetic Construct

<220>
<221> misc_feature
<222> (20)..(21)
<223> Synthetic Construct

<400> 156
agaaagcuuc cgaaaaggat t 21

<210> 157
<211> 21
<212> DNA
<213> Artificial Sequence

<220>
<223> Synthetic Construct

<220>
<221> misc_feature

<222> (20)..(21)
<223> deoxythymine (dT) base

<400> 157
aggaaacucc ucccuuuuat t 21

<210> 158
<211> 21
<212> DNA
<213> Artificial Sequence

<220>
<223> deoxythymine (dT) base

<400> 158
cgaggggcca uugaggaaat t 21

<210> 159
<211> 21
<212> DNA
<213> Artificial Sequence

<220>
<223> Synthetic Construct

<220>
<221> misc_feature
<222> (20)..(21)
<223> deoxythymine (dT) base

<400> 159
ccuggguuua acgcauugut t 21

<210> 160
<211> 21
<212> DNA
<213> Artificial Sequence

<220>
<223> Synthetic Construct

<220>
<221> misc_feature
<222> (20)..(21)
<223> deoxythymine (dT) base

<400> 160
cuuugaaagc ucaaagcuat t 21

<210> 161
<211> 21
<212> DNA
<213> Artificial Sequence

<220>
<223> Synthetic Construct

<220>
<221> misc_feature
<222> (20)..(21)
<223> deoxythymine (dT) base

<400> 161
agaaguaaag uguucucgut t 21

<210> 162
<211> 21
<212> DNA
<213> Artificial Sequence

<220>
<223> Synthetic Construct

<220>
<221> misc_feature
<222> (20)..(21)
<223> deoxythymine (dT) base

<400> 162
augcauccuc aucuaugcat t 21

<210> 163
<211> 21
<212> DNA
<213> Artificial Sequence

<220>
<223> Synthetic Construct

<220>
<221> misc_feature
<222> (20)..(21)
<223> deoxythymine (dT) base

<400> 163
cuugaaaggu uccgaggggt t 21

<210> 164
<211> 21
<212> DNA
<213> Artificial Sequence

<220>
<223> Synthetic Construct

<220>
<221> misc_feature
<222> (20)..(21)
<223> deoxythymine (dT) base

<400> 164
gcuuuuccug cucuuuguct t 21

<210> 165
<211> 21
<212> DNA
<213> Artificial Sequence

<220>
<223> Synthetic Construct

<220>
<221> misc_feature
<222> (20)..(21)
<223> deoxythymine (dT) base

<400> 165
agaucagcgg acaaagagct t 21

<210> 166
<211> 21
<212> DNA
<213> Artificial Sequence

<220>
<223> Synthetic Construct

<220>
<221> misc_feature
<222> (20)..(21)
<223> deoxythymine (dT) base

<400> 166
cuuuacuucu gguccaacat t 21

<210> 167
<211> 21
<212> DNA
<213> Artificial Sequence

<220>
<223> Synthetic Construct

<220>
<221> misc_feature
<222> (20)..(21)
<223> deoxythymine (dT) base

<400> 167
augaggaugc auaggaccut t 21

<210> 168
<211> 21
<212> DNA
<213> Artificial Sequence

<220>
<223> Synthetic Construct

<400> 168
gagaucagcg gacaaagagt t 21

<210> 169
<211> 21
<212> DNA
<213> Artificial Sequence

<220>
<223> Synthetic Construct

<220>
<221> misc_feature
<222> (20)..(21)
<223> deoxythymine (dT) base

<400> 169
gaaaggucaa aggcaaauut t 21

<210> 170
<211> 21
<212> DNA
<213> Artificial Sequence

<220>
<223> Synthetic Construct

<220>
<221> misc_feature
<222> (20)..(21)
<223> deoxythymine (dT) base

<400> 170
agaaagguca aaggcaaaut t 21

<210> 171
<211> 21
<212> DNA
<213> Artificial Sequence

<220>
<223> Synthetic Construct

<220>
<221> misc_feature
<222> (20)..(21)
<223> deoxythymine (dT) base

<400> 171
gcggacaaag agcaggaaat t 21

<210> 172
<211> 21
<212> DNA
<213> Artificial Sequence

<220>
<223> Synthetic Construct

<220>
<221> misc_feature
<222> (20)..(21)
<223> deoxythymine (dT) base

<400> 172
ggaccugggc ugagaagaat t 21

<210> 173
<211> 21
<212> DNA
<213> Artificial Sequence

<220>
<223> Synthetic Construct

<220>
<221> misc_feature
<222> (20)..(21)
<223> deoxythymine (dT) base

<400> 173
aaagagcagg aaaagcagat t 21

<210> 174
<211> 21
<212> DNA
<213> Artificial Sequence

<220>
<223> Synthetic Construct

<220>
<221> misc_feature
<222> (20)..(21)
<223> deoxythymine (dT) base

<400> 174
aagagcagga aaagcagaat t 21

<210> 175
<211> 21
<212> DNA
<213> Artificial Sequence

<220>
<223> Synthetic Construct

<220>
<221> misc_feature
<222> (20)..(21)
<223> deoxythymine (dT) base

<400> 175
aauggccccu cggaaccuut t 21

<210> 176
<211> 21
<212> DNA
<213> Artificial Sequence

<220>
<223> Synthetic Construct

<220>
<221> misc_feature
<222> (20)..(21)
<223> deoxythymine (dT) base

<400> 176
ccugaaacgg uguccuuuut t 21

<210> 177
<211> 21
<212> DNA
<213> Artificial Sequence

<220>
<223> Synthetic Construct

<220>
<221> misc_feature
<222> (20)..(21)
<223> deoxythymine (dT) base

<400> 177
cccugaaacg guguccuuut t 21

<210> 178
<211> 21
<212> DNA
<213> Artificial Sequence

<220>
<223> Synthetic Construct

<220>
<221> misc_feature
<222> (20)..(21)
<223> deoxythymine (dT) base

<400> 178
uuuacuucug guccaacaut t 21

<210> 179
<211> 21
<212> DNA
<213> Artificial Sequence

<220>
<223> Synthetic Construct

<220>
<221> misc_feature
<222> (20)..(21)
<223> deoxythymine (dT) base

<400> 179
aggaccuggg cugagaagat t 21

<210> 180
<211> 21
<212> DNA
<213> Artificial Sequence

<220>
<223> Synthetic Construct

<220>
<221> misc_feature
<222> (20)..(21)
<223> deoxythymine (dT) base

<400> 180
ucaaaggcaa auugagacat t 21

<210> 181
<211> 21
<212> DNA
<213> Artificial Sequence

<220>
<223> Synthetic Construct

<220>
<221> misc_feature
<222> (20)..(21)
<223> deoxythymine (dT) base

<400> 181
gaggaugcau aggaccuggt t 21

<210> 182
<211> 21
<212> DNA
<213> Artificial Sequence

<220>
<223> Synthetic Construct

<220>
<221> misc_feature
<222> (20)..(21)
<223> deoxythymine (dT) base

<400> 182
caaagagcag gaaaagcagt t 21

<210> 183
<211> 21
<212> DNA
<213> Artificial Sequence

<220>
<223> Synthetic Construct

<220>
<221> misc_feature
<222> (20)..(21)
<223> deoxythymine (dT) base

<400> 183
gaaaacaagu cccuaagaat t 21

<210> 184
<211> 21
<212> DNA
<213> Artificial Sequence

<220>
<223> Synthetic Construct

<220>
<221> misc_feature
<222> (20)..(21)
<223> deoxythymine (dT) base

<400> 184
auaacagccc ugcauagaut t 21

<210> 185
<211> 21
<212> DNA
<213> Artificial Sequence

<220>
<223> Synthetic Construct

<220>
<221> misc_feature
<222> (20)..(21)
<223> deoxythymine (dT) base

<400> 185
aggcaaauug agacaagaat t 21

<210> 186
<211> 21
<212> DNA
<213> Artificial Sequence

<220>
<223> Synthetic Construct

<220>
<221> misc_feature
<222> (20)..(21)
<223> deoxythymine (dT) base

<400> 186
aacauagcuu ugagcuuuct t 21

<210> 187
<211> 21
<212> DNA
<213> Artificial Sequence

<220>
<223> Synthetic Construct

<220>
<221> misc_feature
<222> (20)..(21)
<223> deoxythymine (dT) base

<400> 187
cauagcuuug agcuuucaat t 21

<210> 188
<211> 21
<212> DNA
<213> Artificial Sequence

<220>
<223> Synthetic Construct

<220>
<221> misc_feature
<222> (20)..(21)
<223> deoxythymine (dT) base

<400> 188
agaagaccgg gugcaaacct t 21

<210> 189
<211> 21
<212> DNA
<213> Artificial Sequence

<220>
<223> Synthetic Construct

<220>
<221> misc_feature
<222> (20)..(21)
<223> deoxythymine (dT) base

<400> 189
agaacacuuu acuucuggut t 21

<210> 190
<211> 21
<212> DNA
<213> Artificial Sequence

<220>
<223> Synthetic Construct

<220>
<221> misc_feature
<222> (20)..(21)
<223> deoxythymine (dT) base

<400> 190
aagaagaccg ggugcaaact t 21

<210> 191
<211> 21
<212> DNA
<213> Artificial Sequence

<220>
<223> Synthetic Construct

<220>
<221> misc_feature
<222> (20)..(21)
<223> deoxythymine (dT) base

<400> 191
gucaaaggca aauugagact t 21

<210> 192
<211> 21
<212> DNA
<213> Artificial Sequence

<220>
<223> Synthetic Construct

<220>
<221> misc_feature
<222> (20)..(21)
<223> deoxythymine (dT) base

<400> 192
uugagacaag aaggcaccut t 21

<210> 193
<211> 21
<212> DNA
<213> Artificial Sequence

<220>
<223> Synthetic Construct

<220>
<221> misc_feature
<222> (20)..(21)
<223> deoxythymine (dT) base

<400> 193
aagggaggag uuuccucaat t 21

<210> 194
<211> 21
<212> DNA
<213> Artificial Sequence

<220>
<223> Synthetic Construct

<220>
<221> misc_feature
<222> (20)..(21)
<223> deoxythymine (dT) base

<400> 194
uagaugagga ugcauaggat t 21

<210> 195
<211> 21
<212> DNA
<213> Artificial Sequence

<220>
<223> Synthetic Construct

<220>
<221> misc_feature
<222> (20)..(21)
<223> deoxythymine (dT) base

<400> 195
acauagcuuu gagcuuucat t 21

<210> 196
<211> 21
<212> DNA
<213> Artificial Sequence

<220>
<223> Synthetic Construct

<220>
<221> misc_feature
<222> (20)..(21)
<223> deoxythymine (dT) base

<400> 196
gcaggaaaag cagaaaacat t 21

<210> 197
<211> 21
<212> DNA
<213> Artificial Sequence

<220>
<223> deoxythymine (dT) base

<220>
<221> misc_feature
<222> (20)..(21)
<223> deoxythymine (dT) base

<400> 197
uugagcuuuc aaagaagagt t 21

<210> 198
<211> 21
<212> DNA
<213> Artificial Sequence

<220>
<223> Synthetic Construct

<220>
<221> misc_feature
<222> (20)..(21)
<223> deoxythymine (dT) base

<400> 198
auggccccuc ggaaccuuut t 21

<210> 199
<211> 21
<212> DNA
<213> Artificial Sequence

<220>
<223> Synthetic Construct

<220>
<221> misc_feature
<222> (20)..(21)
<223> deoxythymine (dT) base

<400> 199
ggcaaauuga gacaagaagt t 21

<210> 200
<211> 21
<212> DNA
<213> Artificial Sequence

<220>
<223> Synthetic Construct

<220>
<221> misc_feature
<222> (20)..(21)
<223> deoxythymine (dT) base

<400> 200
agggaggagu uuccucaaut t 21

<210> 201
<211> 21
<212> DNA
<213> Artificial Sequence

<220>
<223> Synthetic Construct

<220>
<221> misc_feature
<222> (20)..(21)
<223> deoxythymine (dT) base

<400> 201
uuacuucugg uccaacauat t 21

<210> 202
<211> 21
<212> DNA
<213> Artificial Sequence

<220>
<223> Synthetic Construct

<220>
<221> misc_feature
<222> (20)..(21)
<223> deoxythymine (dT) base

<400> 202
ugagacaaga aggcaccugt t 21

<210> 203
<211> 21
<212> DNA
<213> Artificial Sequence

<220>
<223> Synthetic Construct

<220>
<221> misc_feature
<222> (20)..(21)
<223> deoxythymine (dT) base

<400> 203
aaaggucaaa ggcaaauugt t 21

<210> 204
<211> 21
<212> DNA
<213> Artificial Sequence

<220>
<223> Synthetic Construct

<220>
<221> misc_feature
<222> (20)..(21)
<223> deoxythymine (dT) base

<400> 204
agaaacgaga acacuuuact t 21

<210> 205
<211> 21
<212> DNA
<213> Artificial Sequence

<220>
<223> Synthetic Construct

<220>
<221> misc_feature
<222> (20)..(21)
<223> deoxythymine (dT) base

<400> 205
aggaugcaua ggaccugggt t 21

<210> 206
<211> 21
<212> DNA
<213> Artificial Sequence

<220>
<223> Synthetic Construct

<220>
<221> misc_feature
<222> (20)..(21)
<223> deoxythymine (dT) base

<400> 206
auagaugagg augcauaggt t 21

<210> 207
<211> 21
<212> DNA
<213> Artificial Sequence

<220>
<223> Synthetic Construct

<220>
<221> misc_feature
<222> (20)..(21)
<223> deoxythymine (dT) base

<400> 207
guguccuuuu cggaagcuut t 21

<210> 208
<211> 21
<212> DNA
<213> Artificial Sequence

<220>
<223> Synthetic Construct

<220>
<221> misc_feature
<222> (20)..(21)
<223> deoxythymine (dT) base

<400> 208
gcaagaagac cgggugcaat t 21

<210> 209
<211> 21
<212> DNA
<213> Artificial Sequence

<220>
<223> Synthetic Construct

<220>
<221> misc_feature
<222> (20)..(21)
<223> deoxythymine (dT) base

<400> 209
ucccaggaga ucagcggact t 21

<210> 210
<211> 21
<212> DNA
<213> Artificial Sequence

<220>
<223> Synthetic Construct

<220>
<221> misc_feature
<222> (20)..(21)
<223> deoxythymine (dT) base

<400> 210
gccccucgga accuuucaat t 21

<210> 211
<211> 21
<212> DNA
<213> Artificial Sequence

<220>
<223> Synthetic Construct

<220>
<221> misc_feature
<222> (20)..(21)
<223> deoxythymine (dT) base

<400> 211
cccugcauag augaggaugt t 21

<210> 212
<211> 21
<212> DNA
<213> Artificial Sequence

<220>
<223> Synthetic Construct

<220>
<221> misc_feature
<222> (20)..(21)
<223> deoxythymine (dT) base

<400> 212
auugagacaa gaaggcacct t 21

<210> 213
<211> 21
<212> DNA
<213> Artificial Sequence

<220>
<223> Synthetic Construct

<220>
<221> misc_feature
<222> (20)..(21)
<223> deoxythymine (dT) base

<400> 213
cuaagaaagg ucaaaggcat t 21

<210> 214
<211> 21
<212> DNA
<213> Artificial Sequence

<220>
<223>  Synthetic Construct

<220>
<221>  misc_feature
<222>  (20)..(21)
<223>  deoxythymine (dT) base

<400>  214
gguccaacau agcuuugagt t                                                21

<210>  215
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  215
gaacacuuua cuucugguct t                                                21

<210>  216
<211>  21
<212>  DNA
<213>  Artificial Sequence

<220>
<223>  Synthetic Construct

<220>
<221>  misc_feature
<222>  (20)..(21)
<223>  deoxythymine (dT) base

<400>  216
uuucuuccca ggagaucagt t                                                21

<210>  217
<211>  21
<212>  DNA
<213>  Artificial Sequence

<220>
<223>  Synthetic Construct

<220>
<221>  misc_feature
<222>  (20)..(21)
<223>  deoxythymine (dT) base

<400> 217
agcggacaaa gagcaggaat t 21

<210> 218
<211> 21
<212> DNA
<213> Artificial Sequence

<220>
<223> Synthetic Construct

<220>
<221> misc_feature
<222> (20)..(21)
<223> deoxythymine (dT) base

<400> 218
ugaggaugca uaggaccugt t 21

<210> 219
<211> 21
<212> DNA
<213> Artificial Sequence

<220>
<223> Synthetic Construct

<220>
<221> misc_feature
<222> (20)..(21)
<223> deoxythymine (dT) base

<400> 219
uucccaggag aucagcggat t 21

<210> 220
<211> 21
<212> DNA
<213> Artificial Sequence

<220>
<223> Synthetic Construct

<220>
<221> misc_feature
<222> (20)..(21)
<223> deoxythymine (dT) base

<400> 220
aaaauaccac ucgaugcuat t 21

<210> 221
<211> 21
<212> DNA
<213> Artificial Sequence

<220>
<223> Synthetic Construct

<220>
<221> misc_feature
<222> (20)..(21)
<223> deoxythymine (dT) base

<400> 221
gcuuucuucc caggagauct t 21

<210> 222
<211> 21
<212> DNA
<213> Artificial Sequence

<220>
<223> Synthetic Construct

<220>
<221> misc_feature
<222> (20)..(21)
<223> deoxythymine (dT) base

<400> 222
aaggucaaag gcaaauugat t 21

<210> 223
<211> 21
<212> DNA
<213> Artificial Sequence

<220>
<223> Synthetic Construct

<220>
<221> misc_feature
<222> (20)..(21)
<223> deoxythymine (dT) base

<400> 223
uucuggucca acauagcuut t 21

<210> 224
<211> 21
<212> DNA
<213> Artificial Sequence

<220>
<223> Synthetic Construct

<220>
<221> misc_feature
<222> (20)..(21)
<223> deoxythymine (dT) base

<400> 224
uaagaaaggu caaaggcaat t 21

<210> 225
<211> 21
<212> DNA
<213> Artificial Sequence

<220>
<223> Synthetic Construct

<220>
<221> misc_feature
<222> (20)..(21)
<223> deoxythymine (dT) base

<400> 225
agucccuaag aaaggucaat t 21

<210> 226
<211> 21
<212> DNA
<213> Artificial Sequence

<220>
<223> Synthetic Construct

<220>
<221> misc_feature
<222> (20)..(21)
<223> deoxythymine (dT) base

<400> 226
gaagaaaacg ggacuaucat t 21

<210> 227
<211> 21
<212> DNA
<213> Artificial Sequence

<220>
<223> Synthetic Construct

<220>
<221> misc_feature
<222> (20)..(21)
<223> deoxythymine (dT) base

<400> 227
uaacagcccu gcauagaugt t 21

<210> 228
<211> 21
<212> DNA
<213> Artificial Sequence

<220>
<223> Synthetic Construct

<220>
<221> misc_feature
<222> (20)..(21)
<223> deoxythymidine (dT) base

<400> 228
ugagcuuuca aagaagagat t 21

<210> 229
<211> 21
<212> DNA
<213> Artificial Sequence

<220>
<223> Synthetic Construct

<220>
<221> misc_feature
<222> (20)..(21)
<223> deoxythymidine (dT) base

<400> 229
cuucuggucc aacauagcut t 21

<210> 230
<211> 21
<212> DNA
<213> Artificial Sequence

<220>
<223> deoxythymidine (dT) base

<400> 230
agagcaggaa aagcagaaat t 21

<210> 231
<211> 21
<212> DNA
<213> Artificial Sequence

<220>
<223> Synthetic Construct

<220>
<221> misc_feature
<222> (20)..(21)
<223> deoxythymidine (dT) base

<400> 231
gagcaggaaa agcagaaaat t 21

<210> 232
<211> 21

```
<212>  DNA
<213>  Artificial Sequence

<220>
<223>  Synthetic Construct


<220>
<221>  misc_feature
<222>  (20)..(21)
<223>  deoxythymidine (dT) base

<400>  232
ucuucccagg agaucagcgt t                                                 21


<210>  233
<211>  21
<212>  DNA
<213>  Artificial Sequence

<220>
<223>  Synthetic Construct


<220>
<221>  misc_feature
<222>  (20)..(21)
<223>  deoxythymidine (dT) base

<400>  233
acuucugguc caacauagct t                                                 21


<210>  234
<211>  21
<212>  DNA
<213>  Artificial Sequence

<220>
<223>  Synthetic Construct


<220>
<221>  misc_feature
<222>  (20)..(21)
<223>  deoxythymidine (dT) base

<400>  234
aauaccacuc gaugcuacat t                                                 21


<210>  235
<211>  21
<212>  DNA
<213>  Artificial Sequence

<220>
<223>  Synthetic Construct


<220>
<221>  misc_feature
```

<222> (20)..(21)
<223> deoxythymidine (dT) base

<400> 235
ugaaacggug uccuuuucgt t 21

<210> 236
<211> 21
<212> DNA
<213> Artificial Sequence

<220>
<223> Synthetic Construct

<220>
<221> misc_feature
<222> (20)..(21)
<223> deoxythymidine (dT) base

<400> 236
gguguccuuu ucggaagcut t 21

<210> 237
<211> 21
<212> DNA
<213> Artificial Sequence

<220>
<223> Synthetic Construct

<220>
<221> misc_feature
<222> (20)..(21)
<223> deoxythymidine (dT) base

<400> 237
cggaagcuuu cuucccaggt t 21

<210> 238
<211> 21
<212> DNA
<213> Artificial Sequence

<220>
<223> Synthetic Construct

<220>
<221> misc_feature
<222> (20)..(21)
<223> deoxythymidine (dT) base

<400> 238
cggacaaaga gcaggaaaat t 21

<210> 239
<211> 21

<212> DNA
<213> Artificial Sequence

<220>
<223> Synthetic Construct

<220>
<221> misc_feature
<222> (20)..(21)
<223> deoxythymidine (dT) base

<400> 239
gaucagcgga caaagagcat t 21

<210> 240
<211> 21
<212> DNA
<213> Artificial Sequence

<220>
<223> Synthetic Construct

<220>
<221> misc_feature
<222> (20)..(21)
<223> deoxythymidine (dT) base

<400> 240
cacuuuacuu cugguccaat t 21

<210> 241
<211> 21
<212> DNA
<213> Artificial Sequence

<220>
<223> Synthetic Construct

<220>
<221> misc_feature
<222> (20)..(21)
<223> deoxythymidine (dT) base

<400> 241
aggagaucag cggacaaagt t 21

<210> 242
<211> 21
<212> DNA
<213> Artificial Sequence

<220>
<223> Synthetic Construct

<220>
<221> misc_feature

<222> (20)..(21)
<223> deoxythymidine (dT) base

<400> 242
aaaggcaaau ugagacaagt t 21

<210> 243
<211> 21
<212> DNA
<213> Artificial Sequence

<220>
<223> Synthetic Construct

<220>
<221> misc_feature
<222> (20)..(21)
<223> deoxythymidine (dT) base

<400> 243
agggcaagaa gaccgggugt t 21

<210> 244
<211> 21
<212> DNA
<213> Artificial Sequence

<220>
<223> Synthetic Construct

<220>
<221> misc_feature
<222> (20)..(21)
<223> deoxythymidine (dT) base

<400> 244
ucccuaagaa aggucaaagt t 21

<210> 245
<211> 21
<212> DNA
<213> Artificial Sequence

<220>
<223> Synthetic Construct

<220>
<221> misc_feature
<222> (20)..(21)
<223> deoxythymidine (dT) base

<400> 245
cccuaagaaa ggucaaaggt t 21

<210> 246
<211> 21

<212> DNA
<213> Artificial Sequence

<220>
<223> Synthetic Construct

<220>
<221> misc_feature
<222> (20)..(21)
<223> deoxythymidine (dT) base

<400> 246
uucggaagcu uucuucccat t 21

<210> 247
<211> 21
<212> DNA
<213> Artificial Sequence

<220>
<223> Synthetic Construct

<220>
<221> misc_feature
<222> (20)..(21)
<223> deoxythymidine (dT) base

<400> 247
gauaacagcc cugcauagat t 21

<210> 248
<211> 21
<212> DNA
<213> Artificial Sequence

<220>
<223> Synthetic Construct

<220>
<221> misc_feature
<222> (20)..(21)
<223> deoxythymidine (dT) base

<400> 248
ucugguccaa cauagcuuut t 21

<210> 249
<211> 21
<212> DNA
<213> Artificial Sequence

<220>
<223> Synthetic Construct

<220>
<221> misc_feature

<222> (20)..(21)
<223> deoxythymidine (dT) base

<400> 249
ggucaaaggc aaauugagat t 21

<210> 250
<211> 21
<212> DNA
<213> Artificial Sequence

<220>
<223> Synthetic Construct

<220>
<221> misc_feature
<222> (20)..(21)
<223> deoxythymidine (dT) base

<400> 250
cugcauagau gaggaugcat t 21

<210> 251
<211> 21
<212> DNA
<213> Artificial Sequence

<220>
<223> Synthetic Construct

<220>
<221> misc_feature
<222> (20)..(21)
<223> deoxythymidine (dT) base

<400> 251
uggccccucg gaaccuuuct t 21

<210> 252
<211> 21
<212> DNA
<213> Artificial Sequence

<220>
<223> Synthetic Construct

<220>
<221> misc_feature
<222> (20)..(21)
<223> deoxythymidine (dT) base

<400> 252
caaauugaga caagaaggct t 21

<210> 253
<211> 21

<212> DNA
<213> Artificial Sequence

<220>
<223> Synthetic Construct

<220>
<221> misc_feature
<222> (20)..(21)
<223> deoxythymidine (dT) base

<400> 253
gauauuaaaa gggaggagut t 21

<210> 254
<211> 21
<212> DNA
<213> Artificial Sequence

<220>
<223> Synthetic Construct

<220>
<221> misc_feature
<222> (20)..(21)
<223> deoxythymidine (dT) base

<400> 254
cuuuucggaa gcuuucuuct t 21

<210> 255
<211> 21
<212> DNA
<213> Artificial Sequence

<220>
<223> Synthetic Construct

<220>
<221> misc_feature
<222> (20)..(21)
<223> deoxythymidine (dT) base

<400> 255
aagucccuaa gaaaggucat t 21

<210> 256
<211> 21
<212> DNA
<213> Artificial Sequence

<220>
<223> Synthetic Construct

<220>
<221> misc_feature

<222> (20)..(21)
<223> deoxythymidine (dT) base

<400> 256
ccaacauagc uuugagcuut t 21

<210> 257
<211> 21
<212> DNA
<213> Artificial Sequence

<220>
<223> Synthetic Construct

<220>
<221> misc_feature
<222> (20)..(21)
<223> deoxythymidine (dT) base

<400> 257
gggugcaaac ccggaacugt t 21

<210> 258
<211> 21
<212> DNA
<213> Artificial Sequence

<220>
<223> Synthetic Construct

<220>
<221> misc_feature
<222> (20)..(21)
<223> deoxythymidine (dT) base

<400> 258
ggcaagaaga ccgggugcat t 21

<210> 259
<211> 21
<212> DNA
<213> Artificial Sequence

<220>
<223> Synthetic Construct

<220>
<221> misc_feature
<222> (20)..(21)
<223> deoxythymidine (dT) base

<400> 259
augcguuaaa cccaggaggt t 21

<210> 260
<211> 21

<212> DNA
<213> Artificial Sequence

<220>
<223> Synthetic Construct

<220>
<221> misc_feature
<222> (20)..(21)
<223> deoxythymidine (dT) base

<400> 260
cgguguccuu uucggaagct t 21

<210> 261
<211> 21
<212> DNA
<213> Artificial Sequence

<220>
<223> Synthetic Construct

<220>
<221> misc_feature
<222> (20)..(21)
<223> deoxythymidine (dT) base

<400> 261
aggucaaagg caaauugagt t 21

<210> 262
<211> 21
<212> DNA
<213> Artificial Sequence

<220>
<223> Synthetic Construct

<220>
<221> misc_feature
<222> (20)..(21)
<223> deoxythymidine (dT) base

<400> 262
gaugaggaug cauaggacct t 21

<210> 263
<211> 21
<212> DNA
<213> Artificial Sequence

<220>
<223> Synthetic Construct

<220>
<221> misc_feature

<222> (20)..(21)
<223> deoxythymidine (dT) base

<400> 263
agcaggaaaa gcagaaaact t 21

<210> 264
<211> 21
<212> DNA
<213> Artificial Sequence

<220>
<223> Synthetic Construct

<220>
<221> misc_feature
<222> (20)..(21)
<223> deoxythymidine (dT) base

<400> 264
caggaaaagc agaaaacaat t 21

<210> 265
<211> 21
<212> DNA
<213> Artificial Sequence

<220>
<223> Synthetic Construct

<220>
<221> misc_feature
<222> (20)..(21)
<223> deoxythymidine (dT) base

<400> 265
cgagaacacu uuacuucugt t 21

<210> 266
<211> 21
<212> DNA
<213> Artificial Sequence

<220>
<223> Synthetic Construct

<220>
<221> misc_feature
<222> (20)..(21)
<223> deoxythymidine (dT) base

<400> 266
acaaagagca ggaaaagcat t 21

<210> 267
<211> 21

<212> DNA
<213> Artificial Sequence

<220>
<223> Synthetic Construct

<220>
<221> misc_feature
<222> (20)..(21)
<223> deoxythymidine (dT) base

<400> 267
uucuucccag gagaucagct t 21

<210> 268
<211> 21
<212> DNA
<213> Artificial Sequence

<220>
<223> Synthetic Construct

<220>
<221> misc_feature
<222> (20)..(21)
<223> deoxythymidine (dT) base

<400> 268
cucgccccug aaacggugut t 21

<210> 269
<211> 21
<212> DNA
<213> Artificial Sequence

<220>
<223> Synthetic Construct

<220>
<221> misc_feature
<222> (20)..(21)
<223> deoxythymidine (dT) base

<400> 269
cugaaacggu guccuuuuct t 21

<210> 270
<211> 21
<212> DNA
<213> Artificial Sequence

<220>
<223> Synthetic Construct

<220>
<221> misc_feature

<222> (20)..(21)
<223> deoxythymidine (dT) base

<400> 270
gaccugggcu gagaagaaat t 21

<210> 271
<211> 21
<212> DNA
<213> Artificial Sequence

<220>
<223> Synthetic Construct

<220>
<221> misc_feature
<222> (20)..(21)
<223> deoxythymidine (dT) base

<400> 271
aacagcccug cauagaugat t 21

<210> 272
<211> 21
<212> DNA
<213> Artificial Sequence

<220>
<223> Synthetic Construct

<220>
<221> misc_feature
<222> (20)..(21)
<223> deoxythymidine (dT) base

<400> 272
aaagggagga guuuccucat t 21

<210> 273
<211> 21
<212> DNA
<213> Artificial Sequence

<220>
<223> Synthetic Construct

<220>
<221> misc_feature
<222> (20)..(21)
<223> deoxythymidine (dT) base

<400> 273
aguuuccuca auggccccut t 21

<210> 274
<211> 21

<212> DNA
<213> Artificial Sequence

<220>
<223> Synthetic Construct

<220>
<221> misc_feature
<222> (20)..(21)
<223> deoxythymidine (dT) base

<400> 274
aagaaagguc aaaggcaaat t 21

<210> 275
<211> 21
<212> DNA
<213> Artificial Sequence

<220>
<223> Synthetic Construct

<220>
<221> misc_feature
<222> (20)..(21)
<223> deoxythymidine (dT) base

<400> 275
ccaggagauc agcggacaat t 21

<210> 276
<211> 21
<212> DNA
<213> Artificial Sequence

<220>
<223> Synthetic Construct

<220>
<221> misc_feature
<222> (20)..(21)
<223> deoxythymidine (dT) base

<400> 276
auaggaccug ggcugagaat t 21

<210> 277
<211> 21
<212> DNA
<213> Artificial Sequence

<220>
<223> Synthetic Construct

<220>
<221> misc_feature

<222> (20)..(21)
<223> deoxythymidine (dT) base

<400> 277
aauugagaca agaaggcact t 21

<210> 278
<211> 21
<212> DNA
<213> Artificial Sequence

<220>
<223> Synthetic Construct

<220>
<221> misc_feature
<222> (20)..(21)
<223> deoxythymidine (dT) base

<400> 278
aucacaaugc guuaaaccct t 21

<210> 279
<211> 21
<212> DNA
<213> Artificial Sequence

<220>
<223> Synthetic Construct

<220>
<221> misc_feature
<222> (20)..(21)
<223> deoxythymidine (dT) base

<400> 279
acacuuuacu ucugguccat t 21

<210> 280
<211> 21
<212> DNA
<213> Artificial Sequence

<220>
<223> Synthetic Construct

<220>
<221> misc_feature
<222> (20)..(21)
<223> deoxythymidine (dT) base

<400> 280
caaaggcaaa uugagacaat t 21

<210> 281
<211> 21

<212> DNA
<213> Artificial Sequence

<220>
<223> Synthetic Construct

<220>
<221> misc_feature
<222> (20)..(21)
<223> deoxythymidine (dT) base

<400> 281
uuucggaagc uuucuuccct t 21

<210> 282
<211> 21
<212> DNA
<213> Artificial Sequence

<220>
<223> Synthetic Construct

<220>
<221> misc_feature
<222> (20)..(21)
<223> deoxythymidine (dT) base

<400> 282
ggaugcauag gaccugggct t 21

<210> 283
<211> 21
<212> DNA
<213> Artificial Sequence

<220>
<223> Synthetic Construct

<220>
<221> misc_feature
<222> (20)..(21)
<223> deoxythymidine (dT) base

<400> 283
caagaagacc gggugcaaat t 21

<210> 284
<211> 21
<212> DNA
<213> Artificial Sequence

<220>
<223> Synthetic Construct

<220>
<221> misc_feature

<222> (20)..(21)
<223> deoxythymidine (dT) base

<400> 284
ucggaagcuu ucuucccagt t 21

<210> 285
<211> 21
<212> DNA
<213> Artificial Sequence

<220>
<223> Synthetic Construct

<220>
<221> misc_feature
<222> (20)..(21)
<223> deoxythymidine (dT) base

<400> 285
ggugcaaacc cggaacuggt t 21

<210> 286
<211> 21
<212> DNA
<213> Artificial Sequence

<220>
<223> Synthetic Construct

<220>
<221> misc_feature
<222> (20)..(21)
<223> deoxythymidine (dT) base

<400> 286
ccugggcuga gaagaaaact t 21

<210> 287
<211> 21
<212> DNA
<213> Artificial Sequence

<220>
<223> Synthetic Construct

<220>
<221> misc_feature
<222> (20)..(21)
<223> deoxythymidine (dT) base

<400> 287
aaggcaaauu gagacaagat t 21

<210> 288
<211> 21

<212> DNA
<213> Artificial Sequence

<220>
<223> Synthetic Construct

<220>
<221> misc_feature
<222> (20)..(21)
<223> deoxythymidine (dT) base

<400> 288
agaugaggau gcauaggact t 21

<210> 289
<211> 21
<212> DNA
<213> Artificial Sequence

<220>
<223> Synthetic Construct

<220>
<221> misc_feature
<222> (20)..(21)
<223> deoxythymidine (dT) base

<400> 289
aacggugucc uuuucggaat t 21

<210> 290
<211> 21
<212> DNA
<213> Artificial Sequence

<220>
<223> Synthetic Construct

<220>
<221> misc_feature
<222> (20)..(21)
<223> deoxythymidine (dT) base

<400> 290
accugggcug agaagaaaat t 21

<210> 291
<211> 21
<212> DNA
<213> Artificial Sequence

<220>
<223> Synthetic Construct

<220>
<221> misc_feature

<222> (20)..(21)
<223> deoxythymidine (dT) base

<400> 291
aacgggacua ucacaaugct t 21

<210> 292
<211> 21
<212> DNA
<213> Artificial Sequence

<220>
<223> Synthetic Construct

<220>
<221> misc_feature
<222> (20)..(21)
<223> deoxythymidine (dT) base

<400> 292
cccucggaac cuuucaagut t 21

<210> 293
<211> 21
<212> DNA
<213> Artificial Sequence

<220>
<223> Synthetic Construct

<220>
<221> misc_feature
<222> (20)..(21)
<223> deoxythymidine (dT) base

<400> 293
ucaauggccc cucggaacct t 21

<210> 294
<211> 21
<212> DNA
<213> Artificial Sequence

<220>
<223> Synthetic Construct

<220>
<221> misc_feature
<222> (20)..(21)
<223> deoxythymidine (dT) base

<400> 294
gcaaauugag acaagaaggt t 21

<210> 295
<211> 21

<212> DNA
<213> Artificial Sequence

<220>
<223> Synthetic Construct

<220>
<221> misc_feature
<222> (20)..(21)
<223> deoxythymidine (dT) base

<400> 295
ucacaaugcg uuaaacccat t 21

<210> 296
<211> 21
<212> DNA
<213> Artificial Sequence

<220>
<223> Synthetic Construct

<220>
<221> misc_feature
<222> (20)..(21)
<223> deoxythymidine (dT) base

<400> 296
gucccuaaga aaggucaaat t 21

<210> 297
<211> 21
<212> DNA
<213> Artificial Sequence

<220>
<223> Synthetic Construct

<220>
<221> misc_feature
<222> (20)..(21)
<223> deoxythymidiine (dT) base

<400> 297
aagaaaacgg gacuaucact t 21

<210> 298
<211> 21
<212> DNA
<213> Artificial Sequence

<220>
<223> Synthetic Construct

<220>
<221> misc_feature

<222> (20)..(21)
<223> deoxythymidine (dT) base

<400> 298
agacaagaag gcaccuguat t 21

<210> 299
<211> 21
<212> DNA
<213> Artificial Sequence

<220>
<223> Synthetic Construct

<220>
<221> misc_feature
<222> (20)..(21)
<223> deoxythymidine (dT) base

<400> 299
agaaaacggg acuaucacat t 21

<210> 300
<211> 21
<212> DNA
<213> Artificial Sequence

<220>
<223> Synthetic Construct

<220>
<221> misc_feature
<222> (20)..(21)
<223> deoxythymidine (dT) base

<400> 300
uaugcagaua acagcccugt t 21

<210> 301
<211> 21
<212> DNA
<213> Artificial Sequence

<220>
<223> Synthetic Construct

<220>
<221> misc_feature
<222> (20)..(21)
<223> deoxythymidine (dT) base

<400> 301
ucacucgccc cugaaacggt t 21

<210> 302
<211> 21

<212> DNA
<213> Artificial Sequence

<220>
<223> Synthetic Construct

<220>
<221> misc_feature
<222> (20)..(21)
<223> deoxythymidine (dT) base

<400> 302
gaggaguuuc cucaauggct t 21

<210> 303
<211> 21
<212> DNA
<213> Artificial Sequence

<220>
<223> Synthetic Construct

<220>
<221> misc_feature
<222> (20)..(21)
<223> deoxythymidine (dT) base

<400> 303
ggaggaguuu ccucaauggt t 21

<210> 304
<211> 21
<212> DNA
<213> Artificial Sequence

<220>
<223> Synthetic Construct

<220>
<221> misc_feature
<222> (20)..(21)
<223> deoxythymidine (dT) base

<400> 304
gcauaggacc ugggcugagt t 21

<210> 305
<211> 21
<212> DNA
<213> Artificial Sequence

<220>
<223> Synthetic Construct

<220>
<221> misc_feature

<222> (20)..(21)
<223> deoxythymidine (dT) base

<400> 305
cuuucuuccc aggagaucat t 21

<210> 306
<211> 21
<212> DNA
<213> Artificial Sequence

<220>
<223> Synthetic Construct

<220>
<221> misc_feature
<222> (20)..(21)
<223> deoxythymidine (dT) base

<400> 306
uaccacucga ugcuacauut t 21

<210> 307
<211> 21
<212> DNA
<213> Artificial Sequence

<220>
<223> Synthetic Construct

<220>
<221> misc_feature
<222> (20)..(21)
<223> deoxythymidine (dT) base

<400> 307
cuuugagcuu ucaaagaagt t 21

<210> 308
<211> 21
<212> DNA
<213> Artificial Sequence

<220>
<223> Synthetic Construct

<220>
<221> misc_feature
<222> (20)..(21)
<223> deoxythymidine (dT) base

<400> 308
ccccugaaac gguguccuut t 21

<210> 309
<211> 21

<212> DNA
<213> Artificial Sequence

<220>
<223> Synthetic Construct

<220>
<221> misc_feature
<222> (20)..(21)
<223> deoxythymidine (dT) base

<400> 309
gaaacggugu ccuuuucggt t 21

<210> 310
<211> 21
<212> DNA
<213> Artificial Sequence

<220>
<223> Synthetic Construct

<220>
<221> misc_feature
<222> (20)..(21)
<223> deoxythymidine (dT) base

<400> 310
ccucggaacc uuucaaguut t 21

<210> 311
<211> 21
<212> DNA
<213> Artificial Sequence

<220>
<223> Synthetic Construct

<220>
<221> misc_feature
<222> (20)..(21)
<223> deoxythymidine (dT) base

<400> 311
gagaacacuu uacuucuggt t 21

<210> 312
<211> 21
<212> DNA
<213> Artificial Sequence

<220>
<223> Synthetic Construct

<220>
<221> misc_feature

<222> (20)..(21)
<223> deoxythymidine (dT) base

<400> 312
gcugagaaga aaacgggact t 21

<210> 313
<211> 21
<212> DNA
<213> Artificial Sequence

<220>
<223> Synthetic Construct

<220>
<221> misc_feature
<222> (20)..(21)
<223> deoxythymidine (dT) base

<400> 313
cagcccugca uagaugaggt t 21

<210> 314
<211> 21
<212> DNA
<213> Artificial Sequence

<220>
<223> Synthetic Construct

<220>
<221> misc_feature
<222> (20)..(21)
<223> deoxythymidine (dT) base

<400> 314
guccuuuucg gaagcuuuct t 21

<210> 315
<211> 21
<212> DNA
<213> Artificial Sequence

<220>
<223> Synthetic Construct

<220>
<221> misc_feature
<222> (20)..(21)
<223> deoxythymidine (dT) base

<400> 315
cagcggacaa agagcaggat t 21

<210> 316
<211> 21

<212> DNA
<213> Artificial Sequence

<220>
<223> Synthetic Construct

<220>
<221> misc_feature
<222> (20)..(21)
<223> deoxythymidine (dT) base

<400> 316
aacaaguccc uaagaaaggt t 21

<210> 317
<211> 21
<212> DNA
<213> Artificial Sequence

<220>
<223> Synthetic Construct

<220>
<221> misc_feature
<222> (20)..(21)
<223> deoxythymidine (dT) base

<400> 317
cagaaaacaa gucccuaagt t 21

<210> 318
<211> 21
<212> DNA
<213> Artificial Sequence

<220>
<223> Synthetic Construct

<220>
<221> misc_feature
<222> (20)..(21)
<223> deoxythymidine (dT) base

<400> 318
guuaaaccca ggagguuuut t 21

<210> 319
<211> 21
<212> DNA
<213> Artificial Sequence

<220>
<223> Synthetic Construct

<220>
<221> misc_feature

<222> (20)..(21)
<223> deoxythymidine (dT) base

<400> 319
gccccugaaa cgguguccut t 21

<210> 320
<211> 21
<212> DNA
<213> Artificial Sequence

<220>
<223> Synthetic Construct

<220>
<221> misc_feature
<222> (20)..(21)
<223> deoxythymidine (dT) base

<400> 320
uccuuuucgg aagcuuucut t 21

<210> 321
<211> 21
<212> DNA
<213> Artificial Sequence

<220>
<223> Synthetic Construct

<220>
<221> misc_feature
<222> (20)..(21)
<223> deoxythymidine (dT) base

<400> 321
uaaaagggag gaguuuccut t 21

<210> 322
<211> 21
<212> DNA
<213> Artificial Sequence

<220>
<223> Synthetic Construct

<220>
<221> misc_feature
<222> (20)..(21)
<223> deoxythymidine (dT) base

<400> 322
uuuccucaau ggccccucgt t 21

<210> 323
<211> 21

<212> DNA
<213> Artificial Sequence

<220>
<223> Synthetic Construct

<220>
<221> misc_feature
<222> (20)..(21)
<223> deoxythymidine (dT) base

<400> 323
acaaugcguu aaacccaggt t 21

<210> 324
<211> 21
<212> DNA
<213> Artificial Sequence

<220>
<223> Synthetic Construct

<220>
<221> misc_feature
<222> (20)..(21)
<223> deoxythymidine (dT) base

<400> 324
uagcuuugag cuuucaaagt t 21

<210> 325
<211> 21
<212> DNA
<213> Artificial Sequence

<220>
<223> Synthetic Construct

<220>
<221> misc_feature
<222> (20)..(21)
<223> deoxythymidine (dT) base

<400> 325
acgagaacac uuuacuucut t 21

<210> 326
<211> 21
<212> DNA
<213> Artificial Sequence

<220>
<223> Synthetic Construct

<220>
<221> misc_feature

<222> (20)..(21)
<223> deoxythymidine (dT) base

<400> 326
ugcauagaug aggaugcaut t 21

<210> 327
<211> 21
<212> DNA
<213> Artificial Sequence

<220>
<223> Synthetic Construct

<220>
<221> misc_feature
<222> (20)..(21)
<223> deoxythymidine (dT) base

<400> 327
ccccucggaa ccuuucaagt t 21

<210> 328
<211> 21
<212> DNA
<213> Artificial Sequence

<220>
<223> Synthetic Construct

<220>
<221> misc_feature
<222> (20)..(21)
<223> deoxythymidine (dT) base

<400> 328
gacaaagagc aggaaaagct t 21

<210> 329
<211> 19
<212> DNA
<213> Artificial Sequence

<220>
<223> Synthetic Construct

<400> 329
gctctttgtc cgctgatct 19

<210> 330
<211> 19
<212> DNA
<213> Artificial Sequence

<220>
<223> Synthetic Construct

<400> 330
tgttggacca gaagtaaag 19

<210> 331
<211> 19
<212> DNA
<213> Artificial Sequence

<220>
<223> Synthetic Construct

<400> 331
aggtcctatg catcctcat 19

<210> 332
<211> 19
<212> DNA
<213> Artificial Sequence

<220>
<223> Synthetic Construct

<400> 332
ctctttgtcc gctgatctc 19

<210> 333
<211> 19
<212> DNA
<213> Artificial Sequence

<220>
<223> Synthetic Construct

<400> 333
aatttgcctt tgacctttc 19

<210> 334
<211> 19
<212> DNA
<213> Artificial Sequence

<220>
<223> Synthetic Construct

<400> 334
atttgccttt gacctttct 19

<210> 335
<211> 19
<212> DNA
<213> Artificial Sequence

<220>
<223> Synthetic Construct

<400> 335
tttcctgctc tttgtccgc 19

<210> 336
<211> 19
<212> DNA
<213> Artificial Sequence

<220>
<223> Synthetic Construct

<400> 336
ttcttctcag cccaggtcc 19

<210> 337
<211> 19
<212> DNA
<213> Artificial Sequence

<220>
<223> Synthetic Construct

<400> 337
tctgcttttc ctgctcttt 19

<210> 338
<211> 19
<212> DNA
<213> Artificial Sequence

<220>
<223> Synthetic Construct

<400> 338
ttctgctttt cctgctctt 19

<210> 339
<211> 19
<212> DNA
<213> Artificial Sequence

<220>
<223> Synthetic Construct

<400> 339
aaggttccga ggggccatt 19

<210> 340
<211> 19
<212> DNA
<213> Artificial Sequence

<220>
<223> Synthetic Construct

<400> 340
aaaaggacac cgtttcagg 19

<210> 341
<211> 19

<212> DNA
<213> Artificial Sequence

<220>
<223> Synthetic Construct

<400> 341
aaaggacacc gtttcaggg 19

<210> 342
<211> 19
<212> DNA
<213> Artificial Sequence

<220>
<223> Synthetic Construct

<400> 342
atgttggacc agaagtaaa 19

<210> 343
<211> 19
<212> DNA
<213> Artificial Sequence

<220>
<223> Synthetic Construct

<400> 343
tcttctcagc ccaggtcct 19

<210> 344
<211> 19
<212> DNA
<213> Artificial Sequence

<220>
<223> Synthetic Construct

<220>
<221> misc_feature
<222> (20)..(21)
<223> deoxythymidine (dT) base

<400> 344
tgtctcaatt tgcctttga 19

<210> 345
<211> 19
<212> DNA
<213> Artificial Sequence

<220>
<223> Synthetic Construct

<220>
<221> misc_feature

<222> (20)..(21)
<223> deoxythymidine (dT) base

<400> 345
ccaggtccta tgcatcctc 19

<210> 346
<211> 19
<212> DNA
<213> Artificial Sequence

<220>
<223> Synthetic Construct

<400> 346
ctgcttttcc tgctctttg 19

<210> 347
<211> 19
<212> DNA
<213> Artificial Sequence

<220>
<223> Synthetic Construct

<400> 347
ttcttaggga cttgttttc 19

<210> 348
<211> 19
<212> DNA
<213> Artificial Sequence

<220>
<223> Synthetic Construct

<400> 348
atctatgcag ggctgttat 19

<210> 349
<211> 19
<212> DNA
<213> Artificial Sequence

<220>
<223> Synthetic Construct

<400> 349
ttcttgtctc aatttgcct 19

<210> 350
<211> 19
<212> DNA
<213> Artificial Sequence

<220>
<223> Synthetic Construct

<400> 350
gaaagctcaa agctatgtt 19

<210> 351
<211> 19
<212> DNA
<213> Artificial Sequence

<220>
<223> Synthetic Construct

<400> 351
ttgaaagctc aaagctatg 19

<210> 352
<211> 19
<212> DNA
<213> Artificial Sequence

<220>
<223> Synthetic Construct

<400> 352
ggtttgcacc cggtcttct 19

<210> 353
<211> 19
<212> DNA
<213> Artificial Sequence

<220>
<223> Synthetic Construct

<400> 353
accagaagta aagtgttct 19

<210> 354
<211> 19
<212> DNA
<213> Artificial Sequence

<220>
<223> Synthetic Construct

<400> 354
gtttgcaccc ggtcttctt 19

<210> 355
<211> 19
<212> DNA
<213> Artificial Sequence

<220>
<223> Synthetic Construct

<400> 355
gtctcaattt gcctttgac 19

<210> 356
<211> 19
<212> DNA
<213> Artificial Sequence

<220>
<223> Synthetic Construct

<400> 356
aggtgccttc ttgtctcaa 19

<210> 357
<211> 19
<212> DNA
<213> Artificial Sequence

<220>
<223> Synthetic Construct

<400> 357
ttgaggaaac tcctccctt 19

<210> 358
<211> 19
<212> DNA
<213> Artificial Sequence

<220>
<223> Synthetic Construct

<400> 358
tcctatgcat cctcatcta 19

<210> 359
<211> 19
<212> DNA
<213> Artificial Sequence

<220>
<223> Synthetic Construct

<400> 359
tgaaagctca aagctatgt 19

<210> 360
<211> 19
<212> DNA
<213> Artificial Sequence

<220>
<223> Synthetic Construct

<400> 360
tgttttctgc ttttcctgc 19

<210> 361
<211> 19

<212> DNA
<213> Artificial Sequence

<220>
<223> Synthetic Construct

<400> 361
ctcttctttg aaagctcaa 19

<210> 362
<211> 19
<212> DNA
<213> Artificial Sequence

<220>
<223> Synthetic Construct

<400> 362
aaaggttccg aggggccat 19

<210> 363
<211> 19
<212> DNA
<213> Artificial Sequence

<220>
<223> Synthetic Construct

<400> 363
cttcttgtct caatttgcc 19

<210> 364
<211> 19
<212> DNA
<213> Artificial Sequence

<220>
<223> Synthetic Construct

<400> 364
attgaggaaa ctcctccct 19

<210> 365
<211> 19
<212> DNA
<213> Artificial Sequence

<220>
<223> Synthetic Construct

<400> 365
tatgttggac cagaagtaa 19

<210> 366
<211> 19
<212> DNA
<213> Artificial Sequence

<220>
<223> Synthetic Construct

<400> 366
caggtgcctt cttgtctca 19

<210> 367
<211> 19
<212> DNA
<213> Artificial Sequence

<220>
<223> Synthetic Construct

<400> 367
caatttgcct ttgaccttt 19

<210> 368
<211> 19
<212> DNA
<213> Artificial Sequence

<220>
<223> Synthetic Construct

<400> 368
gtaaagtgtt ctcgtttct 19

<210> 369
<211> 19
<212> DNA
<213> Artificial Sequence

<220>
<223> Synthetic Construct

<400> 369
cccaggtcct atgcatcct 19

<210> 370
<211> 19
<212> DNA
<213> Artificial Sequence

<220>
<223> Synthetic Construct

<400> 370
cctatgcatc ctcatctat 19

<210> 371
<211> 19
<212> DNA
<213> Artificial Sequence

<220>
<223> Synthetic Construct

<400> 371
aagcttccga aaaggacac 19

<210> 372
<211> 19
<212> DNA
<213> Artificial Sequence

<220>
<223> Synthetic Construct

<400> 372
ttgcacccgg tcttcttgc 19

<210> 373
<211> 19
<212> DNA
<213> Artificial Sequence

<220>
<223> Synthetic Construct

<400> 373
gtccgctgat ctcctggga 19

<210> 374
<211> 19
<212> DNA
<213> Artificial Sequence

<220>
<223> Synthetic Construct

<400> 374
ttgaaaggtt ccgaggggc 19

<210> 375
<211> 19
<212> DNA
<213> Artificial Sequence

<220>
<223> Synthetic Construct

<400> 375
catcctcatc tatgcaggg 19

<210> 376
<211> 19
<212> DNA
<213> Artificial Sequence

<220>
<223> Synthetic Construct

<400> 376
ggtgccttct tgtctcaat 19

<210> 377
<211> 19
<212> DNA
<213> Artificial Sequence

<220>
<223> Synthetic Construct

<400> 377
tgcctttgac ctttcttag 19

<210> 378
<211> 19
<212> DNA
<213> Artificial Sequence

<220>
<223> Synthetic Construct

<400> 378
ctcaaagcta tgttggacc 19

<210> 379
<211> 19
<212> DNA
<213> Artificial Sequence

<220>
<223> Synthetic Construct

<400> 379
gaccagaagt aaagtgttc 19

<210> 380
<211> 19
<212> DNA
<213> Artificial Sequence

<220>
<223> Synthetic Construct

<400> 380
ctgatctcct gggaagaaa 19

<210> 381
<211> 19
<212> DNA
<213> Artificial Sequence

<220>
<223> Synthetic Construct

<400> 381
ttcctgctct ttgtccgct 19

<210> 382
<211> 19

<212> DNA
<213> Artificial Sequence

<220>
<223> Synthetic Construct

<400> 382
caggtcctat gcatcctca 19

<210> 383
<211> 19
<212> DNA
<213> Artificial Sequence

<220>
<223> Synthetic Construct

<400> 383
tccgctgatc tcctgggaa 19

<210> 384
<211> 19
<212> DNA
<213> Artificial Sequence

<220>
<223> Synthetic Construct

<400> 384
tagcatcgag tggtatttt 19

<210> 385
<211> 19
<212> DNA
<213> Artificial Sequence

<220>
<223> Synthetic Construct

<400> 385
gatctcctgg gaagaaagc 19

<210> 386
<211> 19
<212> DNA
<213> Artificial Sequence

<220>
<223> Synthetic Construct

<400> 386
tcaatttgcc tttgacctt 19

<210> 387
<211> 19
<212> DNA
<213> Artificial Sequence

<220>
<223> Synthetic Construct

<400> 387
aagctatgtt ggaccagaa 19

<210> 388
<211> 19
<212> DNA
<213> Artificial Sequence

<220>
<223> Synthetic Construct

<400> 388
ttgcctttga cctttctta 19

<210> 389
<211> 19
<212> DNA
<213> Artificial Sequence

<220>
<223> Synthetic Construct

<400> 389
ttgacctttc ttagggact 19

<210> 390
<211> 19
<212> DNA
<213> Artificial Sequence

<220>
<223> Synthetic Construct

<400> 390
tgatagtccc gttttcttc 19

<210> 391
<211> 19
<212> DNA
<213> Artificial Sequence

<220>
<223> Synthetic Construct

<400> 391
catctatgca gggctgtta 19

<210> 392
<211> 19
<212> DNA
<213> Artificial Sequence

<220>
<223> Synthetic Construct

<400> 392
tctcttcttt gaaagctca 19

<210> 393
<211> 19
<212> DNA
<213> Artificial Sequence

<220>
<223> Synthetic Construct

<400> 393
agctatgttg gaccagaag 19

<210> 394
<211> 19
<212> DNA
<213> Artificial Sequence

<220>
<223> Synthetic Construct

<400> 394
tttctgcttt tcctgctct 19

<210> 395
<211> 19
<212> DNA
<213> Artificial Sequence

<220>
<223> Synthetic Construct

<400> 395
ttttctgctt ttcctgctc 19

<210> 396
<211> 19
<212> DNA
<213> Artificial Sequence

<220>
<223> Synthetic Construct

<400> 396
cgctgatctc ctgggaaga 19

<210> 397
<211> 19
<212> DNA
<213> Artificial Sequence

<220>
<223> Synthetic Construct

<400> 397
gctatgttgg accagaagt 19

<210> 398
<211> 19
<212> DNA
<213> Artificial Sequence

<220>
<223> Synthetic Construct

<400> 398
tgtagcatcg agtggtatt 19

<210> 399
<211> 19
<212> DNA
<213> Artificial Sequence

<220>
<223> Synthetic Construct

<400> 399
cgaaaaggac accgtttca 19

<210> 400
<211> 19
<212> DNA
<213> Artificial Sequence

<220>
<223> Synthetic Construct

<400> 400
agcttccgaa aaggacacc 19

<210> 401
<211> 19
<212> DNA
<213> Artificial Sequence

<220>
<223> Synthetic Construct

<400> 401
cctgggaaga aagcttccg 19

<210> 402
<211> 19
<212> DNA
<213> Artificial Sequence

<220>
<223> Synthetic Construct

<400> 402
ttttcctgct ctttgtccg 19

<210> 403
<211> 19

<212>  DNA
<213>  Artificial Sequence

<220>
<223>  Synthetic Construct

<400>  403
tgctctttgt ccgctgatc                                                    19

<210>  404
<211>  19
<212>  DNA
<213>  Artificial Sequence

<220>
<223>  Synthetic Construct

<400>  404
ttggaccaga agtaaagtg                                                    19

<210>  405
<211>  19
<212>  DNA
<213>  Artificial Sequence

<220>
<223>  Synthetic Construct

<400>  405
ctttgtccgc tgatctcct                                                    19

<210>  406
<211>  19
<212>  DNA
<213>  Artificial Sequence

<220>
<223>  Synthetic Construct

<400>  406
cttgtctcaa tttgccttt                                                    19

<210>  407
<211>  19
<212>  DNA
<213>  Artificial Sequence

<220>
<223>  Synthetic Construct

<400>  407
cacccggtct tcttgccct                                                    19

<210>  408
<211>  19
<212>  DNA
<213>  Artificial Sequence

<220>
<223> Synthetic Construct

<400> 408
ctttgacctt tcttaggga 19

<210> 409
<211> 19
<212> DNA
<213> Artificial Sequence

<220>
<223> Synthetic Construct

<400> 409
cctttgacct ttcttaggg 19

<210> 410
<211> 19
<212> DNA
<213> Artificial Sequence

<220>
<223> Synthetic Construct

<400> 410
tgggaagaaa gcttccgaa 19

<210> 411
<211> 19
<212> DNA
<213> Artificial Sequence

<220>
<223> Synthetic Construct

<400> 411
tctatgcagg gctgttatc 19

<210> 412
<211> 19
<212> DNA
<213> Artificial Sequence

<220>
<223> Synthetic Construct

<400> 412
aaagctatgt tggaccaga 19

<210> 413
<211> 19
<212> DNA
<213> Artificial Sequence

<220>
<223> Synthetic Construct

<400> 413
tctcaatttg cctttgacc 19

<210> 414
<211> 19
<212> DNA
<213> Artificial Sequence

<220>
<223> Synthetic Construct

<400> 414
tgcatcctca tctatgcag 19

<210> 415
<211> 19
<212> DNA
<213> Artificial Sequence

<220>
<223> Synthetic Construct

<400> 415
gaaaggttcc gaggggcca 19

<210> 416
<211> 19
<212> DNA
<213> Artificial Sequence

<220>
<223> Synthetic Construct

<400> 416
gccttcttgt ctcaatttg 19

<210> 417
<211> 19
<212> DNA
<213> Artificial Sequence

<220>
<223> Synthetic Construct

<400> 417
actcctccct tttaatatc 19

<210> 418
<211> 19
<212> DNA
<213> Artificial Sequence

<220>
<223> Synthetic Construct

<400> 418
gaagaaagct tccgaaaag 19

<210> 419
<211> 19
<212> DNA
<213> Artificial Sequence

<220>
<223> Synthetic Construct

<400> 419
tgacctttct tagggactt 19

<210> 420
<211> 19
<212> DNA
<213> Artificial Sequence

<220>
<223> Synthetic Construct

<400> 420
aagctcaaag ctatgttgg 19

<210> 421
<211> 19
<212> DNA
<213> Artificial Sequence

<220>
<223> Synthetic Construct

<400> 421
cagttccggg tttgcaccc 19

<210> 422
<211> 19
<212> DNA
<213> Artificial Sequence

<220>
<223> Synthetic Construct

<400> 422
tgcacccggt cttcttgcc 19

<210> 423
<211> 19
<212> DNA
<213> Artificial Sequence

<220>
<223> Synthetic Construct

<400> 423
cctcctgggt ttaacgcat 19

<210> 424
<211> 19

<212> DNA
<213> Artificial Sequence

<220>
<223> Synthetic Construct

<400> 424
gcttccgaaa aggacaccg 19

<210> 425
<211> 19
<212> DNA
<213> Artificial Sequence

<220>
<223> Synthetic Construct

<400> 425
ctcaatttgc ctttgacct 19

<210> 426
<211> 19
<212> DNA
<213> Artificial Sequence

<220>
<223> Synthetic Construct

<400> 426
ggtcctatgc atcctcatc 19

<210> 427
<211> 19
<212> DNA
<213> Artificial Sequence

<220>
<223> Synthetic Construct

<400> 427
gttttctgct tttcctgct 19

<210> 428
<211> 19
<212> DNA
<213> Artificial Sequence

<220>
<223> Synthetic Construct

<400> 428
ttgttttctg cttttcctg 19

<210> 429
<211> 19
<212> DNA
<213> Artificial Sequence

<220>
<223> Synthetic Construct

<400> 429
cagaagtaaa gtgttctcg 19

<210> 430
<211> 19
<212> DNA
<213> Artificial Sequence

<220>
<223> Synthetic Construct

<400> 430
tgcttttcct gctctttgt 19

<210> 431
<211> 19
<212> DNA
<213> Artificial Sequence

<220>
<223> Synthetic Construct

<400> 431
gctgatctcc tgggaagaa 19

<210> 432
<211> 19
<212> DNA
<213> Artificial Sequence

<220>
<223> Synthetic Construct

<400> 432
acaccgtttc aggggcgag 19

<210> 433
<211> 19
<212> DNA
<213> Artificial Sequence

<220>
<223> Synthetic Construct

<400> 433
gaaaaggaca ccgtttcag 19

<210> 434
<211> 19
<212> DNA
<213> Artificial Sequence

<220>
<223> Synthetic Construct

<400> 434
tttcttctca gcccaggtc 19

<210> 435
<211> 19
<212> DNA
<213> Artificial Sequence

<220>
<223> Synthetic Construct

<400> 435
tcatctatgc agggctgtt 19

<210> 436
<211> 19
<212> DNA
<213> Artificial Sequence

<220>
<223> Synthetic Construct

<400> 436
tgaggaaact cctcccttt 19

<210> 437
<211> 19
<212> DNA
<213> Artificial Sequence

<220>
<223> Synthetic Construct

<400> 437
aggggccatt gaggaaact 19

<210> 438
<211> 19
<212> DNA
<213> Artificial Sequence

<220>
<223> Synthetic Construct

<400> 438
tttgcctttg acctttctt 19

<210> 439
<211> 19
<212> DNA
<213> Artificial Sequence

<220>
<223> Synthetic Construct

<400> 439
ttgtccgctg atctcctgg 19

<210> 440
<211> 19
<212> DNA
<213> Artificial Sequence

<220>
<223> Synthetic Construct

<400> 440
ttctcagccc aggtcctat 19

<210> 441
<211> 19
<212> DNA
<213> Artificial Sequence

<220>
<223> Synthetic Construct

<400> 441
gtgccttctt gtctcaatt 19

<210> 442
<211> 19
<212> DNA
<213> Artificial Sequence

<220>
<223> Synthetic Construct

<400> 442
gggtttaacg cattgtgat 19

<210> 443
<211> 19
<212> DNA
<213> Artificial Sequence

<220>
<223> Synthetic Construct

<400> 443
tggaccagaa gtaaagtgt 19

<210> 444
<211> 19
<212> DNA
<213> Artificial Sequence

<220>
<223> Synthetic Construct

<400> 444
ttgtctcaat ttgcctttg 19

<210> 445
<211> 19

<212> DNA
<213> Artificial Sequence

<220>
<223> Synthetic Construct

<400> 445
gggaagaaag cttccgaaa 19

<210> 446
<211> 19
<212> DNA
<213> Artificial Sequence

<220>
<223> Synthetic Construct

<400> 446
gcccaggtcc tatgcatcc 19

<210> 447
<211> 19
<212> DNA
<213> Artificial Sequence

<220>
<223> Synthetic Construct

<400> 447
tttgcacccg gtcttcttg 19

<210> 448
<211> 19
<212> DNA
<213> Artificial Sequence

<220>
<223> Synthetic Construct

<400> 448
ctgggaagaa agcttccga 19

<210> 449
<211> 19
<212> DNA
<213> Artificial Sequence

<220>
<223> Synthetic Construct

<400> 449
ccagttccgg gtttgcacc 19

<210> 450
<211> 19
<212> DNA
<213> Artificial Sequence

<220>
<223> Synthetic Construct

<400> 450
gttttcttct cagcccagg 19

<210> 451
<211> 19
<212> DNA
<213> Artificial Sequence

<220>
<223> Synthetic Construct

<400> 451
tcttgtctca atttgcctt 19

<210> 452
<211> 19
<212> DNA
<213> Artificial Sequence

<220>
<223> Synthetic Construct

<400> 452
gtcctatgca tcctcatct 19

<210> 453
<211> 19
<212> DNA
<213> Artificial Sequence

<220>
<223> Synthetic Construct

<400> 453
ttccgaaaag gacaccgtt 19

<210> 454
<211> 19
<212> DNA
<213> Artificial Sequence

<220>
<223> Synthetic Construct

<400> 454
ttttcttctc agcccaggt 19

<210> 455
<211> 19
<212> DNA
<213> Artificial Sequence

<220>
<223> Synthetic Construct

<400> 455
gcattgtgat agtcccgtt 19

<210> 456
<211> 19
<212> DNA
<213> Artificial Sequence

<220>
<223> Synthetic Construct

<400> 456
acttgaaagg ttccgaggg 19

<210> 457
<211> 19
<212> DNA
<213> Artificial Sequence

<220>
<223> Synthetic Construct

<400> 457
ggttccgagg ggccattga 19

<210> 458
<211> 19
<212> DNA
<213> Artificial Sequence

<220>
<223> Synthetic Construct

<400> 458
ccttcttgtc tcaatttgc 19

<210> 459
<211> 19
<212> DNA
<213> Artificial Sequence

<220>
<223> Synthetic Construct

<400> 459
tgggtttaac gcattgtga 19

<210> 460
<211> 19
<212> DNA
<213> Artificial Sequence

<220>
<223> Synthetic Construct

<400> 460
tttgaccttt cttagggac 19

<210> 461
<211> 19
<212> DNA
<213> Artificial Sequence

<220>
<223> Synthetic Construct

<400> 461
gtgatagtcc cgttttctt 19

<210> 462
<211> 19
<212> DNA
<213> Artificial Sequence

<220>
<223> Synthetic Construct

<400> 462
tacaggtgcc ttcttgtct 19

<210> 463
<211> 19
<212> DNA
<213> Artificial Sequence

<220>
<223> Synthetic Construct

<400> 463
tgtgatagtc ccgttttct 19

<210> 464
<211> 19
<212> DNA
<213> Artificial Sequence

<220>
<223> Synthetic Construct

<400> 464
cagggctgtt atctgcata 19

<210> 465
<211> 19
<212> DNA
<213> Artificial Sequence

<220>
<223> Synthetic Construct

<400> 465
ccgtttcagg ggcgagtga 19

<210> 466
<211> 19

<212>  DNA
<213>  Artificial Sequence

<220>
<223>  Synthetic Construct

<400>  466
gccattgagg aaactcctc                                                    19

<210>  467
<211>  19
<212>  DNA
<213>  Artificial Sequence

<220>
<223>  Synthetic Construct

<400>  467
ccattgagga aactcctcc                                                    19

<210>  468
<211>  19
<212>  DNA
<213>  Artificial Sequence

<220>
<223>  Synthetic Construct

<400>  468
ctcagcccag gtcctatgc                                                    19

<210>  469
<211>  19
<212>  DNA
<213>  Artificial Sequence

<220>
<223>  Synthetic Construct

<400>  469
tgatctcctg ggaagaaag                                                    19

<210>  470
<211>  19
<212>  DNA
<213>  Artificial Sequence

<220>
<223>  Synthetic Construct

<400>  470
aatgtagcat cgagtggta                                                    19

<210>  471
<211>  19
<212>  DNA
<213>  Artificial Sequence

<220>
<223> Synthetic Construct

<400> 471
cttctttgaa agctcaaag 19

<210> 472
<211> 19
<212> DNA
<213> Artificial Sequence

<220>
<223> Synthetic Construct

<400> 472
aaggacaccg tttcagggg 19

<210> 473
<211> 19
<212> DNA
<213> Artificial Sequence

<220>
<223> Synthetic Construct

<400> 473
ccgaaaagga caccgtttc 19

<210> 474
<211> 19
<212> DNA
<213> Artificial Sequence

<220>
<223> Synthetic Construct

<400> 474
aacttgaaag gttccgagg 19

<210> 475
<211> 19
<212> DNA
<213> Artificial Sequence

<220>
<223> Synthetic Construct

<400> 475
ccagaagtaa agtgttctc 19

<210> 476
<211> 19
<212> DNA
<213> Artificial Sequence

<220>
<223> Synthetic Construct

<400> 476
gtcccgtttt cttctcagc 19

<210> 477
<211> 19
<212> DNA
<213> Artificial Sequence

<220>
<223> Synthetic Construct

<400> 477
cctcatctat gcagggctg 19

<210> 478
<211> 19
<212> DNA
<213> Artificial Sequence

<220>
<223> Synthetic Construct

<400> 478
gaaagcttcc gaaaaggac 19

<210> 479
<211> 19
<212> DNA
<213> Artificial Sequence

<220>
<223> Synthetic Construct

<400> 479
tcctgctctt tgtccgctg 19

<210> 480
<211> 19
<212> DNA
<213> Artificial Sequence

<220>
<223> Synthetic Construct

<400> 480
cctttcttag ggacttgtt 19

<210> 481
<211> 19
<212> DNA
<213> Artificial Sequence

<220>
<223> Synthetic Construct

<400> 481
cttagggact tgttttctg 19

<210> 482
<211> 19
<212> DNA
<213> Artificial Sequence

<220>
<223> Synthetic Construct

<400> 482
aaaacctcct gggtttaac 19

<210> 483
<211> 19
<212> DNA
<213> Artificial Sequence

<220>
<223> Synthetic Construct

<400> 483
aggacaccgt ttcaggggc 19

<210> 484
<211> 19
<212> DNA
<213> Artificial Sequence

<220>
<223> Synthetic Construct

<400> 484
agaaagcttc cgaaaagga 19

<210> 485
<211> 19
<212> DNA
<213> Artificial Sequence

<220>
<223> Synthetic Construct

<400> 485
aggaaactcc tccctttta 19

<210> 486
<211> 19
<212> DNA
<213> Artificial Sequence

<220>
<223> Synthetic Construct

<400> 486
cgaggggcca ttgaggaaa 19

<210> 487
<211> 19

<212> DNA
<213> Artificial Sequence

<220>
<223> Synthetic Construct

<400> 487
cctgggttta acgcattgt 19

<210> 488
<211> 19
<212> DNA
<213> Artificial Sequence

<220>
<223> Synthetic Construct

<400> 488
ctttgaaagc tcaaagcta 19

<210> 489
<211> 19
<212> DNA
<213> Artificial Sequence

<220>
<223> Synthetic Construct

<400> 489
agaagtaaag tgttctcgt 19

<210> 490
<211> 19
<212> DNA
<213> Artificial Sequence

<220>
<223> Synthetic Construct

<400> 490
atgcatcctc atctatgca 19

<210> 491
<211> 19
<212> DNA
<213> Artificial Sequence

<220>
<223> Synthetic Construct

<400> 491
cttgaaaggt tccgagggg 19

<210> 492
<211> 19
<212> DNA
<213> Artificial Sequence

<220>
<223> Synthetic Construct

<400> 492
gcttttcctg ctctttgtc 19

<210> 493
<211> 1000
<212> DNA
<213> Artificial Sequence

<220>
<223> Synthetic Construct

<400> 493
ttgaacccca taacatttca acgaattcct catcctttct gtgaatcaag agcctgaaaa 60

gaaatggtga aataatatga tcctctcttc tttgaaagct caaagctatg ttggaccaga 120

agtaaagtgt tctcgtttct atttaataac ttgaaaggtt ccgaggggcc attgaggaaa 180

ctcctccctt ttaatatcaa tgtgtattta ttgcaaaaat aatgtagcat cgagtggtat 240

tttatagctt atccaaaaac ctcctgggtt taacgcattg tgatagtccc gttttcttct 300

cagcccaggt cctatgcatc ctcatctatg cagggctgtt atctgcatat aatttttttt 360

tttttaaga caaagtcttg ctctgtcgcc ccggctggag tgcagtggtg caatctcggc 420

tcactgcaac ctccgcctcc caggttcaag cggttcttcc gcctcagcct accgagtagc 480

tgggactaca ggcatgcgcc accacaccta ggtgattttt gtatttttag tagagacagg 540

ggtttcacca tgttgaccag gctggtctcg aactcctgat ctcaagcgat ccacccgcct 600

cagcctccca aagtgctggg attacaggca taagccacta cgcccggcct caattttgta 660

ttgtactttt tctttctttc tttaatagag acagggtctc actatgttga ctaggttggt 720

ctagaactcc tgggcacaag ctgtccgccc gcttctgcct cccaaagtgc tgggattgca 780

ggcgtgaacc accgcccctg gctacaggtg ccttcttgtc tcaatttgcc tttgaccttt 840

cttagggact tgttttctgc ttttcctgct ctttgtccgc tgatctcctg ggaagaaagc 900

ttccgaaaag gacaccgttt caggggcgag tgacgccggg gtgcccaggc cgcgccccag 960

ttccgggttt gcacccggtc ttcttgccct gccccgcccg 1000

<210> 494
<211> 20
<212> DNA
<213> Artificial Sequence

<220>
<223> Synthetic Construct

<400> 494
aaggcgcttg agtatgcctg 20

<210> 495
<211> 20
<212> DNA
<213> Artificial Sequence

<220>
<223> Synthetic Construct

<400> 495
gtgggcttcc actcctatcg 20

<210> 496
<211> 21
<212> DNA
<213> Artificial Sequence

<220>
<223> Synthetic Construct

<400> 496
atggacagga ctgaacgtct t 21

<210> 497
<211> 20
<212> DNA
<213> Artificial Sequence

<220>
<223> Synthetic Construct

<400> 497
tccagcaggt cagcaaagaa 20

<210> 498
<211> 20
<212> DNA
<213> Artificial Sequence

<220>
<223> Synthetic Construct

<400> 498
ataatcccaa gcggtttgct 20

<210> 499
<211> 20
<212> DNA
<213> Artificial Sequence

<220>
<223> Synthetic Construct

<400> 499
ctgccagtct ggactgttct 20

<210> 500
<211> 74
<212> DNA

<213> Artificial Sequence

<220>
<223> Synthetic Construct

<400> 500
tctcttcttt gaaagctcaa agctatgttg gaccagaagt aaagtgttct cgtttctatt 60

taataacttg aaag 74

<210> 501
<211> 36
<212> DNA
<213> Artificial Sequence

<220>
<223> Synthetic Construct

<400> 501
gttttcttct cagcccaggt cctatgcatc ctcatc 36

<210> 502
<211> 31
<212> DNA
<213> Artificial Sequence

<220>
<223> Synthetic Construct

<400> 502
tacaggtgcc ttcttgtctc aatttgcctt t 31

<210> 503
<211> 124
<212> DNA
<213> Artificial Sequence

<220>
<223> Synthetic Construct

<400> 503
ttgttttctg cttttcctgc tctttgtccg ctgatctcct gggaagaaag cttccgaaaa 60

ggacaccgtt tcaggggcga gtgacgccgg ggtgcccagg ccgcgcccca gttccgggtt 120

tgca 124

<210> 504
<211> 125
<212> DNA
<213> Artificial Sequence

<220>
<223> Synthetic Construct

<400> 504
aggttccgag gggccattga ggaaactcct ccccttttaat atcaatgtgt atttattgca 60

aaaataatgt agcatcgagt ggtattttat agcttatcca aaaacctcct gggtttaacg 120

cattg 125

<210> 505
<211> 21
<212> DNA
<213> Artificial Sequence

<220>
<223> Synthetic Construct

<220>
<221> misc_feature
<222> (20)..(21)
<223> deoxythymidine (dT) base

<400> 505
acuacugagu gacaguagat t 21

<210> 506
<211> 21
<212> DNA
<213> Artificial Sequence

<220>
<223> Synthetic Construct

<220>
<221> misc_feature
<222> (20)..(21)
<223> deoxythymidine (dT) base

<400> 506
ucuacuguca cucaguagut t 21

<210> 507
<211> 21
<212> DNA
<213> Artificial Sequence

<220>
<223> Synthetic Construct

<220>
<221> misc_feature
<222> (20)..(21)
<223> deoxythymidine (dT) base

<400> 507
gaaguucaga gccgcucaat t 21

<210> 508
<211> 21
<212> DNA
<213> Artificial Sequence

```
<220>
<223>  Synthetic Construct


<220>
<221>  misc_feature
<222>  (20)..(21)
<223>  deoxythymidine (dT) base

<400>  508
uugagcggcu cugaacuuct t                                                21
```

**Description**

TECHNICAL FIELD

The present invention belongs to the technical field of nucleic acids, and more particularly it relates to oligomeric nucleic acids associated with gene activation, e.g., small activating nucleic acid molecules, uses of the small activating nucleic acid molecules in activating/up-regulating the transcription of histidine phosphatase LHPP (Phospholysine phosphohistidine inorganic pyrophosphate phosphatase) gene, and uses thereof in treating LHPP deficiency-associated diseases, such as tumors.

BACKGROUND

The histidine phosphatase LHPP (Phospholysine phosphohistidine inorganic pyrophosphate phosphatase) gene on the chromosome 10q26.13 of the human genome has seven exons, and can be transcribed to produce nine LHPP splice variants. LHPP is expressed in various human tissues, including liver, kidney, brain tissues, and the like (1-3).

Multiple genome-wide association studies (GWAS) have showed that LHPP is a risk factor in causing severe depression (2; 4-7) and cancer (8-10). A recent study on hepatocellular carcinoma (HCC) showed that LHPP was proved to be histidine phosphatase with a tumor suppressor function (11). Using a hepatocellular carcinoma mouse model (mTOR-driven), this study showed an significant increase in histidine phosphorylation levels in the tumor tissues during the tumor formation. A proteome analysis showed increased expression levels of histidine kinase NME1 (Nucleoside diphosphate kinase A) and histidine kinase NME2 (Nucleoside diphosphate kinase B) in tumor tissue, with decreased expression level of histidine phosphatase LHPP. Interestingly, after the LHPP gene was introduced into the hepatocellular carcinoma mouse model, tumor weight was reduced and liver function was protected. Furthermoreaccording to an analysis of LHPP expression in a liver cancer patient sample, LHPP expression was negatively correlated with tumor severity and positively correlatedwith the overall survival rate of the patient (11). Therefore, LHPP has the potential as a therapeutic target for hepatocellular carcinoma.

China is a country with the prevalence of liver cancer and more than half of the cases of HCC worldwide occur in China (12). However, there are few approaches to treat liver cancer and a limited curative effect exists. Therefore, there is an urgent need to develop an innovative drug. The present invention provides a highly specific, small activating RNA capable of continuously activating/upregulating LHPP transcription, which can in turn increase LHPP protein expression and have a remarkable tumor inhibition effect as shown by in *in-vitro* and *in-vivo* studies. The small activating RNA is expected to become an effective treatment for cancers.

SUMMARY

**Disclosure**

One objective of the present invention is to provide small activating nucleic acid molecules, which can increase the expression of LHPP protein by activating/up-regulating the transcription of the LHPP gene via an RNA activation process to thereby treat an LHPP deficiency-associated disease, such as tumors.

Another objective of the present invention is to provide compositions and formulations comprising the small activating nucleic acid molecules with the tumor inhibitory activity.

Yet another objective of the present invention is to provide a use of the small activating nucleic acid molecule with the tumor inhibitory activityor the compositions or formulationscomprising the same in preparing a medicament for activating/up-regulating the expression of the LHPP gene in a cell.

Still yet another objective of the present invention is to provide a method for activating/up-regulating the expression of LHPP gene in a cell.

Still another objective of the present invention is to provide the use of the small activating nucleic acid molecule with the tumor inhibitory activity or the compositions or formulations comprising the same in preparing a therapeutic for treating a disease or condition,e.g., tumors, which is related to LHPP deficiency or insufficiency, or a method for treating a disease or condition,e.g., tumors, related to LHPP deficiency or insufficiency.

Another objective of the present invention is to provide an isolated target site of a small activating nucleic acid molecule on an LHPP gene, wherein the target site comprises or is selected from any sequence of continuous 16 to 35 nucleotides in any of the sequences set forth in SEQ ID NOs: 500-504.

**Technical Solution**

In one aspect of the present invention, a small activating nucleic acid molecule is provided, which can activate/up-regulate the expression of LHPP gene in a cell. One strand of the small activating nucleic acid molecule has at least 75% homology or complementarity to a nucleic acid sequence of 16 to 35 nucleotides in length in a promoter region of LHPP gene, thereby activating or up-regulating the expression of the gene, wherein the promoter region comprises 1000 nucleotides upstream of a transcription start site. Specifically, one strand of the small activating nucleic acid molecule comprises or is selected from a nucleic acid sequence having at least 75%, e.g., at least about 79%, about 80%, about 85%, about 90%, about 95%, about 99% or 100%, homology or complementarity to a sequence of continuous 16 to 35 nucleotides in positions -917 to -844 (H1, SEQ ID NO: 500), positions -710 to -675 (H2, SEQ ID NO: 501), positions -198 to -168 (H3, SEQ ID NO: 502), positions -151 to -28 (H4, SEQ ID NO: 503) or positions -845 to -711 (H5, SEQ ID NO: 504) upstream of the transcription start site in the LHPP gene promoter. More specifically, one strand of the small activating nucleic acid molecule of the present invention has at least 75%, e.g., at least about 79%, about 80%, about 85%, about 90%, about 95%, about 99% or about 100%, homology or complementarity to any nucleotide sequence selected from SEQ ID NOs: 329-492. In one specific embodiment, one strand of the small activating nucleic acid molecule of the present invention comprises a nucleic acid sequence having at least 75%, e.g., at least about 79%, about 80%, about 85%, about 90%, about 95%, about 99% or about 100% homology or complementarity to any nucleotide sequence selected from SEQ ID NOs: 329-492. In another embodiment, one strand of the small activating nucleic acid molecule of the present invention consists of a nucleic acid sequence having at least 75%, e.g., at least about 79%, about 80%, about 85%, about 90%, about 95%, about 99% or about 100%, homology or complementarity to any nucleotide sequence selected from SEQ ID NOs: 329-492.

The small activating nucleic acid molecule of the present invention comprises a double-stranded small activating nucleic acid molecule targeting the promoter region of LHPP gene comprising a first nucleic acid strand and a second nucleic acid strand, wherein the first nucleic acid strand has at least 75% homology or complementarity to any sequence of 16 to 35 continuous nucleotides in positions -917 to -844 (SEQ ID NO: 500), positions -710 to -675 (SEQ ID NO: 501), positions -198 to -168 (SEQ ID NO: 502), positions -151 to -28 (SEQ ID NO: 503) or positions -845 to -711 (SEQ ID NO: 504) upstream of the transcription start site in the LHPP gene promoter, and the first nucleic acid strand and the second nucleic acid strand can complementarily form a double-stranded nucleic acid structure capable of activating the expression of LHPP gene in a cell.

The first nucleic acid strand and the second nucleic acid strand of the small activating nucleic acid molecule of the present invention can be present either on two different nucleic acid strands or on the same nucleic acid strand. When the first nucleic acid strand and the second nucleic acid strand are located on two different strands, at least one strand of the small activating nucleic acid molecule can have overhangs at the 5' terminus and/or the 3' terminus, e.g. overhangs of 0 to 6 nucleotides in length at 3' terminus, such as overhangs of 0, 1, 2, 3, 4, 5 or 6 nucleotides in length. Preferably, both strands of the small activating nucleic acid molecule of the present invention have overhangs; more preferably, the 3' terminus of both strands of the small activating nucleic acid molecule can have overhangs of 0 to 6 nucleotides in length, e.g., overhangs of 0, 1, 2, 3, 4, 5 or 6 nucleotides in length; and most preferably overhangs of 2 or 3 nucleotides in length. Preferably, the nucleotide of the overhang can be dT.

The small activating nucleic acid molecule of the present invention can also comprise a small activating nucleic acid molecule capable of forming a double-stranded region hairpin structure, e.g., a single-stranded small activating RNA molecule. In one embodiment, the small activating nucleic acid molecule of the present invention comprises a single-stranded small activating RNA molecule targeting the promoter region of LHPP gene, wherein the single-stranded small activating nucleic acid molecule can form a double-stranded region hairpin structure. When the first nucleic acid strand and the second nucleic acid strand are present on the same nucleic acid strand, preferably, the small activating nucleic acid molecule of the present invention can be a hairpin single-stranded nucleic acid molecule, wherein the first nucleic acid strand and the second nucleic acid strand have complementary regions capable of forming a double-stranded nucleic acid structure, and the double-stranded nucleic acid structure can promote the expression of LHPP gene in a cell with, for example, a RNA activation mechanism.

In the aforementioned small activating nucleic acid molecule, the first nucleic acid strand and the second nucleic acid strand can have 16 to 35 nucleotides, e.g., 16, 17, 18,19, 20, 21, 22, 23, 24, 25, 26, 27, 28, 29, 30, 31, 32, 33, 34 or 35 nucleotides in length.

In one embodiment, the first nucleic acid strand of the small activating nucleic acid molecule of the present invention has at least 75%, e.g., at least about 79%, about 80%, about 85%, about 90%, about 95%, about 99% or about 100%, identity or homology to any nucleotide sequence selected from SEQ ID NOs: 1-164, and the second nucleic acid strand of the small activating nucleic acid molecule has at least 75%, e.g., at least about 79%, about 80%, about 85%, about 90%, about 95%, about 99% or about 100%, identity or homology to any nucleotide sequence selected from SEQ ID NOs: 165-328. In one embodiment, the first nucleic acid strand of the small activating nucleic acid molecule of the present invention comprises a nucleic acid sequence having at least 75%, e.g., at least about 79%, about 80%, about 85%, about 90%, about 95%, about 99% or about 100%, identity or homology to any nucleotide sequence selected from SEQ ID NOs: 1-164, or consists of a nucleic acid sequence having at least 75%, e.g., at least about 79%, about 80%, about 85%, about 90%, about 95%, about 99% or about 100%, identity or homology to any nucleotide sequence selected from SEQ ID NOs: 1-164; and the second nucleic acid strand of the small activating nucleic acid molecule of the present invention comprises a nucleic acid sequence having at least 75%, e.g., at least about 79%, about 80%, about 85%, about 90%, about 95%, about 99% or about 100%, identity or homology to any nucleotide sequence selected from SEQ ID NOs: 165-328, or consists of a nucleic acid sequence having at least 75%,e.g., at least about 79%, about 80%, about 85%, about 90%, about 95%, about 99% or about 100%, identity or homology to any nucleotide sequence selected from SEQ ID NOs: 165-328. In a specific embodiment, the first nucleic acid strand of the small activating nucleic acid molecule of the present invention can comprise or be selected from any nucleotide sequence set forth in SEQ ID NOs: 1-164, and the second strand can comprise or be selected from any nucleotide sequence set forth in SEQ ID NOs: 165-328. In one embodiment, the small activating nucleic acid molecule described herein can be synthesized, transcribed *in vitro* or expressed by a vector.

All the nucleotides in the small activating nucleic acid molecule described herein can be natural non-chemically modified nucleotides or can comprise at least one modification. In one embodiment, the modification in the small activating nucleic acid molecule described herein can be chemical modification, for example, at least one nucleotide can have chemical modification, and the chemical modification used in the present invention can comprise or be selected from one or more or any combination of the following modifications:

(1) modification of a phosphodiester bond of nucleotides in the nucleotide sequence of the small activating nucleic acid molecule;
(2) modification of 2'-OH of a ribose in the nucleotide sequence of the small activating nucleic acid molecule; and
(3) modification of a base in the nucleotide sequence of the small activating nucleic acid molecule;
(4) at least one nucleotide in the nucleotide sequence of the small activating nucleic acid molecule being a locked nucleic acid.

The chemical modification described herein is well-known to those skilled in the art, and the modification of the phosphodiester bond refers to the modification of oxygen in the phosphodiester bond including, but not limited to phosphorothioate modification and boranophosphate modification. Both modifications can stabilize an saRNA structure and maintain high specificity and high affinity for base pairing.

The ribose modification refers to the modification of 2'-OH in pentose of a nucleotide, i.e., the introduction of some substituents into hydroxyl positions of the ribose, for example, including, but not limited to 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, *etc.*

The base modification refers to the modification of the base of a nucleotide, for example, including, but not limited to 5'-bromouracil modification, 5'-iodouracil modification, N-methyluracil modification, 2,6-diaminopurine modification, *etc.*

These modifications can increase the bioavailability of the small activating nucleic acid molecule, improve affinity to a target sequence, and enhance resistance to nuclease hydrolysis in a cell.

In addition, in order to promote the access of the small activating nucleic acid molecule into a cell, on the basis of the aforementioned modifications, a lipophilic group (such as cholesterol) can be introduced into the terminus of the first nucleic acid strand or the second nucleic acid strand of the small activating nucleic acid molecule to facilitate the interaction with the gene promoter region in the cell nucleus through the cell membrane and nuclear membrane composed of lipid bilayers.

After contacting a cell, the small activating nucleic acid molecule provided by the present invention can effectively activate or up-regulate the expression of the LHPP gene in the cell, preferably up-regulate the expression by at least 10%.

Another aspect of the present invention relates to a nucleic acid coding the small activating nucleic acid molecule described herein. In one embodiment, the nucleic acid can be a DNA molecule.

Another aspect of the present invention provides a cell comprising the aforementioned small activating nucleic acid molecule or the nucleic acid coding the small activating nucleic acid molecule described herein. In one embodiment, the small activating nucleic acid molecule of the present invention can be a double-stranded small activating nucleic acid molecule targeting the promoter region of LHPP gene, comprising a first nucleic acid strand and a second nucleic acid strand. In another embodiment, the small activating nucleic acid molecule of the present invention can be a single-stranded small activating nucleic acid molecule targeting the promoter region of LHPP gene.

Another aspect of the present invention provides a composition, e.g., a pharmaceutical composition. The composition comprises the small activating nucleic acid molecule of the present invention or the nucleic acid coding the small activating nucleic acid molecule described herein and optionally, a pharmaceutically acceptable carrier. In one embodiment, the pharmaceutically acceptable carrier can comprise or be chosen from a liposome, a high-molecular polymer, and a polypeptide. In one embodiment, the composition of the present invention contains 1 nM to 150 nM, e.g., 1 nM to 100 nM, such as 1 nM to 50 nM, e.g., 10 nM, 20 nM, 30 nM, 40 nM, 50 nM, 60 nM, 70 nM, 80 nM, 90 nM, 100 nM, 110 nM, 120 nM, 130 nM, 140 nM or 150 nM of small activating nucleic acid molecule of the present invention. In another embodiment, the composition of the present invention can further comprise other compounds, such as a low-molecular-weight compound for tumor treatment. In one embodiment, the low-molecular-weight compound for tumor treatment can comprise multi-target anti-tumor drugs (e.g., Sorafenib (targets: PDGFR, KIT and RAF) (SELLECK, S1040)), tyrosine kinase inhibitors (e.g., Lenvatinib (targets: FGFR, VEGFR2, PDGFR and KIT) (SELLECK, S1164)), kinase inhibitors (e.g., Regorafenib (targets: FGFR, VEGFR2, PDGFR, KIT and RAF) (SELLECK, S1178)), or Cabozantini (inhibiting MET, VEGFR2 and RET signal transduction) (SELLECK, S1119).

In another aspect of the present invention, a kit is provided, which comprises the aforementioned small activating nucleic acid molecule, the nucleic acid coding the small activating nucleic acid molecule described herein, the cell comprising the small activating nucleic acid molecule of the present invention or the nucleic acid coding the small activating nucleic acid molecule of the present invention, or the composition comprising the small activating nucleic acid molecule of the present invention.

Another aspect of the present invention relates to a use of the small activating nucleic acid molecule of the present invention, the nucleic acid coding the small activating nucleic acid molecule of the present invention, the cell comprising the small activating nucleic acid molecule of the present invention and the nucleic acid coding the small activating nucleic acid molecule of the present invention or the composition comprising the small activating nucleic acid molecule of the present invention in preparing a drug or formulation for activating/up-regulating the expression of LHPP gene in a cell.

Another aspect of the present invention also relates to a method for activating/up-regulating the expression of LHPP gene in a cell. The method comprises administering the small activating nucleic acid molecule of the present invention, the nucleic acid coding the small activating nucleic acid molecule of the present invention or the composition comprising the small activating nucleic acid molecule of the present invention to the cell.

The small activating nucleic acid molecule of the present invention can be directly introduced into a cell or it can be produced in the cell after a nucleotide sequence coding the small activating nucleic acid molecule of the present invention is introduced into the cell. The cell is preferably a mammalian cell, more preferably a human cell. The aforementioned cell can be *in vitro,* such as a cell line or a cell strain, or can be present in a mammalian body, such as a human body. The human body can be a patient suffering from a disease or condition related to insufficient or decreased expression of LHPP protein. The small activating nucleic acid molecule of the present invention can be administered at a sufficient dose to treat the disease or condition related to a deficiency in the amount of LHPP protein or insufficient or decreased expression of LHPP protein. Specifically, the disease or condition related to a deficiency in the amount of LHPP protein or insufficient or decreased expression of LHPP protein can comprise, for example, tumors, e.g., solid tumors, and the solid tumors can comprise, for example, liver cancer, lung cancer, bladder cancer, prostatic cancer, glioma, *etc.*

Another aspect of the present invention provides an isolated small activating nucleic acid molecule targeting site of an LHPP gene, which has any sequence of 16 to 35 continuous nucleotides in the promoter region of the LHPP gene, and preferably, the acting site comprises or is chosen from any sequence of 16 to 35 continuous nucleotides in any of the sequences set forth in SEQ ID NOs: 500-504. Specifically, the targeting site can comprise or be chosen from any nucleotide sequence set forth in SEQ ID NOs: 329-492.

Another aspect of the present invention relates to a method for treating a disease or condition related to insufficient or decreased expression of LHPP protein in a subject, which comprises administering the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention at a therapeutically effective amount to the subject. In one embodiment, the method for treating a disease or condition related to insufficient or decreased expression of LHPP protein in a subject in the present invention comprises administering the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention and a low-molecular-weight compound, antibody, polypeptide or protein at therapeutically effective amounts to the subject. The subject may be a mammal, such as a human. In one embodiment, the disease or condition related to insufficient or decreased expression of LHPP protein may comprise, for example, tumors, e.g., solid tumors, and the solid tumors may comprise, for example, liver cancer, lung cancer, bladder cancer, prostatic cancer, glioma, etc.

Another aspect of the present invention relates to a use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention in preparing a drug for treating a disease or condition related to insufficient or decreased expression of LHPP protein. The subject may be a mammal, such as a human. In one embodiment, the disease related to insufficient or decreased expression of LHPP protein may comprise, for example, tumors, e.g., solid tumors, and the solid tumors may comprise, for example, liver cancer, lung cancer, bladder cancer, prostatic cancer, glioma, *etc.*

In one embodiment, provided is a use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention in preparing a drug for treating tumors, such as solid tumors, wherein the solid tumors can comprise, for example, liver cancer, lung cancer, bladder cancer, prostatic cancer, glioma, *etc.*

Another aspect of the present invention relates to a use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention and a chemotherapeutic agent, radiotherapy, cell therapy, micromolecule, polypeptide, protein, antibody or other anti-tumor drugs in preparing a drug or pharmaceutical composition for treating a disease or condition related to insufficient or decreased expression of LHPP protein.

In one embodiment, provided is a use of the small activating nucleic acid molecule of the present invention, the nucleic acid coding the small activating nucleic acid molecule of the present invention, the cell comprising the small activating nucleic acid molecule of the present invention or the nucleic acid coding the small activating nucleic acid molecule of the present invention or the composition comprising the small activating nucleic acid molecule of the present invention and a chemotherapeutic agent, radiotherapy, cell therapy, micromolecule, polypeptide, protein, antibody or other anti-tumor drugs in preparing a drug or pharmaceutical composition for treating tumors, such as solid tumors, wherein the solid tumors can comprise, for example, liver cancer, lung cancer, bladder cancer, prostatic cancer, glioma, *etc.* In one embodiment, the chemotherapeutic agent comprises or is chosen from Sorafenib, Lenvatinib, Regorafenib and Cabozantinib.

Further embodiments include methods and uses of the small activating nucleic acid molecule of the present invention in combination with a chemotherapeutic agent, radiotherapy, cell therapy, micromolecule, polypeptide, protein, antibody, or other anti-tumor drugs, and the chemotherapeutic agent is preferably selected from Sorafenib, Lenvatinib, Regorafenib and Cabozantinib.

**Advantages of the Present Invention**

The small activating nucleic acid molecule capable of activating/up-regulating the expression of LHPP gene provided by the present invention can permanently activate LHPP gene, therefore efficiently and specifically up-regulating or restoring the expression of LHPP gene and protein while featuring lower toxic and side effects, and it can be used in preparing a drug or formulation for a disease or symptom related to insufficient or decreased expression of LHPP protein. Moreover, the activating saRNA of LHPP shows a good synergistic effect when used in combination with other anti-tumor drugs, reflecting a synergistic effect in terms of anti-tumor effect.

## BRIEF DESCRIPTION OF THE DRAWINGS

**FIG. 1** is a schematic of LHPP gene. The drawing shows the LHPP gene structure and a 1 kb promoter region used for designing the saRNA, in which a 449 bp Alu repeat sequence is excluded.

**FIG. 2** shows changes in the expression levels of LHPP mRNA mediated by saRNA. 290 LHPP promoter-targeting saRNAs were individually transfected into Huh7 cells, and 72 hours later, the expression level of LHPP mRNA was analyzed by one-step RT-qPCR. The drawing shows changes in the expression level of LHPP caused by each saRNA relative to a control treatment (control, mock) ordered by the target positions of the saRNA in the promoter region from -917 to -28.

**FIG. 3** shows saRNA hot spot regions inLHPP promoter. 290 LHPP saRNAs promoter-targeting saRNAs were individually transfected into Huh7 cells, and 72 hours later, the expression level of LHPP mRNA was analyzed by one-step RT-qPCR. The drawing shows changes in the expression level of LHPP mRNA caused by each saRNA relative to a control treatment (control, mock) ordered by the target positions of the saRNA in the promoter region from -917 to -28 . The numbers above or below indicate the boundaries of the hot spot regions (relative to the LHPP transcription start site (TSS)).

**FIG. 4** shows a negative correlation between expression levels of LHPP mRNA and cell viability. 290 LHPP promoter-targeting saRNAs were individually transfected into Huh7 cells, and 72 hours later, one-step RT-qPCR was employed to analyze the expression level of LHPP mRNA and cell viability was detected by the CCK-8 method. The thin line represents relative LHPP mRNA expression level ($log_2$), and the thick line represents cell viability.

**FIG. 5** shows saRNAs inducing the expression of LHPP and inhibiting AKT phosphorylation. 10 LHPP promoter-targeting saRNAs were individually transfected into Huh7 cells and were analyzed 72 hours later. In **FIG. 5A,** the expression levels of LHPP mRNA was analyzed by RT-qPCR. (In **FIG.5B,** the protein levels of LHPP, pAKT and AKT were detected by a Western blot method.

**FIG. 6** shows saRNAs inducingthe mRNA expression of LHPP and inhibiting the proliferation of liver cancer cells. 8 LHPP promoter-targeting saRNAs were individually transfected into liver cancer cells at 10 nM for 72 h. In **FIG. 6A,** the expression level of LHPP mRNA was analyzed by RT-qPCR. In **FIG. 6B,** cell viability in the saRNA treatment group was evaluated by the CCK-8 method and recorded as a percentage relative to the viability of cells in a control (Mock) treatment group.

**FIG. 7** shows saRNAs inducing the expression levels of LHPP mRNA and inhibiting the proliferation of several other cancer cells. 8 LHPP promoter-targeting saRNAs were individually transfected into cancer cells at 10 nM for 72 h. In **FIG. 7A,** the expression level of LHPP mRNA was analyzed by RT-qPCR. In **FIG. 7B,** cell viability in the saRNA treatment group was evaluated by the CCK-8 method and recorded as a percentage relative to the viability of cells in a control (Mock) treatment group.

**FIG. 8** shows saRNA inhibiting the proliferation of HepG2 cells in combination with chemical drugs. The saRNAs were transfected into cancer cells at different concentrations in combination with a panel of chemical drugs. In **FIG. 8A,** cell viability in the saRNA treatment group was evaluated by the CCK-8 method and recorded as a percentage relative to the viability of cells in a control (Mock) treatment group. In **FIG. 8B,** Compusyn© version 1.0 software was used to draw a combination index graphs; and to calculate combination index values as shown in **FIG. 8C.**

**FIG. 9** shows saRNA inhibiting the proliferation of U87MG cells in combination with chemical drugs. The sRNAs were transfected into cancer cells at different concentrations in combination with different chemical drugs. In **FIG. 9A,** cell viability in the saRNA treatment group was evaluated by the CCK-8 method and recorded as a percentage relative to the viability of cells in a control (Mock) treatment group. In **FIG. 9B,** Compusyn© version 1.0 software was used to draw a combination index graphs; and to calculate combination index values as shown in **FIG. 9C.**

**FIG. 10** shows saRNA inhibiting the growth of a HepG2 transplanted tumor in combination with a chemical drug. The small activating RNA shown was injected into a tumor at a dose of 1 mg/kg in combination with a chemical drug. The volume change of the transplanted tumor was recorded during administration.

**FIG. 11** shows saRNA inhibiting the growth of a U87MG transplanted tumor in combination with a chemical drug. The saRNA was injected into a tumor at a dose of 1 mg/kg in combination with a chemical drug, and the volume change of the transplanted tumor was recorded during administration.

## DETAILED DESCRIPTION

In the present invention, the related terms are defined as follows:
The term "complementarity" as used herein refers to the capability of forming base pairs between two oligonucleotide strands. The base pairs are generally formed through hydrogen bonds between nucleotides in the antiparallel oligonucleotide strands. The bases of the complementary oligonucleotide strands can be paired in the Watson-Crick manner (such as A to T, A to U, and C to G) or in any other manner allowing the formation of a duplex (such as Hoogsteen or reverse Hoogsteen base pairing).

Complementarity includes complete complementarity and incomplete complementarity. "Complete complementarity" or "100% complementarity" means that each nucleotide from the first oligonucleotide strand can form a hydrogen bond with a nucleotide at a corresponding position in the second oligonucleotide strand in the double-stranded region of the double-stranded oligonucleotide molecule without "mispairing". "Incomplete complementarity" means that not all the nucleotide units of the two strands are bonded with each other by hydrogen bonds. For example, for two oligonucleotide strands each of 20 nucleotides in length in the double-stranded region, if only two base pairs in this double-stranded region can be formed through hydrogen bonds, the oligonucleotide strands have a complementarity of 10%. In the same example, if 18 base pairs in this double-stranded region can be formed through hydrogen bonds, the oligonucleotide strands have a complementarity of 90%. Substantial complementarity refers to at least about 75%, about 79%, about 80%, about 85%, about 90%, about 95%, about 99% or about 100% complementarity.

The term "oligonucleotide" as used herein refers to polymers of nucleotides, and includes, but is not limited to, single-stranded or double-stranded molecules of DNA, RNA, or DNA/RNA hybrid, oligonucleotide strands containing regularly and irregularly alternating deoxyribosyl portions and ribosyl portions, as well as modified and naturally or unnaturally existing frameworks for such oligonucleotides. The oligonucleotide for activating target gene transcription described herein is a small activating nucleic acid molecule.

The terms "oligonucleotide strand" and "oligonucleotide sequence" as used herein can be used interchangeably, referring to a generic term for short nucleotide sequences having less than 35 bases (including nucleotides in deoxyribonucleic acid (DNA) or ribonucleic acid (RNA)). In the present invention, an oligonucleotide strand can have any of 16 to 35 nucleotides in length.

As used herein, the term "first nucleic acid strand" can be a sense strand or an antisense strand. The sense strand of a small activating RNA refers to a nucleic acid strand contained in a small activating RNA duplex which has identity to the coding strand of the promoter DNA sequence of a target gene, and the antisense strand refers to a nucleic acid strand in the small activating RNA duplex which is complementary with the sense strand.

As used herein, the term "second nucleic acid strand" can also be a sense strand or an antisense strand. If the first oligonucleotide strand is a sense strand, the second oligonucleotide strand is an antisense strand; and if the first oligonucleotide strand is an antisense strand, the second oligonucleotide strand is a sense strand.

The term "gene" as used herein refers to all nucleotide sequences required to encode a polypeptide chain or to transcribe a functional RNA. "Gene" can be an endogenous or fully or partially recombinant gene for a host cell (for example, because an exogenous oligonucleotide and a coding sequence for encoding a promoter are introduced into a host cell, or a heterogeneous promoter adjacent to an endogenous coding sequence is introduced into a host cell). For example, the term "gene" comprises a nucleic acid sequence consisting of exons and introns. Protein-coding sequences are, for example, sequences contained within exons in an open reading frame between an initiation codon and a termination codon, and as used herein, "gene" can comprise such as a gene regulatory sequence, such as a promoter, an enhancer, and all other sequences known in the art for controlling the transcription, expression or activity of another gene, no matter whether the gene comprises a coding sequence or a non-coding sequence. In one case, for example, "gene" can be used to describe a functional nucleic acid comprising a regulatory sequence such as a promoter or an enhancer. The expression of a recombinant gene can be controlled by one or more types of heterogeneous regulatory sequences.

The term "target gene" as used herein can refer to nucleic acid sequences naturally present in organisms, transgenes, viral or bacterial sequences, can be chromosomes or extrachromosomal genes, and/or can be transiently or stably transfected or incorporated into cells and/or chromatins thereof. The target gene can be a protein-coding gene or a non-protein-coding gene (such as a microRNA gene and a long non-coding RNA gene). The target gene generally contains a promoter sequence, and the positive regulation for the target gene can be achieved by designing a small activating nucleic acid molecule having sequence identity (also called homology) to the promoter sequence, characterized as the up-regulation of expression of the target gene. "Sequence of a target gene promoter" refers to a non-coding sequence of the target gene, and the reference of the sequence of a target gene promoter in the phrase "complementary with the sequence of a target gene promoter" of the present invention refers to a coding strand of the sequence, also known as a non-template strand, *i.e.,* a nucleic acid sequence having the same sequence as the coding sequence of the gene. "Target" or "target sequence" refers to a sequence fragment in the sequence of a target gene promoter which is homologous or complementary with a sense oligonucleotide strand or an antisense oligonucleotide strand of a small activating nucleic acid molecule.

As used herein, the terms "sense strand" and "sense nucleic acid strand" can be used interchangeably, and the sense oligonucleotide strand of a small activating nucleic acid molecule refers to the first nucleic acid strand having sequence identity to the coding strand of the sequence of a target gene promoter in the small activating nucleic acid molecule duplex.

As used herein, the terms "antisense strand" and "antisense nucleic acid strand" can be used interchangeably, and the antisense oligonucleotide strand of a small activating nucleic acid molecule refers to the second nucleic acid strand which is complementary with the sense oligonucleotide strand in the small activating nucleic acid molecule duplex.

The term "coding strand" as used herein refers to a DNA strand in the target gene which cannot be used for transcription, and the nucleotide sequence of this strand is the same as that of a RNA produced from transcription (in the RNA, T in DNA is replaced by U). The coding strand of the double-stranded DNA sequence of the target gene promoter described herein refers to a promoter sequence on the same DNA strand as the DNA coding strand of the target gene.

The term "template strand" as used herein refers to the other strand complementary with the coding strand in the double-stranded DNA of the target gene, *i.e.*, the strand that, as a template, can be transcribed into RNA, and this strand is complementary with the transcribed RNA (A to U and G to C). In the process of transcription, RNA polymerase binds to the template strand, moves along the 3'→5' direction of the template strand, and catalyzes the synthesis of the RNA along the 5'→3' direction. The template strand of the double-stranded DNA sequence of the target gene promoter described herein refers to a promoter sequence on the same DNA strand as the DNA template strand of the target gene.

The term "promoter" as used herein refers to a sequence which plays a regulatory role for the transcription of a protein-coding or RNA-coding nucleic acid sequence by spacially associating with the coding sequence. Generally, a eukaryotic gene promoter contains 100 to 5000 base pairs, although this length range is not intended to limit the term "promoter" as used herein. Although the promoter sequence is generally located at the 5' terminus of a protein-coding or RNA-coding sequence, it can also exist in exon and intron sequences.

The term "transcription start site" as used herein refers to a nucleotide marking the transcription start on the template strand of a gene. The transcription start site can appear on the template strand of the promoter region. A gene can have more than one transcription start site.

The term "identity" or "homology" as used herein means that one oligonucleotide strand (sense or antisense strand) of an small activating RNA has sequence similarity with a coding strand or a template strand in a region of the promoter sequence of a target gene. As used herein, the "identity" or "homology" can be at least about 75%, about 79%, about 80%, about 85%, about 90%, about 95%, about 99% or about 100%.

The term "overhang" as used herein refers to non-base-paired nucleotides at the terminus (5' or 3') of an oligonucleotide strand, which is formed by one strand extending out of the other strand in a double-stranded oligonucleotide. A single-stranded region extending out of the 3' terminus and/or 5' terminus of a duplex is referred to as an overhang.

As used herein, the terms "gene activation", "activating gene expression", "gene up-regulation" and "up-regulating gene expression" can be used interchangeably, and mean an increase in transcription, translation, expression or activity of a certain nucleic acid as determined by measuring the transcriptional level, mRNA level, protein level, enzymatic activity, methylation state, chromatin state or configuration, translation level or the activity or state in a cell or biological system of a gene. These activities or states can be determined directly or indirectly. In addition, "gene activation", "activating gene expression", "gene up-regulation" or "up-regulating gene expression" refers to an increase in activity associated with a nucleic acid sequence, regardless of the mechanism of such activation. For example, the nucleic acid sequence plays a regulatory role as a regulatory sequence, the nucleic acid sequence is transcribed into RNA and the RNA is translated into a protein, thereby increasing the expression of the protein.

As used herein, the terms "small activating RNA", "saRNA", and "small activating nucleic acid molecule" can be used interchangeably, and refer to a nucleic acid molecule that can upregulate target gene expression and can be composed of the first nucleic acid fragment (antisense nucleic acid strand, also referred to as antisense oligonucleotide strand) containing a nucleotide sequence having sequence identity or homology with the non-coding nucleic acid sequence (e.g., a promoter and an enhancer) of a target gene and a second nucleic acid fragment (sense nucleic acid strand, also referred to as sense oligonucleotide strand) containing a nucleotide sequence complementary with the first nucleic acid fragment, wherein the first nucleic acid fragment and the second nucleic acid fragment form a duplex. The small activating nucleic acid molecule can also be composed of a synthesized or vector-expressed single-stranded RNA molecule that can form a hairpin structure by two complementary regions within the molecule, wherein the first region comprises a nucleotide sequence having sequence identity to the target sequence of a promoter of a gene, and the second region comprises a nucleotide sequence which is complementary with the first region. The length of the duplex region of the small activating nucleic acid molecule is typically about 10 to about 50, about 12 to about 48, about 14 to about 46, about 16 to about 44, about 18 to about 42, about 20 to about 40, about 22 to about 38, about 24 to about 36, about 26 to about 34, and about 28 to about 32 base pairs, and typically about 10, about 15, about 20, about 25, about 30, about 35, about 40, about 45, or about 50 base pairs. In addition, the terms "saRNA", "small activating RNA", and "small activating nucleic acid molecule" also comprise nucleic acids other than the ribonucleotide, including, but not limited to, modified nucleotides or analogues.

As used herein, the term "hot spot" refers to a promoter region of at least 30 bp in length of a target gene, wherein targets of functional small activating nucleic acid molecules are enriched, i.e., at least 30% of the small activating nucleic acid molecules designed to target this region can induce a 1.2-fold or more change in the mRNA expression of the target gene.

As used herein, the term "synthesis" refers to a method for synthesis of an oligonucleotide, including any method allowing RNA synthesis, such as chemical synthesis, *in vitro* transcription, and/or vector-based expression.

According to the present invention, the expression of LHPP gene is up-regulated by RNA activation, and a related disease (particularly hepatocellular carcinoma) is treated by increasing the expression of full-length LHPP protein. The LHPP gene in the present invention is sometimes also called a target gene.

The method for preparing the small activating nucleic acid molecule provided by the present invention comprises sequence design and synthesis.

Small activating nucleic acid molecules of the present invention can be chemically synthesized or can be obtained from a biotechnology company specialized in nucleic acid synthesis.

Generally speaking, chemical synthesis of nucleic acids comprises the following four steps: (1) synthesis of oligomeric ribonucleotides; (2) deprotection; (3) purification and isolation; and (4) desalination and annealing.

For example, the specific steps for chemically synthesizing saRNAs described herein are as follows:

(1) Synthesis of oligomeric ribonucleotides

Synthesis of 1 μM RNA was set in an automatic DNA/RNA synthesizer (e.g., Applied Biosystems EXPEDITE8909), and the coupling time of each cycle was set as 10 to 15 min. With a solid phase-bonded 5'-O-p-dimethoxytriphenylmethyl-thymidine substrate as an initiator, one base was bonded to the solid phase substrate in the first cycle, and then, in the $n^{th}$ ($19 \geq n \geq 2$) cycle, one base was bonded to the base bonded in the $n-1^{th}$ cycle. This process was repeated until the synthesis of the whole nucleic acid sequence was completed.

(2) Deprotection

The solid phase substrate bonded with the saRNA was put into a test tube, and 1 mL of a mixed solution of ethanol and ammonium hydroxide (volume ratio: 1:3) was added to the test tube. The test tube was then sealed and placed in an incubator, and the mixture was incubated at 25-70 °C for 2 to 30 h. The solution containing the solid phase substrate bonded with the saRNA was filtered, and the filtrate was collected. The solid phase substrate was rinsed with double distilled water twice (1 mL each time), and the filtrate was collected. The collected eluent was combined and dried under vacuum for 1 to 12 h. Then the solution was added with 1 mL of a solution of tetrabutylammonium fluoride in tetrahydrofuran (1 M), let stand at room temperature for 4 to 12 h, followed by addition of 2 mL of *n*-butanol. Precipitate was collected to give a single-stranded crude product of saRNA by high-speed centrifugation.

(3) Purification and isolation

The resulting crude product of saRNA was dissolved in 2 mL of triethylamine acetate solution with a concentration of 1 mol/L, and the solution was separated by a reversed-phase C18 column of high pressure liquid chromatography to give a purified single-stranded product of saRNA.

(4) Desalination and annealing

Salts were removed by gel filtration (size exclusion chromatography). A single sense oligomeric ribonucleic acid strand and a single antisense oligomeric ribonucleic acid strand were mixed in a 1 to 2 mL of buffer (10 mM Tris, pH 7.5-8.0, 50 mM NaCl) at a molar ratio of 1:1. The solution was heated to 95 °C, and was then slowly cooled to room temperature to give a solution containing saRNA.

It was discovered in this study that after being introduced into a cell, the aforementioned saRNA could effectively increase the mRNA and protein expression of full-length LHPP.

The present invention will be further illustrated with reference to specific examples and drawings below. It should be understood that these examples are merely intended to illustrate the present invention rather than limit the scope of the present invention. In the following examples, study methods without specific conditions were generally in accordance with conventional conditions, such as conditions described in Sambrook, et al., Molecular Cloning: A Laboratory Manual (New York: Cold Spring Harbor Laboratory Press, 1989), or conditions recommended by the manufacturer.

## EXAMPLES

### *Example 1*

### Design and Synthesis of Small Activating Nucleic Acid Molecule Targeting LHPP Promoter

Using a 1 kb sequence (SEQ ID No: 493) encompassing -1 kb to -1 bp, but excluding an Alu repeat sequence from -647 bp to -198 bp, in the LHPP promoter region, as a target sequence **(FIG. 1)),** a series of 19-nt saRNA target sequences were selected, moving 1 bp each time, and a total of 453 saRNA target sequences were obtained. The target sequences were filtered to keep those which met the following criteria: (1) having GC content between 35% and 65%; (2) with less than 5 consecutive identical nucleotides; (3) with 2 or less dinucleotide repeats; and (4) with 2 or less trinucleotide repeat sequences. After filtration, 290 target sequences remained.

Each of the sense strand and antisense strand in the double-stranded small activating RNA (saRNA) used in the study had 21 nucleotides in length. The 19 nucleotides in the 5' region of the first nucleic acid strand (sense strand) of the double-stranded saRNA had 100% sequence identity to the target sequence of the promoter, and the 3' terminus of the first nucleic acid strand contained a TT sequence. The 19 nucleotides in the 5' region of the second nucleic acid strand were fully complementary with the first ribonucleic acid strand sequence, and the 3' terminus of the second nucleic acid strand contained a TT sequence. The aforementioned two strands of the double-stranded saRNA were mixed at a molar ratio of 1:1, and after annealed to obtain a duplex saRNA.

The sequence of the LHPP promoter is shown as follows, which corresponds to position 1 to position 1000 from 5' to 3' of SEQ ID NO:493:

- 1000 ttgaacccca taacatttca acgaattcct catcctttct gtgaatcaag
- 950 agcctgaaaa gaaatggtga aataatatga tcctctcttc tttgaaagct

- 900 caaagctatg ttggaccaga agtaaagtgt tctcgtttct atttaataac
- 850 ttgaaaggtt ccgaggggcc attgaggaaa ctcctccctt ttaatatcaa
- 800 tgtgtattta ttgcaaaaat aatgtagcat cgagtggtat tttatagctt
- 750 atccaaaaac ctcctgggtt taacgcattg tgatagtccc gttttcttct
- 700 cagcccaggt cctatgcatc ctcatctatg cagggctgtt atctgcatat
- 650 aatttttttt tttttaaga caaagtcttg ctctgtcgcc ccggctggag
- 600 tgcagtggtg caatctcggc tcactgcaac ctccgcctcc caggttcaag
- 550 cggttcttcc gcctcagcct accgagtagc tgggactaca ggcatgcgcc
- 500 accacaccta ggtgattttt gtatttttag tagagacagg ggtttcacca
- 450 tgttgaccag gctggtctcg aactcctgat ctcaagcgat ccacccgcct
- 400 cagcctccca aagtgctggg attacaggca taagccacta cgcccggcct
- 350 caattttgta ttgtactttt tctttctttc tttaatagag acagggtctc
- 300 actatgttga ctaggttggt ctagaactcc tgggcacaag ctgtccgccc
- 250 gcttctgcct cccaaagtgc tgggattgca ggcgtgaacc accgcccctg
- 200 gctacaggtg ccttcttgtc tcaatttgcc tttgaccttt cttagggact
- 150 tgttttctgc ttttcctgct ctttgtccgc tgatctcctg ggaagaaagc
- 100 ttccgaaaag gacaccgttt caggggcgag tgacgccggg gtgcccaggc
- 50 cgcgccccag ttccgggttt gcacccggtc ttcttgccct gccccgcccg

*Example 2*

High-throughput Screening of saRNAs Targeting LHPP Promoter Region

1. Cell culture and transfection

Human liver cancer cell line Huh7 was cultured in DMEM medium (Gibco), containing 10% of calf serum (Sigma-Aldrich) and 1% of penicillin/streptomycin (Gibco). The cells were cultured at 5% $CO_2$ and 37°C. According to the instructions provided by the manufacturer, RNAiMax (Invitrogen, Carlsbad, CA) was used to transfect small activating RNAs at a concentration of 10 nM (unless otherwise specified).

2. One-step RT-qPCR

At the end of transfection, the media were discarded, and each well was washed with 150 μL of PBS once. After discarding the PBS, 50 μL of cell lysis buffer (Takara) was added to each well and incubated at room temperature for 5 min. 1 μL of the resulted cell lysis was taken from each well and analyzed by qPCR on an ABI 7500 fast real-time PCR system (Applied Biosystems) using a one-step TB Green™ PrimeScrip™ RT-PCR kit II (Takara, RR086A) Each transfection sample was repeatedly amplified in 3 replicate wells. PCR reaction conditions are shown in Table 1 below.

Table 1. PCR reaction preparation

| Reagen | Volume/Reaction |
|---|---|
| 2 × One-step TB Green RT-PCR buffer 4 | 2.5 μL |
| PrimeScript 1 step enzyme mixture 2 | 0.2 μL |
| Mixture of forward and reverse primers (5 μM) | 0.4 μL |
| No RNase $dH_2O$ | 1.4 μL |
| Crude lysate (RNA) | 0.5 μL |
| Sum | 5 μL |

Reaction conditions were as follows: reverse transcription reaction (stage 1): 5 min at 42 °C, 10 s at 95 °C; PCR reaction (stage 2): 5 s at 95 °C, 20 s at 60 °C, 45 cycles of amplification. HPRT1 and TBP were used as internal reference genes. PCR primers used for amplifying LHPP, HPRT1 and TBP genes are shown in Table 2, wherein LHPP was amplified using the LHPP F1/R1 primer pair.

Table 2. Primer sequences for RT-qPCR analysis

| Primer | Sequence No. | Sequence (5'-3') |
|---|---|---|
| LHPP F1 | SEQ ID NO:494 | AAGGCGCTTGAGTATGCCTG |
| LHPP R1 | SEQ ID NO:495 | GTGGGCTTCCACTCCTATCG |
| LPRT1 F | SEQ ID NO:496 | ATGGACAGGACTGAACGTCTT |
| LPRT1 R | SEQ ID NO:497 | TCCAGCAGGTCAGCAAAGAA |
| TBPF | SEQ ID NO:498 | ATAATCCCAAGCGGTTTGCT |
| TBP R | SEQ ID NO:499 | CTGCCAGTCTGGACTGTTCT |

To calculate the relative expression level ($E_{rel}$) of LHPP (target gene) in an saRNA-transfected sample relative to control treatment (Mock), the Ct values of the target gene and the two internal reference genes were substituted into formula 1

$$E_{rel} = 2^{(CtTm - CtTs)} / ((2^{(CtR1m - CtR1s)} * 2^{(CtR2m - CtR2s)})^{(1/2)})$$

(formula 1)

wherein CtTm was the Ct value of the target gene from the control (Mock) sample; CtTs was the Ct value of the target gene from the saRNA-treated sample; CtRlm was the Ct value of the internal reference gene 1 from the control (Mock) sample; CtR1s was the Ct value of the internal reference gene 1 from the saRNA-treated sample; CtR2m was the Ct value of the internal reference gene 2 from the control (Mock) sample; and CtR2s was the Ct value of the internal reference gene 2 from the saRNA-treated sample.

3. Screening of functional saRNAs

In order to obtain saRNAs capable of activating LHPP transcription, Huh7 cells were transfected with each of the aforementioned 290 saRNAs with a transfection concentration of 10 nM, and 72 hours later, according to the same method as described above, the cells were lysed and analyzed by one-step RT-qPCR to obtain the relative (compared with the control (Mock)) expression level of LHPP gene for each saRNA-treated sample. As shown in Table 3, 164 (56.6%) and 37 (12.8%) saRNAs exhibited activating and inhibiting activities, respectively, and 89 (30.7%) saRNAs had no effect on the expression of LHPP. The observed maximum activation and maximum inhibition is 3.46 fold and 0.49 fold, respectively. saRNAs with activating activity are referred to as activating saRNAs, and the saRNAs with inhibiting activity are referred to as inhibiting saRNAs.

Table 3. High-throughput screening results of LHPP

| saRNA activity | $log_2$ value of change in LHPP (fold) | Number of saRNAs | Percentage |
|---|---|---|---|
| High activation | ≥ 0.49 (1.50) ~ ≤ 1.79 (3.46) | 30 | 10.3 |
| Moderate activation | ≥ 0.26 (1.20) ~ < 0.49 (1.50) | 81 | 27.9 |
| Mild activation | ≥ 0.13 (1.10) ~ < 0.26 (1.20) | 53 | 18.3 |
| No effect | < 0.13 (1.10) ~ > -0.13 (0.91) | 89 | 30.7 |
| Mild inhibition | ≤ -0.13 (0.91) ~ > -0.26 (0.84) | 18 | 6.2 |
| Moderate inhibition | ≤ -0.26 (0.84) ~ > -0.49 (0.71) | 14 | 4.8 |
| High inhibition | ≤ -0.49 (0.71) ~ ≥ -0.73 (0.49) | 5 | 1.7 |
| Total | | 290 | 100 |

**FIG. 2** further shows the distribution of the activities of the LHPP saRNAs sorted from high activation to high inhibition.

Table 4. Functional saRNA sequences, functional target sequences thereof and changes in LHPP mRNA expression level

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-133** | GCTCTTTGTCCGCTGATCT (SEQ ID NO:329) | GCUCUUUGUCCGCUGAUCUTT (SEQ ID NO:1) | AGAUCAGCGGACAAAGAGCTT (SEQ ID NO: 165) | 2.54 | 1.35 |
| **RAG7-892** | TGTTGGACCAGAAGTAAAG (SEQ ID NO:330) | UGUUGGACCAGAAGUAAAGTT (SEQ ID NO:2) | CUUUACUUCUGGUCCAACATT (SEQ ID NO: 166) | 2.05 | 1.03 |
| **RAG7-694** | AGGTCCTATGCATCCTCAT (SEQ ID NO:331) | AGGUCCUAUGCAUCCUCAUTT (SEQ ID NO:3) | AUGAGGAUGCAUAGGACCUTT (SEQ ID NO: 167) | 1.88 | 0.91 |
| **RAG7-132** | CTCTTTGTCCGCTGATCTC (SEQ ID NO:332) | CUCUUUGUCCGCUGAUCUCTT (SEQ ID NO:4) | GAGAUCAGCGGACAAAGAGTT (SEQ ID NO: 168) | 1.86 | 0.90 |
| **RAG7-178** | AATTTGCCTTTGACCTTTC (SEQ ID NO:333) | AAUUUGCCUUUGACCUUUCTT (SEQ ID NO:5) | GAAAGGUCAAAGGCAAAUUTT (SEQ ID NO: 169) | 1.76 | 0.82 |
| **RAG7-177** | ATTTGCCTTTGACCTTTCT (SEQ ID NO:334) | AUUUGCCUUUGACCUUUCUTT (SEQ ID NO:6) | AGAAAGGUCAAAGGCAAAUTT (SEQ ID NO: 170) | 1.74 | 0.80 |
| **RAG7-139** | TTTCCTGCTCTTTGTCCGC (SEQ ID NO: 335) | UUUCCUGCUCUUUGUCCGCTT (SEQ ID NO:7) | GCGGACAAAGAGCAGGAAATT (SEQ ID NO: 171) | 1.73 | 0.79 |
| **RAG7-707** | TTCTTCTCAGCCCAGGTCC (SEQ ID NO:336) | UUCUUCUCAGCCCAGGUCCTT (SEQ ID NO:8) | GGACCUGGGCUGAGAAGAATT (SEQ ID NO: 172) | 1.72 | 0.78 |
| **RAG7-145** | TCTGCTTTTCCTGCTCTTT (SEQ ID NO:337) | UCUGCUUUUCCUGCUCUUUTT (SEQ ID NO:9) | AAAGAGCAGGAAAAGCAGATT (SEQ ID NO: 173) | 1.71 | 0.77 |
| **RAG7-146** | TTCTGCTTTTCCTGCTCTT (SEQ ID NO: 338) | UUCUGCUUUUCCUGCUCUUTT (SEQ ID NO:10) | AAGAGCAGGAAAAGCAGAATT (SEQ ID NO: 174) | 1.69 | 0.76 |
| **RAG7-846** | AAGGTTCCGAGGGGCCATT (SEQ ID NO: 339) | AAGGUUCCGAGGGGCCAUUTT (SEQ ID NO: 11) | AAUGGCCCCUCGGAACCUUTT (SEQ ID NO: 175) | 1.68 | 0.75 |
| **RAG7-95** | AAAAGGACACCGTTTCAGG (SEQ ID NO:340) | AAAAGGACACCGUUUCAGGTT (SEQ ID NO: 12) | CCUGAAACGGUGUCCUUUUTT (SEQ ID NO: 176) | 1.67 | 0.74 |
| **RAG7-94** | AAAGGACACCGTTTCAGGG (SEQ ID NO:341) | AAAGGACACCGUUUCAGGGTT (SEQ ID NO: 13) | CCCUGAAACGGUGUCCUUUTT (SEQ ID NO: 177) | 1.64 | 0.72 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-893** | ATGTTGGACCAGAAGTAAA (SEQ ID NO:342) | AUGUUGGACCAGAAGUAAATT (SEQ ID NO:14) | UUUACUUCUGGUCCAACAUTT (SEQ ID NO: 178) | 1.63 | 0.70 |
| **RAG7-706** | TCTTCTCAGCCCAGGTCCT (SEQ ID NO:343) | UCUUCUCAGCCCAGGUCCUTT (SEQ ID NO: 15) | AGGACCUGGGCUGAGAAGATT (SEQ ID NO: 179) | 1.63 | 0.70 |
| **RAG7-184** | TGTCTCAATTTGCCTTTGA (SEQ ID NO:344) | UGUCUCAAUUUGCCUUUGATT (SEQ ID NO: 16) | UCAAAGGCAAAUUGAGACATT (SEQ ID NO: 180) | 1.59 | 0.67 |
| **RAG7-696** | CCAGGTCCTATGCATCCTC (SEQ ID NO:345) | CCAGGUCCUAUGCAUCCUCTT (SEQ ID NO: 17) | GAGGAUGCAUAGGACCUGGTT (SEQ ID NO: 181) | 1.58 | 0.66 |
| **RAG7-144** | CTGCTTTTCCTGCTCTTTG (SEQ ID NO:346) | CUGCUUUUCCUGCUCUUUGTT (SEQ ID NO: 18) | CAAAGAGCAGGAAAAGCAGTT (SEQ ID NO: 182) | 1.56 | 0.64 |
| **RAG7-162** | TTCTTAGGGACTTGTTTTC (SEQ ID NO:347) | UUCUUAGGGACUUGUUUUCTT (SEQ ID NO:19) | GAAAACAAGUCCCUAAGAATT (SEQ ID NO: 183) | 1.56 | 0.64 |
| **RAG7-677** | ATCTATGCAGGGCTGTTAT (SEQ ID NO:348) | AUCUAUGCAGGGCUGUUAUTT (SEQ ID NO:20) | AUAACAGCCCUGCAUAGAUTT (SEQ ID NO: 184) | 1.56 | 0.64 |
| **RAG7-188** | TTCTTGTCTCAATTTGCCT (SEQ ID NO:349) | UUCUUGUCUCAAUUUGCCUTT (SEQ ID NO:21) | AGGCAAAUUGAGACAAGAATT (SEQ ID NO: 185) | 1.55 | 0.64 |
| **RAG7-907** | GAAAGCTCAAAGCTATGTT (SEQ ID NO:350) | GAAAGCUCAAAGCUAUGUUTT (SEQ ID NO:22) | AACAUAGCUUUGAGCUUUCTT (SEQ ID NO: 186) | 1.55 | 0.63 |
| **RAG7-909** | TTGAAAGCTCAAAGCTATG (SEQ ID NO:351) | UUGAAAGCUCAAAGCUAUGTT (SEQ ID NO:23) | CAUAGCUUUGAGCUUUCAATT (SEQ ID NO: 187) | 1.53 | 0.61 |
| **RAG7-35** | GGTTTGCACCCGGTCTTCT (SEQ ID NO:352) | GGUUUGCACCCGGUCUUCUTT (SEQ ID NO:24) | AGAAGACCGGGUGCAAACCTT (SEQ ID NO:188) | 1.53 | 0.61 |
| **RAG7-886** | ACCAGAAGTAAAGTGTTCT (SEQ ID NO:353) | ACCAGAAGUAAAGUGUUCUTT (SEQ ID NO:25) | AGAACACUUUACUUCUGGUTT (SEQ ID NO: 189) | 1.52 | 0.60 |
| **RAG7-34** | GTTTGCACCCGGTCTTCTT (SEQ ID NO:354) | GUUUGCACCCGGUCUUCUUTT (SEQ ID NO:26) | AAGAAGACCGGGUGCAAACTT (SEQ ID NO: 190) | 1.51 | 0.59 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-183** | GTCTCAATTTGCCTTTGAC (SEQ ID NO:355) | GUCUCAAUUUGCCUUUGACTT (SEQ ID NO:27) | GUCAAAGGCAAAUUGAGACTT (SEQ ID NO: 191) | 1.51 | 0.59 |
| **RAG7-195** | AGGTGCCTTCTTGTCTCAA (SEQ ID NO:356) | AGGUGCCUUCUUGUCUCAATT (SEQ ID NO:28) | UUGAGACAAGAAGGCACCUTT (SEQ ID NO: 192) | 1.51 | 0.59 |
| **RAG7-829** | TTGAGGAAACTCCTCCCTT (SEQ ID NO:357) | UUGAGGAAACUCCUCCCUUTT (SEQ ID NO:29) | AAGGGAGGAGUUUCCUCAATT (SEQ ID NO: 193) | 1.50 | 0.59 |
| **RAG7-691** | TCCTATGCATCCTCATCTA (SEQ ID NO:358) | UCCUAUGCAUCCUCAUCUATT (SEQ ID NO:30) | UAGAUGAGGAUGCAUAGGATT (SEQ ID NO:194) | 1.50 | 0.58 |
| **RAG7-908** | TGAAAGCTCAAAGCTATGT (SEQ ID NO:359) | UGAAAGCUCAAAGCUAUGUTT (SEQ ID NO:31) | ACAUAGCUUUGAGCUUUCATT (SEQ ID NO: 195) | 1.49 | 0.57 |
| **RAG7-150** | TGTTTTCTGCTTTTCCTGC (SEQ ID NO:360) | UGUUUUCUGCUUUUCCUGCTT (SEQ ID NO:32) | GCAGGAAAAGCAGAAAACATT (SEQ ID NO: 196) | 1.48 | 0.56 |
| **RAG7-916** | CTCTTCTTTGAAAGCTCAA (SEQ ID NO:361) | CUCUUCUUUGAAAGCUCAATT (SEQ ID NO:33) | UUGAGCUUUCAAAGAAGAGTT (SEQ ID NO: 197) | 1.48 | 0.56 |
| **RAG7-847** | AAAGGTTCCGAGGGGCCAT (SEQ ID NO:362) | AAAGGUUCCGAGGGGCCAUTT (SEQ ID NO:34) | AUGGCCCCUCGGAACCUUUTT (SEQ ID NO: 198) | 1.48 | 0.56 |
| **RAG7-189** | CTTCTTGTCTCAATTTGCC (SEQ ID NO:363) | CUUCUUGUCUCAAUUUGCCTT (SEQ ID NO:35) | GGCAAAUUGAGACAAGAAGTT (SEQ ID NO: 199) | 1.47 | 0.56 |
| **RAG7-830** | ATTGAGGAAACTCCTCCCT (SEQ ID NO:364) | AUUGAGGAAACUCCUCCCUTT (SEQ ID NO:36) | AGGGAGGAGUUUCCUCAAUTT (SEQ ID NO: 200) | 1.45 | 0.54 |
| **RAG7-894** | TATGTTGGACCAGAAGTAA (SEQ ID NO:365) | UAUGUUGGACCAGAAGUAATT (SEQ ID NO:37) | UUACUUCUGGUCCAACAUATT (SEQ ID NO:201) | 1.45 | 0.54 |
| **RAG7-196** | CAGGTGCCTTCTTGTCTCA (SEQ ID NO:366) | CAGGUGCCUUCUUGUCUCATT (SEQ ID NO:38) | UGAGACAAGAAGGCACCUGTT (SEQ ID NO:202) | 1.45 | 0.54 |
| **RAG7-179** | CAATTTGCCTTTGACCTTT (SEQ ID NO:367) | CAAUUUGCCUUUGACCUUUTT (SEQ ID NO:39) | AAAGGUCAAAGGCAAAUUGTT (SEQ ID NO:203) | 1.45 | 0.53 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-879** | GTAAAGTGTTCTCGTTTCT (SEQ ID NO:368) | GUAAAGUGUUCUCGUUUCUTT (SEQ ID NO:40) | AGAAACGAGAACACUUUACTT (SEQ ID NO:204) | 1.44 | 0.53 |
| **RAG7-697** | CCCAGGTCCTATGCATCCT (SEQ ID NO:369) | CCCAGGUCCUAUGCAUCCUTT (SEQ ID NO:41) | AGGAUGCAUAGGACCUGGGTT (SEQ ID NO:205) | 1.43 | 0.51 |
| **RAG7-690** | CCTATGCATCCTCATCTAT (SEQ ID NO:370) | CCUAUGCAUCCUCAUCUAUTT (SEQ ID NO:42) | AUAGAUGAGGAUGCAUAGGTT (SEQ ID NO:206) | 1.42 | 0.51 |
| **RAG7-104** | AAGCTTCCGAAAAGGACAC (SEQ ID NO:371) | AAGCUUCCGAAAAGGACACTT (SEQ ID NO:43) | GUGUCCUUUUCGGAAGCUUTT (SEQ ID NO:207) | 1.41 | 0.50 |
| **RAG7-32** | TTGCACCCGGTCTTCTTGC (SEQ ID NO:372) | UUGCACCCGGUCUUCUUGCTT (SEQ ID NO:44) | GCAAGAAGACCGGGUGCAATT (SEQ ID NO:208) | 1.40 | 0.49 |
| **RAG7-126** | GTCCGCTGATCTCCTGGGA (SEQ ID NO:373) | GUCCGCUGAUCUCCUGGGATT (SEQ ID NO:45) | UCCCAGGAGAUCAGCGGACTT (SEQ ID NO:209) | 1.39 | 0.48 |
| **RAG7-850** | TTGAAAGGTTCCGAGGGGC (SEQ ID NO:374) | UUGAAAGGUUCCGAGGGGCTT (SEQ ID NO:46) | GCCCCUCGGAACCUUUCAATT (SEQ ID NO:210) | 1.39 | 0.47 |
| **RAG7-684** | CATCCTCATCTATGCAGGG (SEQ ID NO:375) | CAUCCUCAUCUAUGCAGGGTT (SEQ ID NO:47) | CCCUGCAUAGAUGAGGAUGTT (SEQ ID NO:211) | 1.39 | 0.47 |
| **RAG7-194** | GGTGCCTTCTTGTCTCAAT (SEQ ID NO:376) | GGUGCCUUCUUGUCUCAAUTT (SEQ ID NO:48) | AUUGAGACAAGAAGGCACCTT (SEQ ID NO:212) | 1.38 | 0.47 |
| **RAG7-174** | TGCCTTTGACCTTTCTTAG (SEQ ID NO:377) | UGCCUUUGACCUUUCUUAGTT (SEQ ID NO:49) | CUAAGAAAGGUCAAAGGCATT (SEQ ID NO:213) | 1.38 | 0.47 |
| **RAG7-902** | CTCAAAGCTATGTTGGACC (SEQ ID NO:378) | CUCAAAGCUAUGUUGGACCTT (SEQ ID NO:50) | GGUCCAACAUAGCUUUGAGTT (SEQ ID NO:214) | 1.38 | 0.46 |
| **RAG7-887** | GACCAGAAGTAAAGTGTTC (SEQ ID NO:379) | GACCAGAAGUAAAGUGUUCTT (SEQ ID NO:51) | GAACACUUUACUUCUGGUCTT (SEQ ID NO:215) | 1.37 | 0.46 |
| **RAG7-121** | CTGATCTCCTGGGAAGAAA (SEQ ID NO:380) | CUGAUCUCCUGGGAAGAAATT (SEQ ID NO:52) | UUUCUUCCCAGGAGAUCAGTT (SEQ ID NO:216) | 1.36 | 0.45 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-138** | TTCCTGCTCTTTGTCCGCT (SEQ ID NO:381) | UUCCUGCUCUUUGUCCGCUTT (SEQ ID NO:53) | AGCGGACAAAGAGCAGGAATT (SEQ ID NO:217) | 1.36 | 0.45 |
| **RAG7-695** | CAGGTCCTATGCATCCTCA (SEQ ID NO: 382) | CAGGUCCUAUGCAUCCUCATT (SEQ ID NO:54) | UGAGGAUGCAUAGGACCUGTT (SEQ ID NO:218) | 1.36 | 0.44 |
| **RAG7-125** | TCCGCTGATCTCCTGGGAA (SEQ ID NO:383) | UCCGCUGAUCUCCUGGGAATT (SEQ ID NO:55) | UUCCCAGGAGAUCAGCGGATT (SEQ ID NO:219) | 1.36 | 0.44 |
| **RAG7-776** | TAGCATCGAGTGGTATTTT (SEQ ID NO: 384) | UAGCAUCGAGUGGUAUUUUTT (SEQ ID NO:56) | AAAAUACCACUCGAUGCUATT (SEQ ID NO:220) | 1.35 | 0.44 |
| **RAG7-119** | GATCTCCTGGGAAGAAAGC (SEQ ID NO:385) | GAUCUCCUGGGAAGAAAGCTT (SEQ ID NO:57) | GCUUUCUUCCCAGGAGAUCTT (SEQ ID NO:221) | 1.35 | 0.43 |
| **RAG7-180** | TCAATTTGCCTTTGACCTT (SEQ ID NO:386) | UCAAUUUGCCUUUGACCUUTT (SEQ ID NO:58) | AAGGUCAAAGGCAAAUUGATT (SEQ ID NO:222) | 1.35 | 0.43 |
| **RAG7-898** | AAGCTATGTTGGACCAGAA (SEQ ID NO:387) | AAGCUAUGUUGGACCAGAATT (SEQ ID NO:59) | UUCUGGUCCAACAUAGCUUTT (SEQ ID NO:223) | 1.34 | 0.43 |
| **RAG7-175** | TTGCCTTTGACCTTTCTTA (SEQ ID NO:388) | UUGCCUUUGACCUUUCUUATT (SEQ ID NO:60) | UAAGAAAGGUCAAAGGCAATT (SEQ ID NO:224) | 1.34 | 0.42 |
| **RAG7-169** | TTGACCTTTCTTAGGGACT (SEQ ID NO:389) | UUGACCUUUCUUAGGGACUTT (SEQ ID NO:61) | AGUCCCUAAGAAAGGUCAATT (SEQ ID NO:225) | 1.34 | 0.42 |
| **RAG7-720** | TGATAGTCCCGTTTTCTTC (SEQ ID NO:390) | UGAUAGUCCCGUUUUCUUCTT (SEQ ID NO:62) | GAAGAAAACGGGACUAUCATT (SEQ ID NO:226) | 1.33 | 0.41 |
| **RAG7-678** | CATCTATGCAGGGCTGTTA (SEQ ID NO:391) | CAUCUAUGCAGGGCUGUUATT (SEQ ID NO:63) | UAACAGCCCUGCAUAGAUGTT (SEQ ID NO:227) | 1.33 | 0.41 |
| **RAG7-917** | TCTCTTCTTTGAAAGCTCA (SEQ ID NO:392) | UCUCUUCUUUGAAAGCUCATT (SEQ ID NO:64) | UGAGCUUUCAAAGAAGAGATT (SEQ ID NO:228) | 1.32 | 0.40 |
| **RAG7-897** | AGCTATGTTGGACCAGAAG (SEQ ID NO:393) | AGCUAUGUUGGACCAGAAGTT (SEQ ID NO:65) | CUUCUGGUCCAACAUAGCUTT (SEQ ID NO:229) | 1.31 | 0.39 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-147** | TTTCTGCTTTTCCTGCTCT (SEQ ID NO:394) | UUUCUGCUUUUCCUGCUCUTT (SEQ ID NO:66) | AGAGCAGGAAAAGCAGAAATT (SEQ ID NO:230) | 1.31 | 0.39 |
| **RAG7-148** | TTTTCTGCTTTTCCTGCTC (SEQ ID NO:395) | UUUUCUGCUUUUCCUGCUCTT (SEQ ID NO:67) | GAGCAGGAAAAGCAGAAAATT (SEQ ID NO:231) | 1.30 | 0.38 |
| **RAG7-123** | CGCTGATCTCCTGGGAAGA (SEQ ID NO:396) | CGCUGAUCUCCUGGGAAGATT (SEQ ID NO:68) | UCUUCCCAGGAGAUCAGCGTT (SEQ ID NO:232) | 1.30 | 0.38 |
| **RAG7-896** | GCTATGTTGGACCAGAAGT (SEQ ID NO:397) | GCUAUGUUGGACCAGAAGUTT (SEQ ID NO:69) | ACUUCUGGUCCAACAUAGCTT (SEQ ID NO:233) | 1.30 | 0.38 |
| **RAG7-778** | TGTAGCATCGAGTGGTATT (SEQ ID NO:398) | UGUAGCAUCGAGUGGUAUUTT (SEQ ID NO:70) | AAUACCACUCGAUGCUACATT (SEQ ID NO:234) | 1.29 | 0.37 |
| **RAG7-97** | CGAAAAGGACACCGTTTCA (SEQ ID NO:399) | CGAAAAGGACACCGUUUCATT (SEQ ID NO:71) | UGAAACGGUGUCCUUUUCGTT (SEQ ID NO:235) | 1.29 | 0.37 |
| **RAG7-103** | AGCTTCCGAAAAGGACACC (SEQ ID NO:400) | AGCUUCCGAAAAGGACACCTT (SEQ ID NO:72) | GGUGUCCUUUUCGGAAGCUTT (SEQ ID NO:236) | 1.29 | 0.37 |
| **RAG7-114** | CCTGGGAAGAAAGCTTCCG (SEQ ID NO:401) | CCUGGGAAGAAAGCUUCCGTT (SEQ ID NO:73) | CGGAAGCUUUCUUCCCAGGTT (SEQ ID NO:237) | 1.28 | 0.36 |
| **RAG7-140** | TTTTCCTGCTCTTTGTCCG (SEQ ID NO:402) | UUUUCCUGCUCUUUGUCCGTT (SEQ ID NO:74) | CGGACAAAGAGCAGGAAAATT (SEQ ID NO:238) | 1.28 | 0.36 |
| **RAG7-134** | TGCTCTTTGTCCGCTGATC (SEQ ID NO:403) | UGCUCUUUGUCCGCUGAUCTT (SEQ ID NO:75) | GAUCAGCGGACAAAGAGCATT (SEQ ID NO:239) | 1.28 | 0.36 |
| **RAG7-890** | TTGGACCAGAAGTAAAGTG (SEQ ID NO:404) | UUGGACCAGAAGUAAAGUGTT (SEQ ID NO:76) | CACUUUACUUCUGGUCCAATT (SEQ ID NO:240) | 1.28 | 0.36 |
| **RAG7-130** | CTTTGTCCGCTGATCTCCT (SEQ ID NO:405) | CUUUGUCCGCUGAUCUCCUTT (SEQ ID NO:77) | AGGAGAUCAGCGGACAAAGTT (SEQ ID NO:241) | 1.28 | 0.36 |
| **RAG7-186** | CTTGTCTCAATTTGCCTTT (SEQ ID NO:406) | CUUGUCUCAAUUUGCCUUUTT (SEQ ID NO:78) | AAAGGCAAAUUGAGACAAGTT (SEQ ID NO:242) | 1.28 | 0.35 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-29** | CACCCGGTCTTCTTGCCCT (SEQ ID NO:407) | CACCCGGUCUUCUUGCCCUTT (SEQ ID NO:79) | AGGGCAAGAAGACCGGGUGTT (SEQ ID NO:243) | 1.28 | 0.35 |
| **RAG7-171** | CTTTGACCTTTCTTAGGGA (SEQ ID NO:408) | CUUUGACCUUUCUUAGGGATT (SEQ ID NO:80) | UCCCUAAGAAAGGUCAAAGTT (SEQ ID NO:244) | 1.27 | 0.34 |
| **RAG7-172** | CCTTTGACCTTTCTTAGGG (SEQ ID NO:409) | CCUUUGACCUUUCUUAGGGTT (SEQ ID NO:81) | CCCUAAGAAAGGUCAAAGGTT (SEQ ID NO:245) | 1.27 | 0.34 |
| **RAG7-112** | TGGGAAGAAAGCTTCCGAA (SEQ ID NO:410) | UGGGAAGAAAGCUUCCGAATT (SEQ ID NO:82) | UUCGGAAGCUUUCUUCCCATT (SEQ ID NO:246) | 1.26 | 0.33 |
| **RAG7-676** | TCTATGCAGGGCTGTTATC (SEQ ID NO:411) | UCUAUGCAGGGCUGUUAUCTT (SEQ ID NO:83) | GAUAACAGCCCUGCAUAGATT (SEQ ID NO:247) | 1.26 | 0.33 |
| **RAG7-899** | AAAGCTATGTTGGACCAGA (SEQ ID NO:412) | AAAGCUAUGUUGGACCAGATT (SEQ ID NO:84) | UCUGGUCCAACAUAGCUUUTT (SEQ ID NO:248) | 1.26 | 0.33 |
| **RAG7-182** | TCTCAATTTGCCTTTGACC (SEQ ID NO:413) | UCUCAAUUUGCCUUUGACCTT (SEQ ID NO:85) | GGUCAAAGGCAAAUUGAGATT (SEQ ID NO:249) | 1.26 | 0.33 |
| **RAG7-686** | TGCATCCTCATCTATGCAG (SEQ ID NO:414) | UGCAUCCUCAUCUAUGCAGTT (SEQ ID NO:86) | CUGCAUAGAUGAGGAUGCATT (SEQ ID NO:250) | 1.25 | 0.32 |
| **RAG7-848** | GAAAGGTTCCGAGGGGCCA (SEQ ID NO:415) | GAAAGGUUCCGAGGGGCCATT (SEQ ID NO:87) | UGGCCCCUCGGAACCUUUCTT (SEQ ID NO:251) | 1.25 | 0.32 |
| **RAG7-191** | GCCTTCTTGTCTCAATTTG (SEQ ID NO:416) | GCCUUCUUGUCUCAAUUUGTT (SEQ ID NO:88) | CAAAUUGAGACAAGAAGGCTT (SEQ ID NO:252) | 1.25 | 0.32 |
| **RAG7-821** | ACTCCTCCCTTTTAATATC (SEQ ID NO:417) | ACUCCUCCCUUUUAAUAUCTT (SEQ ID NO:89) | GAUAUUAAAAGGGAGGAGUTT (SEQ ID NO:253) | 1.25 | 0.32 |
| **RAG7-109** | GAAGAAAGCTTCCGAAAAG (SEQ ID NO:418) | GAAGAAAGCUUCCGAAAAGTT (SEQ ID NO:90) | CUUUUCGGAAGCUUUCUUCTT (SEQ ID NO:254) | 1.24 | 0.31 |
| **RAG7-168** | TGACCTTTCTTAGGGACTT (SEQ ID NO:419) | UGACCUUUCUUAGGGACUUTT (SEQ ID NO:91) | AAGUCCCUAAGAAAGGUCATT (SEQ ID NO:255) | 1.24 | 0.31 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-905** | AAGCTCAAAGCTATGTTGG (SEQ ID N0:420) | AAGCUCAAAGCUAUGUUGGTT (SEQ ID NO:92) | CCAACAUAGCUUUGAGCUUTT (SEQ ID NO:256) | 1.24 | 0.31 |
| **RAG7-43** | CAGTTCCGGGTTTGCACCC (SEQ ID NO: 42 1) | CAGUUCCGGGUUUGCACCCTT (SEQ ID NO:93) | GGGUGCAAACCCGGAACUGTT (SEQ ID NO:257) | 1.23 | 0.30 |
| **RAG7-31** | TGCACCCGGTCTTCTTGCC (SEQ ID NO:422) | UGCACCCGGUCUUCUUGCCTT (SEQ ID NO:94) | GGCAAGAAGACCGGGUGCATT (SEQ ID NO:258) | 1.23 | 0.30 |
| **RAG7-741** | CCTCCTGGGTTTAACGCAT (SEQ ID NO:423) | CCUCCUGGGUUUAACGCAUTT (SEQ ID NO:95) | AUGCGUUAAACCCAGGAGGTT (SEQ ID NO:259) | 1.23 | 0.30 |
| **RAG7-102** | GCTTCCGAAAAGGACACCG (SEQ ID NO:424) | GCUUCCGAAAAGGACACCGTT (SEQ ID NO:96) | CGGUGUCCUUUUCGGAAGCTT (SEQ ID NO:260) | 1.23 | 0.30 |
| **RAG7-181** | CTCAATTTGCCTTTGACCT (SEQ ID NO:425) | CUCAAUUUGCCUUUGACCUTT (SEQ ID NO:97) | AGGUCAAAGGCAAAUUGAGTT (SEQ ID NO:261) | 1.22 | 0.29 |
| **RAG7-693** | GGTCCTATGCATCCTCATC (SEQ ID NO:426) | GGUCCUAUGCAUCCUCAUCTT (SEQ ID NO:98) | GAUGAGGAUGCAUAGGACCTT (SEQ ID NO:262) | 1.22 | 0.29 |
| RAG7-149 | GTTTTCTGCTTTTCCTGCT (SEQ ID NO:427) | GUUUUCUGCUUUUCCUGCUTT (SEQ ID NO:99) | AGCAGGAAAAGCAGAAAACTT (SEQ ID NO:263) | 1.22 | 0.29 |
| **RAG7-151** | TTGTTTTCTGCTTTTCCTG (SEQ ID NO:428) | UUGUUUUCUGCUUUUCCUGTT (SEQ ID NO:100) | CAGGAAAAGCAGAAAACAATT (SEQ ID NO:264) | 1.22 | 0.28 |
| **RAG7-884** | CAGAAGTAAAGTGTTCTCG (SEQ ID NO:429) | CAGAAGUAAAGUGUUCUCGTT (SEQ ID NO: 101) | CGAGAACACUUUACUUCUGTT (SEQ ID NO:265) | 1.22 | 0.28 |
| **RAG7-143** | TGCTTTTCCTGCTCTTTGT (SEQ ID NO:430) | UGCUUUUCCUGCUCUUUGUTT (SEQ ID NO: 102) | ACAAAGAGCAGGAAAAGCATT (SEQ ID NO:266) | 1.21 | 0.28 |
| **RAG7-122** | GCTGATCTCCTGGGAAGAA (SEQ ID NO:431) | GCUGAUCUCCUGGGAAGAATT (SEQ ID NO: 103) | UUCUUCCCAGGAGAUCAGCTT (SEQ ID NO:267) | 1.21 | 0.28 |
| **RAG7-89** | ACACCGTTTCAGGGGCGAG (SEQ ID NO:432) | ACACCGUUUCAGGGGCGAGTT (SEQ ID NO:104) | CUCGCCCCUGAAACGGUGUTT (SEQ ID NO:268) | 1.21 | 0.28 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-96** | GAAAAGGACACCGTTTCAG (SEQ ID NO:433) | GAAAAGGACACCGUUUCAGTT (SEQ ID NO: 105) | CUGAAACGGUGUCCUUUUCTT (SEQ ID NO:269) | 1.21 | 0.27 |
| **RAG7-708** | TTTCTTCTCAGCCCAGGTC (SEQ ID NO:434) | UUUCUUCUCAGCCCAGGUCTT (SEQ ID NO: 106) | GACCUGGGCUGAGAAGAAATT (SEQ ID NO:270) | 1.21 | 0.27 |
| **RAG7-679** | TCATCTATGCAGGGCTGTT (SEQ ID NO:435) | UCAUCUAUGCAGGGCUGUUTT (SEQ ID NO: 107) | AACAGCCCUGCAUAGAUGATT (SEQ ID NO:271) | 1.20 | 0.27 |
| **RAG7-828** | TGAGGAAACTCCTCCCTTT (SEQ ID NO:436) | UGAGGAAACUCCUCCCUUUTT (SEQ ID NO: 108) | AAAGGGAGGAGUUUCCUCATT (SEQ ID NO:272) | 1.20 | 0.26 |
| **RAG7-837** | AGGGGCCATTGAGGAAACT (SEQ ID NO:437) | AGGGGCCAUUGAGGAAACUTT (SEQ ID NO: 109) | AGUUUCCUCAAUGGCCCCUTT (SEQ ID NO:273) | 1.20 | 0.26 |
| **RAG7-176** | TTTGCCTTTGACCTTTCTT (SEQ ID NO:438) | UUUGCCUUUGACCUUUCUUTT (SEQ ID NO: 110) | AAGAAAGGUCAAAGGCAAATT (SEQ ID NO:274) | 1.20 | 0.26 |
| **RAG7-128** | TTGTCCGCTGATCTCCTGG (SEQ ID NO:439) | UUGUCCGCUGAUCUCCUGGTT (SEQ ID NO:111) | CCAGGAGAUCAGCGGACAATT (SEQ ID NO:275) | 1.20 | 0.26 |
| **RAG7-704** | TTCTCAGCCCAGGTCCTAT (SEQ ID NO:440) | UUCUCAGCCCAGGUCCUAUTT (SEQ ID NO: 112) | AUAGGACCUGGGCUGAGAATT (SEQ ID NO:276) | 1.19 | 0.26 |
| **RAG7-193** | GTGCCTTCTTGTCTCAATT (SEQ ID NO:441) | GUGCCUUCUUGUCUCAAUUTT (SEQ ID NO: 113) | AAUUGAGACAAGAAGGCACTT (SEQ ID NO:277) | 1.19 | 0.26 |
| **RAG7-735** | GGGTTTAACGCATTGTGAT (SEQ ID NO:442) | GGGUUUAACGCAUUGUGAUTT (SEQ ID NO: 114) | AUCACAAUGCGUUAAACCCTT (SEQ ID NO:278) | 1.19 | 0.25 |
| **RAG7-889** | TGGACCAGAAGTAAAGTGT (SEQ ID NO:443) | UGGACCAGAAGUAAAGUGUTT (SEQ ID NO: 115) | ACACUUUACUUCUGGUCCATT (SEQ ID NO:279) | 1.19 | 0.25 |
| **RAG7-185** | TTGTCTCAATTTGCCTTTG (SEQ ID NO:444) | UUGUCUCAAUUUGCCUUUGTT (SEQ ID NO: 116) | CAAAGGCAAAUUGAGACAATT (SEQ ID NO:280) | 1.19 | 0.25 |
| **RAG7-111** | GGGAAGAAAGCTTCCGAAA (SEQ ID NO:445) | GGGAAGAAAGCUUCCGAAATT (SEQ ID NO:117) | UUUCGGAAGCUUUCUUCCCTT (SEQ ID NO:281) | 1.18 | 0.24 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-698** | GCCCAGGTCCTATGCATCC (SEQ ID NO:446) | GCCCAGGUCCUAUGCAUCCTT (SEQ ID NO: 118) | GGAUGCAUAGGACCUGGGCTT (SEQ ID NO:282) | 1.18 | 0.24 |
| **RAG7-33** | TTTGCACCCGGTCTTCTTG (SEQ ID NO:447) | UUUGCACCCGGUCUUCUUGTT (SEQ ID NO: 119) | CAAGAAGACCGGGUGCAAATT (SEQ ID NO:283) | 1.18 | 0.24 |
| **RAG7-113** | CTGGGAAGAAAGCTTCCGA (SEQ ID NO:448) | CUGGGAAGAAAGCUUCCGATT (SEQ ID NO: 120) | UCGGAAGCUUUCUUCCCAGTT (SEQ ID NO:284) | 1.18 | 0.24 |
| **RAG7-44** | CCAGTTCCGGGTTTGCACC (SEQ ID NO:449) | CCAGUUCCGGGUUUGCACCTT (SEQ ID NO: 121) | GGUGCAAACCCGGAACUGGTT (SEQ ID NO:285) | 1.18 | 0.24 |
| **RAG7-710** | GTTTTCTTCTCAGCCCAGG (SEQ ID NO:450) | GUUUUCUUCUCAGCCCAGGTT (SEQ ID NO: 122) | CCUGGGCUGAGAAGAAAACTT (SEQ ID NO:286) | 1.18 | 0.24 |
| **RAG7-187** | TCTTGTCTCAATTTGCCTT (SEQ ID NO:451) | UCUUGUCUCAAUUUGCCUUTT (SEQ ID NO: 123) | AAGGCAAAUUGAGACAAGATT (SEQ ID NO:287) | 1.17 | 0.22 |
| **RAG7-692** | GTCCTATGCATCCTCATCT (SEQ ID NO:452) | GUCCUAUGCAUCCUCAUCUTT (SEQ ID NO: 124) | AGAUGAGGAUGCAUAGGACTT (SEQ ID NO:288) | 1.17 | 0.22 |
| **RAG7-100** | TTCCGAAAAGGACACCGTT (SEQ ID NO:453) | UUCCGAAAAGGACACCGUUTT (SEQ ID NO: 125) | AACGGUGUCCUUUUCGGAATT (SEQ ID NO:289) | 1.17 | 0.22 |
| **RAG7-709** | TTTTCTTCTCAGCCCAGGT (SEQ ID NO:454) | UUUUCUUCUCAGCCCAGGUTT (SEQ ID NO: 126) | ACCUGGGCUGAGAAGAAAATT (SEQ ID NO:290) | 1.17 | 0.22 |
| **RAG7-726** | GCATTGTGATAGTCCCGTT (SEQ ID NO:455) | GCAUUGUGAUAGUCCCGUUTT (SEQ ID NO: 127) | AACGGGACUAUCACAAUGCTT (SEQ ID NO:291) | 1.17 | 0.22 |
| **RAG7-852** | ACTTGAAAGGTTCCGAGGG (SEQ ID NO:456) | ACUUGAAAGGUUCCGAGGGTT (SEQ ID NO:128) | CCCUCGGAACCUUUCAAGUTT (SEQ ID NO:292) | 1.17 | 0.22 |
| **RAG7-844** | GGTTCCGAGGGGCCATTGA (SEQ ID NO:457) | GGUUCCGAGGGGCCAUUGATT (SEQ ID NO: 129) | UCAAUGGCCCCUCGGAACCTT (SEQ ID NO:293) | 1.17 | 0.22 |
| **RAG7-190** | CCTTCTTGTCTCAATTTGC (SEQ ID NO:458) | CCUUCUUGUCUCAAUUUGCTT (SEQ ID NO: 130) | GCAAAUUGAGACAAGAAGGTT (SEQ ID NO:294) | 1.16 | 0.22 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-736** | TGGGTTTAACGCATTGTGA (SEQ ID NO:459) | UGGGUUUAACGCAUUGUGATT (SEQ ID NO: 131) | UCACAAUGCGUUAAACCCATT (SEQ ID NO:295) | 1.16 | 0.22 |
| **RAG7-170** | TTTGACCTTTCTTAGGGAC (SEQ ID NO:460) | UUUGACCUUUCUUAGGGACTT (SEQ ID NO:132) | GUCCCUAAGAAAGGUCAAATT (SEQ ID NO:296) | 1.16 | 0.21 |
| **RAG7-721** | GTGATAGTCCCGTTTTCTT (SEQ ID NO:461) | GUGAUAGUCCCGUUUUCUUTT (SEQ ID NO: 133) | AAGAAAACGGGACUAUCACTT (SEQ ID NO:297) | 1.16 | 0.21 |
| **RAG7-198** | TACAGGTGCCTTCTTGTCT (SEQ ID NO:462) | UACAGGUGCCUUCUUGUCUTT (SEQ ID NO: 134) | AGACAAGAAGGCACCUGUATT (SEQ ID NO:298) | 1.16 | 0.21 |
| **RAG7-722** | TGTGATAGTCCCGTTTTCT (SEQ ID NO:463) | UGUGAUAGUCCCGUUUUCUTT (SEQ ID NO: 135) | AGAAAACGGGACUAUCACATT (SEQ ID NO:299) | 1.15 | 0.21 |
| **RAG7-670** | CAGGGCTGTTATCTGCATA (SEQ ID NO:464) | CAGGGCUGUUAUCUGCAUATT (SEQ ID NO:136) | UAUGCAGAUAACAGCCCUGTT (SEQ ID NO:300) | 1.15 | 0.20 |
| **RAG7-86** | CCGTTTCAGGGGCGAGTGA (SEQ ID NO:465) | CCGUUUCAGGGGCGAGUGATT (SEQ ID NO: 137) | UCACUCGCCCCUGAAACGGTT (SEQ ID NO:301) | 1.15 | 0.20 |
| **RAG7-833** | GCCATTGAGGAAACTCCTC (SEQ ID NO:466) | GCCAUUGAGGAAACUCCUCTT (SEQ ID NO: 138) | GAGGAGUUUCCUCAAUGGCTT (SEQ ID NO:302) | 1.14 | 0.20 |
| **RAG7-832** | CCATTGAGGAAACTCCTCC (SEQ ID NO:467) | CCAUUGAGGAAACUCCUCCTT (SEQ ID NO: 139) | GGAGGAGUUUCCUCAAUGGTT (SEQ ID NO:303) | 1.14 | 0.19 |
| **RAG7-702** | CTCAGCCCAGGTCCTATGC (SEQ ID NO:468) | CUCAGCCCAGGUCCUAUGCTT (SEQ ID NO: 140) | GCAUAGGACCUGGGCUGAGTT (SEQ ID NO:304) | 1.14 | 0.19 |
| **RAG7-120** | TGATCTCCTGGGAAGAAAG (SEQ ID NO:469) | UGAUCUCCUGGGAAGAAAGTT (SEQ ID NO: 141) | CUUUCUUCCCAGGAGAUCATT (SEQ ID NO:305) | 1.14 | 0.19 |
| **RAG7-780** | AATGTAGCATCGAGTGGTA (SEQ ID NO:470) | AAUGUAGCAUCGAGUGGUATT (SEQ ID NO: 142) | UACCACUCGAUGCUACAUUTT (SEQ ID NO:306) | 1.14 | 0.19 |
| **RAG7-914** | CTTCTTTGAAAGCTCAAAG (SEQ ID NO:471) | CUUCUUUGAAAGCUCAAAGTT (SEQ ID NO: 143) | CUUUGAGCUUUCAAAGAAGTT (SEQ ID NO:307) | 1.14 | 0.19 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-93** | AAGGACACCGTTTCAGGGG (SEQ ID NO:472) | AAGGACACCGUUUCAGGGGTT (SEQ ID NO: 144) | CCCCUGAAACGGUGUCCUUTT (SEQ ID NO:308) | 1.14 | 0.19 |
| **RAG7-98** | CCGAAAAGGACACCGTTTC (SEQ ID NO:473) | CCGAAAAGGACACCGUUUCTT (SEQ ID NO:145) | GAAACGGUGUCCUUUUCGGTT (SEQ ID NO:309) | 1.14 | 0.19 |
| **RAG7-853** | AACTTGAAAGGTTCCGAGG (SEQ ID NO:474) | AACUUGAAAGGUUCCGAGGTT (SEQ ID NO: 146) | CCUCGGAACCUUUCAAGUUTT (SEQ ID NO:310) | 1.14 | 0.18 |
| **RAG7-885** | CCAGAAGTAAAGTGTTCTC (SEQ ID NO:475) | CCAGAAGUAAAGUGUUCUCTT (SEQ ID NO: 147) | GAGAACACUUUACUUCUGGTT (SEQ ID NO:311) | 1.14 | 0.18 |
| **RAG7-715** | GTCCCGTTTTCTTCTCAGC (SEQ ID NO:476) | GUCCCGUUUUCUUCUCAGCTT (SEQ ID NO: 148) | GCUGAGAAGAAAACGGGACTT (SEQ ID NO:312) | 1.13 | 0.18 |
| **RAG7-681** | CCTCATCTATGCAGGGCTG (SEQ ID NO:477) | CCUCAUCUAUGCAGGGCUGTT (SEQ ID NO: 149) | CAGCCCUGCAUAGAUGAGGTT (SEQ ID NO:313) | 1.13 | 0.17 |
| **RAG7-106** | GAAAGCTTCCGAAAAGGAC (SEQ ID NO:478) | GAAAGCUUCCGAAAAGGACTT (SEQ ID NO: 150) | GUCCUUUUCGGAAGCUUUCTT (SEQ ID NO:314) | 1.12 | 0.17 |
| **RAG7-137** | TCCTGCTCTTTGTCCGCTG (SEQ ID NO:479) | UCCUGCUCUUUGUCCGCUGTT (SEQ ID NO: 151) | CAGCGGACAAAGAGCAGGATT (SEQ ID NO:315) | 1.12 | 0.16 |
| **RAG7-165** | CCTTTCTTAGGGACTTGTT (SEQ ID NO:480) | CCUUUCUUAGGGACUUGUUTT (SEQ ID NO: 152) | AACAAGUCCCUAAGAAAGGTT (SEQ ID NO: 316) | 1.11 | 0.16 |
| **RAG7-160** | CTTAGGGACTTGTTTTCTG (SEQ ID NO:481) | CUUAGGGACUUGUUUUCUGTT (SEQ ID NO: 153) | CAGAAAACAAGUCCCUAAGTT (SEQ ID NO: 317) | 1.11 | 0.15 |
| **RAG7-745** | AAAACCTCCTGGGTTTAAC (SEQ ID NO:482) | AAAACCUCCUGGGUUUAACTT (SEQ ID NO: 154) | GUUAAACCCAGGAGGUUUUTT (SEQ ID NO: 318) | 1.11 | 0.15 |
| **RAG7-92** | AGGACACCGTTTCAGGGGC (SEQ ID NO:483) | AGGACACCGUUUCAGGGGCTT (SEQ ID NO: 155) | GCCCCUGAAACGGUGUCCUTT (SEQ ID NO: 319) | 1.11 | 0.15 |
| **RAG7-107** | AGAAAGCTTCCGAAAAGGA (SEQ ID NO:484) | AGAAAGCUUCCGAAAAGGATT (SEQ ID NO: 156) | UCCUUUUCGGAAGCUUUCUTT (SEQ ID NO:320) | 1.11 | 0.15 |

(continued)

| saRNA | Active target sequence (5'-3') | Sense sequence (5'-3') | Antisense sequence (5'-3') | Fold of changes in relative LHPP mRNA expression level | Fold of changes in relative LHPP mRNA expression level ($log_2$) |
|---|---|---|---|---|---|
| **RAG7-826** | AGGAAACTCCTCCCTTTTA (SEQ ID NO:485) | AGGAAACUCCUCCCUUUUATT (SEQ ID NO:157) | UAAAAGGGAGGAGUUUCCUTT (SEQ ID NO:321) | 1.11 | 0.15 |
| **RAG7-839** | CGAGGGGCCATTGAGGAAA (SEQ ID NO:486) | CGAGGGGCCAUUGAGGAAATT (SEQ ID NO:158) | UUUCCUCAAUGGCCCCUCGTT (SEQ ID NO:322) | 1.11 | 0.15 |
| **RAG7-738** | CCTGGGTTTAACGCATTGT (SEQ ID NO:487) | CCUGGGUUUAACGCAUUGUTT (SEQ ID NO:159) | ACAAUGCGUUAAACCCAGGTT (SEQ ID NO:323) | 1.11 | 0.15 |
| **RAG7-911** | CTTTGAAAGCTCAAAGCTA (SEQ ID NO:488) | CUUUGAAAGCUCAAAGCUATT (SEQ ID NO:160) | UAGCUUUGAGCUUUCAAAGTT (SEQ ID NO:324) | 1.11 | 0.15 |
| **RAG7-883** | AGAAGTAAAGTGTTCTCGT (SEQ ID NO:489) | AGAAGUAAAGUGUUCUCGUTT (SEQ ID NO:161) | ACGAGAACACUUUACUUCUTT (SEQ ID NO:325) | 1.11 | 0.14 |
| **RAG7-687** | ATGCATCCTCATCTATGCA (SEQ ID NO:490) | AUGCAUCCUCAUCUAUGCATT (SEQ ID NO:162) | UGCAUAGAUGAGGAUGCAUTT (SEQ ID NO:326) | 1.10 | 0.14 |
| **RAG7-851** | CTTGAAAGGTTCCGAGGGG (SEQ ID NO:491) | CUUGAAAGGUUCCGAGGGGTT (SEQ ID NO:163) | CCCCUCGGAACCUUUCAAGTT (SEQ ID NO:327) | 1.10 | 0.14 |
| **RAG7-142** | GCTTTTCCTGCTCTTTGTC (SEQ ID NO:492) | GCUUUUCCUGCUCUUUGUCTT (SEQ ID NO:164) | GACAAAGAGCAGGAAAAGCTT (SEQ ID NO:328) | 1.10 | 0.14 |

When the 290 saRNAs were sorted by their targeting positions on the LHPP promoter, it can be clearly seen that the functional saRNAs were distributed across the promoter region in a cluster fashion, i.e., at certain promoter regions, there were "hot spots" where functional sRNAs were enriched **(FIG. 3).** As shown in **FIG. 3,** there are five hot spots highly enriched for activating saRNAs in the region (H1) from -917 to -844, the region (H2) from -710 to - 675, the region (H3) from -198 to -168, the region (H4) from -151 to -28, and the region (H5) from -845 to -711 of the promoter. This study result indicates that the activating saRNAs are not randomly distributed on the promoter, but instead they are enriched in the specific hot spot regions.

The sequence of the hot spot H1 (5' to 3': -917 to -844) corresponds to position 1 to position 74 from 5' to 3' of SEQ ID NO: 500:
tctcttc tttgaaagct caaagctatg ttggaccaga agtaaagtgt tctcgtttct atttaataac ttgaaag

The sequence of the hot spot H2 (5' to 3': -710 to -675) corresponds to position 1 to position 36 from 5' to 3' of SEQ ID NO: 501:
gttttcttct cagcccaggt cctatgcatc ctcatc

The sequence of the hot spot H3 (5' to 3': -198 to -168) corresponds to position 1 to position 31 from 5' to 3' of SEQ ID NO: 502:
tacaggtg ccttcttgtc tcaatttgcc ttt

The sequence of the hot spot H4 (5' to 3': -151 to -28) corresponds to position 1 to position 124 from 5' to 3' of SEQ ID NO: 503:
t tgttttctgc ttttcctgct ctttgtccgc tgatctcctg ggaagaaagc ttccgaaaag gacaccgttt caggggcgag tgacgccggg gtgcccaggc cgcgccccag ttccgggttt gca

The sequence of the hot spot HC (5' to 3': -845 to -711) corresponds to position 1 to position 135 from 5' to 3' of SEQ ID NO: 504:
aggtt ccgaggggcc attgaggaaa ctcctccctt ttaatatcaa tgtgtattta ttgcaaaaat aatgtagcat cgagtggtat tttatagctt atccaaaaac ctcctgggtt taacgcattg tgatagtccc.

*Example 3*

saRNAs Promoted LHPP mRNA Expression and Inhibited Tumor Cell Proliferation

The 290 saRNAs targeting the LHPP promoter were individually transfected into Huh7 cells, and 72 hour later, one-step RT-qPCR was employed to analyze the expression levels of LHPP mRNA and cell viability was detected by the CCK-8 method. As shown in **FIG. 4,** cell viability decreased when the activating saRNAs promoted the LHPP mRNA expression, and there was a negative correlation between the LHPP mRNA expression level and the cell viability.

The cell viability was detected by the following CCK-8 method: the cCells were plated into a 96-well plate at 3-5 $\times 10^3$ cells/well, cultured overnight, and transfected with the oligonucleotide duplexes. After 72 hour of transfection, 10 uL of CCK-8 solution (Dojindo Molecular Technologies) was added into each well. After 1 hour of incubation at 37°C, a microplate reader was used to measure absorbances at 450 nm.

*Example 4*

saRNAs Promoted LHPP Protein Expression

Cells were plated into a 96-well plate at 3-5 $\times 10^3$ cells/well, cultured overnight, and transfected with 10 randomly selected oligonucleotide duplexes. After 72 h of transfection, the cells were collected and lysed using cell lysis buffer (1 $\times$RIPA buffer, CST) containing protease inhibitor. Protein quantification was performed by using the BCA method (Thermo). After polyacrylamide gel electrophoresis separation, then the protein was transferred to a 0.45 $\mu$m PVDF membrane. The primary antibody used for the blot assay was a mouse monoclonal anti-LHPP antibody (Invitrogen), a rabbit polyclonal anti-AKT antibody (Cell Signaling Technology), a rabbit polyclonal anti-pAKT antibody (Cell Signaling Technology), or a rabbit polyclonal anti-$\alpha/\beta$-tubulin antibody (Cell Signaling Technology); and the secondary antibody used was an anti-mouse IgG HRP-linked antibody (Cell Signaling Technology) or an anti-rabbit IgG HRP-linked antibody (Cell Signaling Technology). Image Lab (BIO-RAD, Chemistry Doctm MP imaging system) was used to scan detecting signals.

Table 5. Double-stranded RNA sequences as study controls

| Double-stranded RNA | Sequence No. | Sequence (5'-3') |
|---|---|---|
| **dsCon2-sense strand** | SEQ ID NO:505 | ACUACUGAGUGACAGUAGATT |
| **dsCon2-antisense strand** | SEQ ID NO:506 | UCUACUGUCACUCAGUAGUTT |

(continued)

| Double-stranded RNA | Sequence No. | Sequence (5'-3') |
|---|---|---|
| **siLHPP1-sense strand** | SEQ ID NO:507 | GAAGUUCAGAGCCGCUCAATT |
| **siLHPP1-antisense strand** | SEQ ID NO:508 | UUGAGCGGCUCUGAACUUCTT |

As shown in **FIG. 5,** the 10 randomly selected saRNAs downregulated the phosphorylation of AKT, while promoting or increasing the LHPP mRNA and protein expression.

*Example 5*

saRNAs Inhibited Proliferation of A Variety of Tumor Cells

In order to further evaluate the effect of LHPP saRNAs in inducing the mRNA expression of the LHPP gene and inhibiting the proliferation of cancer cells, eight screened saRNAs (RAG7-132, RAG7-133, RAG7-139, RAG7-177, RAG7-178, RAG7-694, RAG7-707 and RAG7-892) each were transfected into the liver cancer cell lines Huh7 (Medical Cell Resource Center, Tohoku University, Japan), HepG2 (ATCC), Hep3B (ATCC), Li-7 (Medical Cell Resource Center, Tohoku University, Japan) and SK-HEP-1 (ATCC), a lung cancer cell line A549 (ATCC), a bladder cancer cell line T24 (ATCC), a prostatic cancer cell line PC3 (ATCC), and a glioma cell line U87MG (ATCC). The mRNA expression and cell viability of the transfected cells were measured. As shown in **FIG. 6,** RAG7-133 induced the expression of LHPP gene to different degrees and inhibited cell proliferation in all five liver cancer cell lines; and RAG7-694 induced the expression of LHPP gene to different degrees and inhibited cell proliferation in four of the liver cancer cell lines, other than Li-7. In another aspect, all of the aforementioned 8 saRNAs induced the expression of LHPP gene to different degrees and inhibited cell proliferation in the cell lines HepG2 and SK-HEP-1 . As shown in **FIG. 7,** RAG7-133 induced the expression of LHPP gene to different degrees and inhibited cell proliferation in the cell lines T24, PC3, and U87MG; RAG7-694 induced the expression of LHPP gene to different degrees and inhibited cell proliferation in the cell lines A549, T24 and PC3; RAG7-177 induced the expression of LHPP gene to different degrees and inhibited cell proliferation in the cell lines A549, T24, and U87MG; and RAG7-178 induced the expression of LHPP gene to different degrees and inhibited cell proliferation in the cell lines A549, PC3, and U87MG.

*Example 6*

saRNAs in Combination with Chemotherapies Inhibited Cell Proliferation

The compounds used in the study included: Sorafenib (Sora) (SELLECK, S1040), Lenvatinib (Lenv) (SELLECK, S1164), Regorafenib (Rego) (SELLECK, S1178), and Cabozantinib (Cabo) (SELLECK, S1119). Cells were transfected with each candidate saRNAs at varying concentration gradients for 24 hours. Thereafter, the aforementioned compounds were added to the transfected cells at a concentration of 5 μM and the cells were incubated with the compounds for 48 hours. Cell viability was measured using the CCK-8 method. Compusyn© version 1.0 software (ComboSyn, Inc. Paramus, NJ, USA) was used to analyze the combination index (CI) of drugs, wherein CI < 1 represented a synergistic effect, CI = 1 represented an additive effect, and CI > 1 represented an antagonistic effect.

As shown for HepG2 cells in **FIG. 8,** a high dose (25 nM to 100 nM) of RAG7-133 saRNA and Regorafenib had a strong synergistic effect (CI < 0.3); a low dose (1.0 nM to 10 nM) of RAG7-133 and Regorafenib had a synergistic effect (0.3 < CI < 0.7); and RAG7-133 saRNA had a synergistic effect with Sorafenib and Cabozantinib (CI <1) , but did not have a synergistic effect with Lenvatinib (CI > 1). As shown for U87MG cells in **FIG. 9,** RAG7-133 saRNA had a synergistic effect (CI < 1) with all the four compounds, in particular, when used in combination with Lenvatinib or Cabozantinib. RAG7-133 saRNA had an extremely strong synergistic effect (CI < 0.1) with the compounds within a wide dosage range (1.0 nM to 100 nM).

*Example 7*

Drug Combination Inhibited Tumor Growth *in vivo* in Mice Xenograped with Human HepG2

To prepare the saRNA formulation, the in vivo-jetPEI (201-10G, Polyplus-transfection, France) was adopted as an saRNA delivery system. The preparation process is briefly described as follows. An saRNA was first diluted in 10% glucose solution to obtain a solution A. According to the instructions of the manufacturer, a required amount of in vivo-jetPEI was diluted in 10% glucose solution to obtain a solution B. Equal volumes of the solution A and the solution B were mixed (nitrogen-to-phosphorus ratio: 8; final concentration of glucose: 5%). After mixing, the mixture was let to stand at room temperature for 15 minutes for later use.

HepG2 cells in the logarithmic growth phase were obtained and counted, and then the cell suspension was regulated to $5 \times 10^7$ cells/mL and subcutaneously inoculated into the right armpit of BALB/c nude mice at a volume of 0.1 mL per mouse. When tumors in nude mice grew to about 100 $mm^3$, the nude mice were randomly divided into four groups each with six mice: (vehicle control (Vehicle) group, saRNA group, regorafenib group, and saRNA and regorafenib combination group (saRNA+regorafenib)). For the saRNA group and the saRNA + regorafenib group, intratumor injection of saRNA was performed at 1 $mg \cdot kg^{-1}$ on days 1, 4, 7 and 10. For the regorafenib group and the saRNA + regorafenib group, intragastric administration of regorafenib was performed at 3 mg $kg^{-1}$ everyday from day 1 through day 12. Starting from the initial administration, the long diameter and the short diameter of each tumor were measured with a vernier caliper every two days. The tumor volume was calculated according to the formula $V = (1 \times w^2)/2$, wherein 1 represents the longest diameter of the tumor and w represents the diameter parallel to the surface of the tumor and perpendicular to the long diameter. A tumor growth curve and size and morphology of the tumor after anatomy were recorded during the administration. As shown in **FIG. 10,** compared with the vehicle control group (Vehicle), the tumors began to show the tendency to grow slowly and shrink as of day 7 in the saRNA group (given RAG7-133 alone), and by day 13, the tumor volume increased by 34% compared with that at the beginning of treatment in the saRNA group, while the tumor volume increased by 118% in the vehicle control group. There is a significant difference ($P < 0.05$) between the tumor volume changes of the two groups, indicating that the LHPP saRNA can remarkably inhibit tumor growth in vivo in mice. In the saRNA and regorafenib combination group (RAG7-133 + regorafenib), the tumors began to show the tendency to grow slowly as of day 4 and began to shrink on day 7 and by day 13 the tumor volume only increased by 4% compared with that at the beginning of treatment, while the tumor volume in the group given the chemotherapy regorafenib (Rego) alone increased by 70% on day 13 of treatment compared with that at the beginning of treatment. There is a significant difference ($P < 0.01$) between the tumor volume changes of the two groups, indicating that saRNA in combination with the chemotherapy synergistically enhances the cancer inhibition effect of the chemotherapy.

*Example 8*

Drug Combination Inhibited Tumor Growth in Vivo in Mice Xenograped with Human U87MG

To prepare the saRNA formulation, in vivo-jetPEI (201-10G, Polyplus-transfection, France) was adopted as an saRNA delivery system. The preparation process is briefly described as follows: an saRNA was first diluted in a 10% glucose solution to give a solution A; a required amount of in vivo-jetPEI was diluted in 10% glucose solution to give a solution B; then, equal volumes of the solution A and the solution B were mixed (nitrogen-to-phosphorus ratio: 8; final concentration of glucose: 5%). After mixing the mixture was let to stand under room temperature for 15 minutes for later use.

The glioma cell line U87MG were grown to the logarithmic phase, counted, then subcutaneously inoculated at a concentration of $9 \times 10^7$ cells/mL into the right armpit of BALB/c nude mice at 0.1 mL per mouse. Tumor-bearing nude mice were randomly divided into four groups after tumors grew to about 100 $mm^3$ (vehicle control group, saRNA group, regorafenib group, and saRNA and regorafenib in combination group (RAG7-133 + regorafenib group)) with seven mice in each group. For the saRNA group and the saRNA + regorafenib group, intratumor injection of saRNA at 1 $mg \cdot kg^{-1}$ was performed on days 1, 4, 7 and 10. For the regorafenib group and the saRNA+regorafenib group, intragastric administeration of regorafenib at 3 $mg \cdot kg^{-1}$ was performed every day on day 1 through day 12 . After the initial administration, the long diameter and short diameter of each tumor were measured with a vernier caliper every two days. The tumor volume was calculated according to the formula $V = (1 \times w^2)/2$, wherein 1 represents the longest diameter of the tumor and w represents the diameter parallel to the surface of the tumor and perpendicular to the long diameter. A tumor growth curve and size and morphology of the tumor anatomy were recorded during the administration. As shown in **FIG. 11,** compared with the vehicle control group (Vehicle), the tumor volume increased by 167% on day 13 when compared with that at the beginning of treatment in the saRNA group (given RAG7-133 alone), while the tumor volume increased by 406% in the control group. There is a significant difference ($P < 0.05$) between the tumor volume changes of the two groups, indicating that the LHPP saRNA can remarkably inhibit tumor growth in vivo in mice. In the saRNA and regorafenib combination group (RAG7-133 + regorafenib), the tumor volume increased by 132% on day 13 compared with that at the beginning of treatment, while the tumor volume increased by 251% on day 13 compared with that at the beginning of treatment in the group given regorafenib (Rego) alone. There is a significant difference ($P < 0.05$) between the tumor volume changes of the two groups, indicating that the saRNA in combination with the chemotherapy synergistically enhances the cancer inhibition effect of the chemotherapy.

Based on the results above, a plurality of saRNAs capable of remarkably activating the expression of LHPP gene were identified through high-throughput screening of saRNAs targeting LHPP gene promoter. These saRNAs inhibit the proliferation of tumor cells *in vitro* or *in vivo* by up-regulating the expression of LHPP gene and protein and downregulating the phosphorylation of AKT. These results clearly suggest that saRNAs targeting the LHPP gene promoter can be a promising strategy for tumor treatment.

References

1. Yokoi F, Hiraishi H, Izuhara K. 2003. Molecular cloning of a cDNA for the human phospholysine phosphohistidine inorganic pyrophosphate phosphatase. J Biochem 133:607-14.
2. Neff CD, Abkevich V, Packer JC, Chen Y, Potter J, et al. 2009. Evidence for HTR1A and LHPP as interacting genetic risk factors in major depression. Mol Psychiatry 14:621-30.
3. Gohla A. 2019. Do metabolic HAD phosphatases moonlight as protein phosphatases? Biochim Biophys Acta Mol Cell Res 1866:153-66.
4. CONVERGE consortium. 2015. Sparse whole-genome sequencing identifies two loci for major depressive disorder. Nature 523:588-91.
5. Knowles EE, Kent JW, Jr., McKay DR, Sprooten E, Mathias SR, et al. 2016. Genome-wide linkage on chromosome 10q26 for a dimensional scale of major depression. J Affect Disord 191:123-31.
6. Polimanti R, Wang Q, Meda SA, Patel KT, Pearlson GD, et al. 2017. The Interplay Between Risky Sexual Behaviors and Alcohol Dependence: Genome-Wide Association and Neuroimaging Support for LHPP as a Risk Gene. Neuropsychopharmacology 42:598-605.
7. Cui L, Gong X, Tang Y, Kong L, Chang M, et al. 2016. Relationship between the LHPP Gene Polymorphism and Resting-State Brain Activity in Major Depressive Disorder. Neural Plast 2016:9162590.
8. Lesseur C, Diergaarde B, Olshan AF, Wunsch-Filho V, Ness AR, et al. 2016. Genome-wide association analyses identify new susceptibility loci for oral cavity and pharyngeal cancer. Nat Genet 48:1544-50.
9. Gutierrez-Camino A, Martin-Guerrero I, Garcia-Orad A. 2017. Genetic susceptibility in childhood acute lymphoblastic leukemia. Med Oncol 34:179.
10. Vijayakrishnan J, Kumar R, Henrion MY, Moorman AV, Rachakonda PS, et al. 2017. A genome-wide association study identifies risk loci for childhood acute lymphoblastic leukemia at 10q26.13 and 12q23.1. Leukemia 31:573-9.
11. Hindupur SK, Colombi M, Fuhs SR, Matter MS, Guri Y, et al. 2018. The protein histidine phosphatase LHPP is a tumour suppressor. Nature 555:678-82.
12. Bray F, Ferlay J, Soerjomataram I, Siegel RL, Torre LA, Jemal A. 2018. Global cancer statistics 2018: GLOBOCAN estimates of incidence and mortality worldwide for 36 cancers in 185 countries. CA Cancer J Clin 68:394-424.

## Claims

1. A small activating nucleic acid molecule, comprising a first nucleic acid strand and a second nucleic acid strand, wherein the first nucleic acid strand has at least 75% homology or complementarity to a sequence of 16 to 35 continuous nucleotides in positions -917 to -844 (SEQ ID NO: 500), -710 to -675 (SEQ ID NO: 501), -198 to -168 (SEQ ID NO: 502), -151 to -28 (SEQ ID NO: 503) or -845 to -711 (SEQ ID NO: 504) upstream of the transcription start site in a promoter region of an LHPP gene, and the first nucleic acid strand and the second nucleic acid strand complementarily form a double-stranded nucleic acid structure capable of activating the expression of the LHPP gene in a cell.

2. The small activating nucleic acid molecule of claim 1, wherein the first nucleic acid strand and the second nucleic acid strand are present on two different nucleic acid strands.

3. The small activating nucleic acid molecule of claim 1, wherein the first nucleic acid strand and the second nucleic acid strand are present on the same nucleic acid strand, and preferably, the small activating nucleic acid molecule is a hairpin single-stranded nucleic acid molecule, wherein the first nucleic acid strand and the second nucleic acid strand comprise complementary regions forming the double-stranded nucleic acid structure.

4. The small activating nucleic acid molecule of claim 2, wherein at least one strand of the small activating nucleic acid molecule has an overhang of 0 to 6 nucleotides in length at the 3' terminus of the strand.

5. The small activating nucleic acid molecule of claim 4, wherein both strands of the small activating nucleic acid molecule have overhangs of 0 to 6 nucleotides in length at the 3' terminus of each strand, preferably the overhangs are of 2 or 3 nucleotides in length.

6. The small activating nucleic acid molecule of any of claims 1-5, wherein the first nucleic acid strand and the second nucleic acid strand independently have 16 to 35 nucleotides in length.

7. The small activating nucleic acid molecule of any of claims 1-6, wherein one strand of the small activating nucleic acid molecule comprises a nucleic acid sequence having at least 75% homology or complementarity to any nucleotide sequence chosen from SEQ ID NOs: 329-492, or consists of a nucleic acid sequence having at least 75% homology or complementarity to any nucleotide sequence chosen from SEQ ID NOs: 329-492.

8. The small activating nucleic acid molecule of claims 1-7, wherein the first nucleic acid strand has at least 75% homology to any nucleotide sequence chosen from SEQ ID NOs: 1-164, and the second nucleic acid strand has at least 75% homology to any nucleotide sequence chosen from SEQ ID NOs: 165-328.

9. The small activating nucleic acid molecule of claims 1-8, wherein the first nucleic acid strand comprises any nucleotide sequence chosen from SEQ ID NOs: 1-164, or consists of any nucleotide sequence chosen from SEQ ID NOs: 1-164; and the second nucleic acid strand comprises any nucleotide sequence chosen from SEQ ID NOs: 165-328, or consists of any nucleotide sequence selected from SEQ ID NOs: 165-328.

10. The small activating nucleic acid molecule of any of claims 1-9, wherein the small activating nucleic acid molecule comprises at least one modification, and preferably, the modification is a chemical modification.

**11.** The small activating nucleic acid molecule of claim 10, wherein the chemical modification comprises or is chosen from at least one or more of the following modifications:

(1) a modification of a phosphodiester bond connecting nucleotides in the nucleotide sequence of the small activating nucleic acid molecule;
(2) a modification of 2'-OH of a ribose in the nucleotide sequence of the small activating nucleic acid molecule; and
(3) a modification of a base in the nucleotide sequence of the small activating nucleic acid molecule; and
(4) at least one nucleotide in the nucleotide sequence of the small activating nucleic acid molecule being a locked nucleic acid.

**12.** The small activating nucleic acid molecule of claim 10, wherein the chemical modification comprises or is chosen from at least one or more of the following modifications: 2'-fluoro modification, 2'-oxymethyl modification, 2'-oxyethylidene methoxy modification, 2,4'-dinitrophenol modification, locked nucleic acid (LNA), 2'-amino modification, 2'-deoxy modification, 5'-bromouracil modification, 5'-iodouracil modification, N-methyluracil modification, 2,6-diaminopurine modification, phosphorothioate modification, and boranophosphate modification.

**13.** The small activating nucleic acid molecule of any of claims 1-12, wherein the small activating nucleic acid molecule activates/upregulates the expression of the LHPP gene by at least 10%.

**14.** A nucleic acid coding the small activating nucleic acid molecule of any of claims 1-9.

**15.** The nucleic acid of claim 14, wherein the nucleic acid is a DNA molecule.

**16.** A cell comprising the small activating nucleic acid molecule of any of claims 1-13 or the nucleic acid of claim 14 or 15.

**17.** A composition comprising the small activating nucleic acid molecule of any of claims 1-13, the nucleic acid of claim 14 or 15, or the cell of claim 16, and, optionally, a pharmaceutically acceptable carrier.

**18.** The composition of claim 17, wherein the pharmaceutically acceptable carrier comprises or is chosen from a liposome, a high-molecular polymer, and a polypeptide.

**19.** The composition of claim 17 or 18, wherein the composition comprises 1-150 nM of the small activating nucleic acid molecule.

**20.** A kit comprising the small activating nucleic acid molecule of any of claims 1-13, the nucleic acid of claim 14 or 15, the cell of claim 16, or the composition of any of claims 17-19.

**21.** A use of the small activating nucleic acid molecule of any of claims 1-13, the nucleic acid of claim 14 or 15, or the composition of any of claims 17-19 in preparing a formulation for activating/up-regulating the expression of the LHPP gene in a cell.

**22.** The use of claim 21, wherein the small activating nucleic acid molecule is directly introduced into the cell.

**23.** The use of claim 21, wherein the small activating nucleic acid molecule is produced in the cell after a nucleotide sequence coding the small activating nucleic acid molecule is introduced into the cell.

**24.** The use of any of claims 21-23, wherein the cell comprises a mammalian cell.

**25.** The use of claim 21, wherein the cell is a human cell.

**26.** The use of claim 25, wherein the cell is present in a human body.

**27.** The use of claim 26, wherein the human body is a patient suffering from a disease or condition related to insufficient or decreased expression of LHPP protein, and the small activating nucleic acid molecule is administered at a sufficient dose to treat the disease or condition.

**28.** The use of claim 27, wherein the disease or condition related to insufficient or decreased expression of LHPP protein comprises tumors, preferably solid tumors, and more preferably liver cancer, lung cancer, bladder cancer, prostatic cancer, and glioma.

**29.** An isolated small activating nucleic acid molecule target site of an LHPP gene, wherein the target site comprises or is chosen from a sequence of continuous 16 to 35 nucleotides in any sequences set forth in SEQ ID NOs: 500-504.

**30.** The small activating nucleic acid molecule target site of claim 29, wherein the target site comprises or is chosen from any of nucleotide sequences set forth in SEQ ID NOs: 329-492.

**31.** A method for activating/upregulating the expression of the LHPP gene in a cell, comprising administering the small activating nucleic acid molecule of any of claims 1-13, the nucleic acid of claim 14 or 15, or the composition of any of claims 17-19 to the cell.

**32.** The method of claim 31, wherein the small activating nucleic acid molecule is directly introduced into the cell.

**33.** The method of claim 31, wherein the small activating nucleic acid molecule is produced in the cell after the nucleic acid coding the small activating nucleic acid molecule is introduced into the cell.

**34.** The method of any of claims 31-33, wherein the cell comprises a mammalian cell.

**35.** The method of claim 31, wherein the cell is a human cell.

**36.** The method of claim 35, wherein the cell is present in a human body.

**37.** The method of claim 36, wherein the human body is a patient suffering from a disease or condition related to insufficient or decreased expression of LHPP protein, and the small activating nucleic acid molecule is administered at a sufficient dose to treat the disease or condition.

**38.** The method of claim 36, wherein the disease or condition related to insufficient or decreased expression of LHPP protein comprises tumors, preferably solid tumors, and more preferably liver cancer, lung cancer, bladder cancer, prostatic cancer, and glioma.

**39.** A method for treating a disease or condition related to insufficient or decreased expression of LHPP protein in a subject, comprising administering to the subject a therapeutically effective amount of the small activating nucleic acid molecule of any of claims 1-13, the nucleic acid of claim 14 or 15, the cell of claim 16, or the composition of any of claims 17-19 .

**40.** The method of claim 39, wherein the disease or condition related to insufficient or decreased expression of LHPP protein comprises tumors, preferably solid tumors, and more preferably liver cancer, lung cancer, bladder cancer, prostatic cancer, and glioma.

**41.** The method of claim 39 or 40, wherein the subject is a mammal.

**42.** The method of claim 39, wherein the subject is a human.

**43.** The method of any of claims 39-42, wherein the method further comprises administering a chemotherapeutic agent, radiotherapy, cell therapy, micromolecule, polypeptide, protein, antibody, or other anti-tumor drugs to the subject, wherein the chemotherapeutic agent preferably comprises or is chosen from Sorafenib, Lenvatinib, Regorafenib and Cabozantinib.

**44.** A use of the small activating nucleic acid molecule of any of claims 1-13, the nucleic acid of claim 14 or 15, the cell of claim 16, or the composition of any of claims 17-19 in the preparation of a medicament for treating a disease or condition related to insufficient or decreased expression of LHPP protein.

**45.** The use of claim 44, wherein the disease or condition related to insufficient or decreased expression of LHPP protein comprises tumors, preferably comprises solid tumors, and more preferably comprises or is chosen from liver cancer, lung cancer, bladder cancer, prostatic cancer, and glioma.

**46.** A use of the small activating nucleic acid molecule of any of claims 1-13, the nucleic acid of claim 14 or 15, the cell of claim 16, or the composition of any of claims 17-19 in the preparation of a medicament for treating a disease caused by insufficient expression of LHPP protein.

**47.** The use of any of claims 44-46, wherein the subject is a mammal.

**48.** The use of any of claims 44-46, wherein the subject is a human.

**49.** A use of the small activating nucleic acid molecule of any of claims 1-13, the nucleic acid of claim 14 or 15, the cell of claim 16 or the composition of any of claims 17-19 and a chemotherapeutic agent in the preparation of a medicament or pharmaceutical composition for treating a disease or condition related to insufficient or decreased expression of LHPP protein, wherein the chemotherapeutic agent preferably comprises or is chosen from Sorafenib, Lenvatinib, Regorafenib and Cabozantinib.

**50.** The use of claim 49, wherein the disease or condition related to insufficient or decreased expression of LHPP protein comprises tumors, preferably comprises solid tumors, and more preferably comprises or is chosen from liver cancer, lung cancer, bladder cancer, prostatic cancer, and glioma.

**51.** The use of claim 49 or 50, wherein the subject is a mammal.

**52.** The use of claim 49 or 50, wherein the subject is a human.

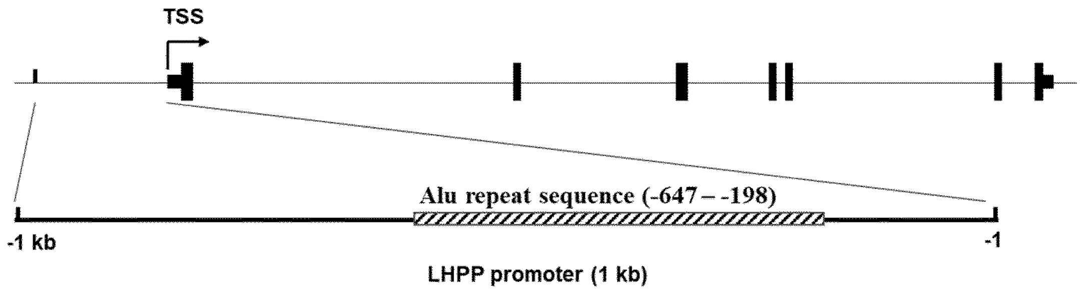


*FIG. 1*

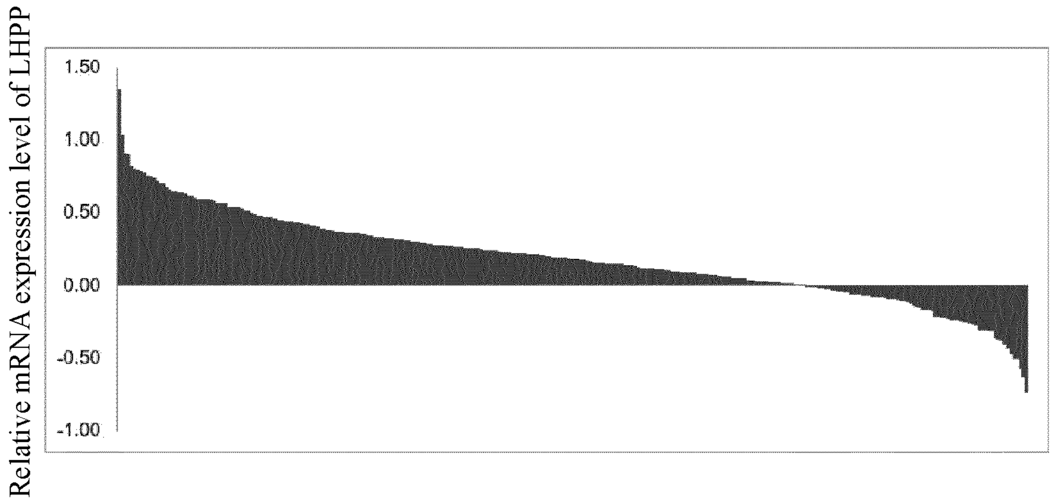


*FIG. 2*

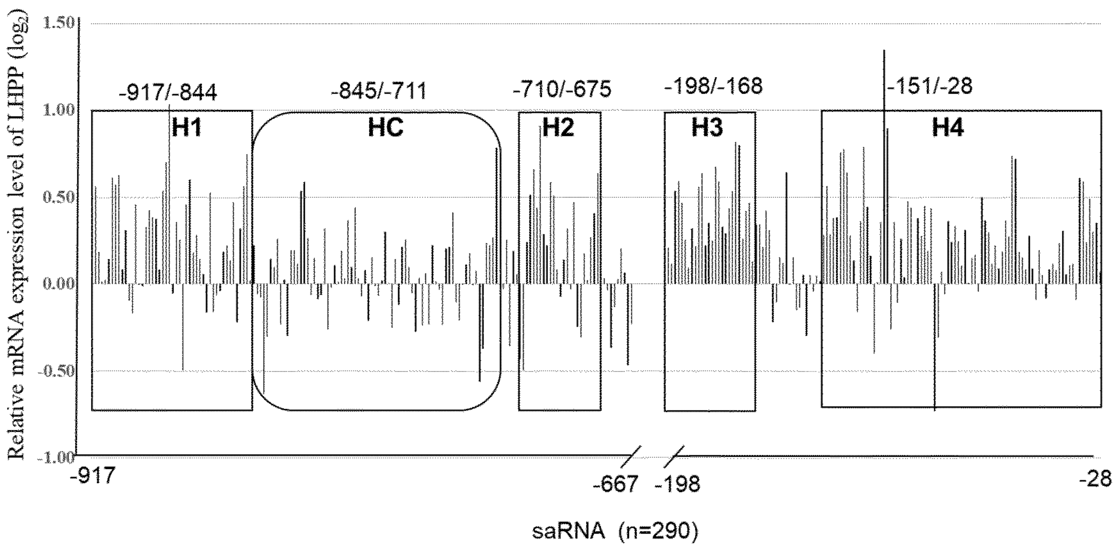


*FIG. 3*

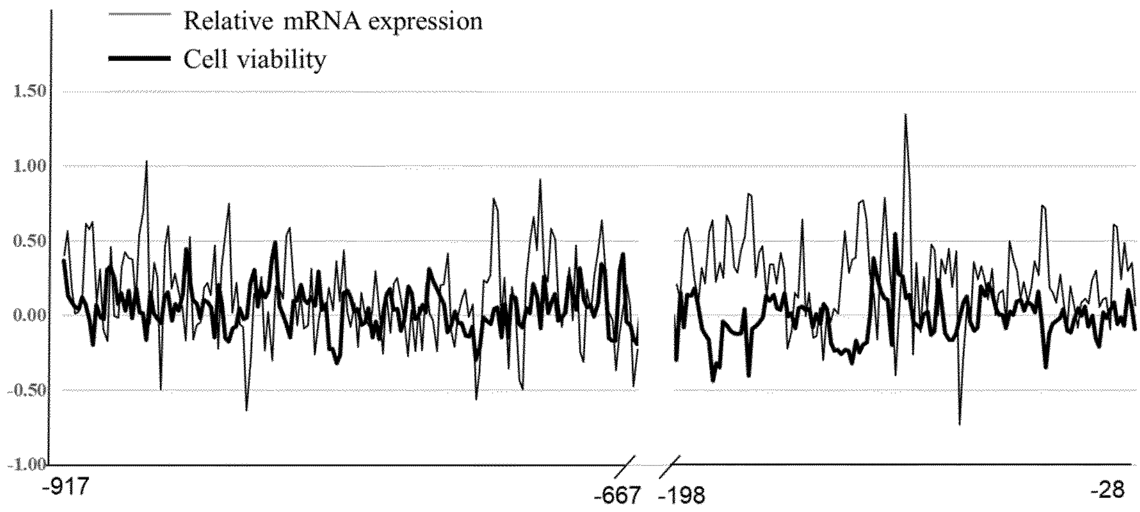
Relative mRNA expression
Cell viability
1.50
1.00
0.50
0.00
-0.50
-1.00
-917
-667
-198
-28

*FIG. 4*

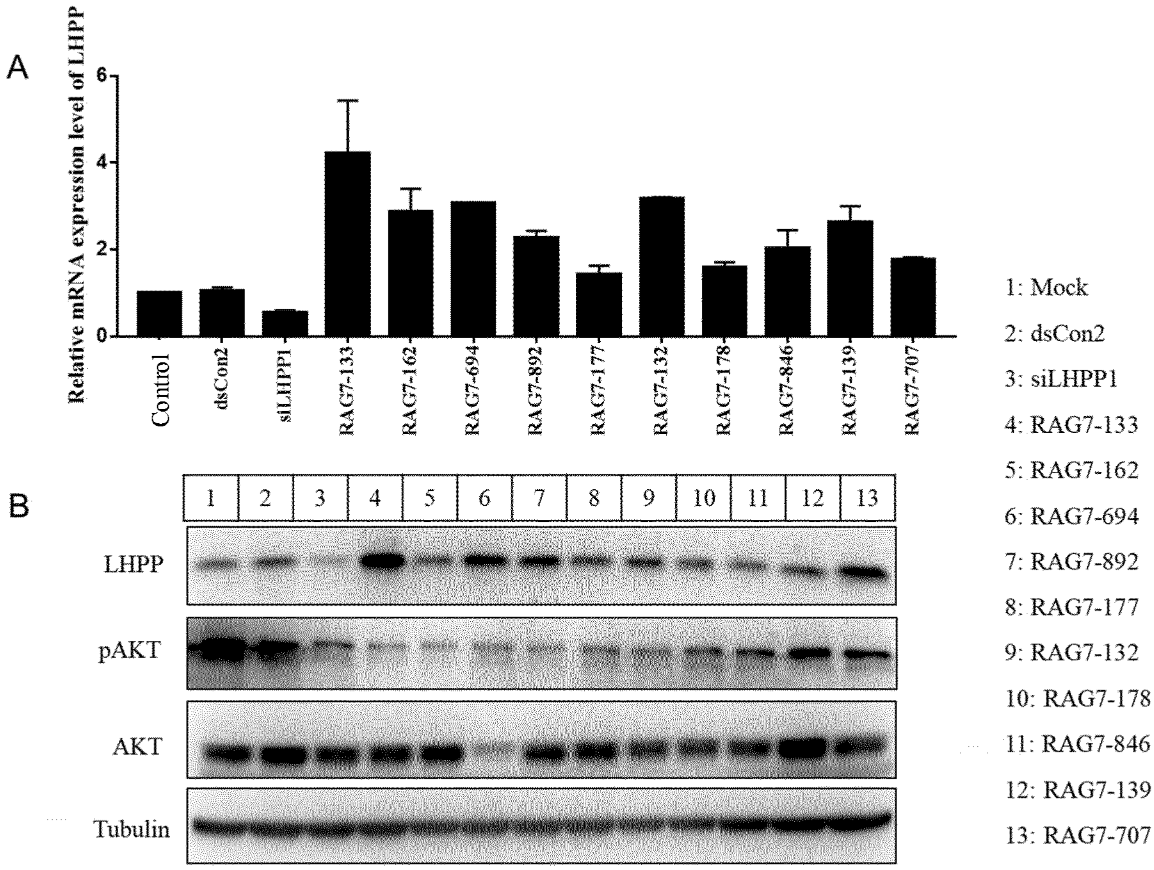
A
Relative mRNA expression level of LHPP
6
4
2
0
Control
dsCon2
siLHPP1
RAG7-133
RAG7-162
RAG7-694
RAG7-892
RAG7-177
RAG7-132
RAG7-178
RAG7-846
RAG7-139
RAG7-707
B
1 2 3 4 5 6 7 8 9 10 11 12 13
LHPP
pAKT
AKT
Tubulin
1: Mock
2: dsCon2
3: siLHPP1
4: RAG7-133
5: RAG7-162
6: RAG7-694
7: RAG7-892
8: RAG7-177
9: RAG7-132
10: RAG7-178
11: RAG7-846
12: RAG7-139
13: RAG7-707

*FIG. 5*

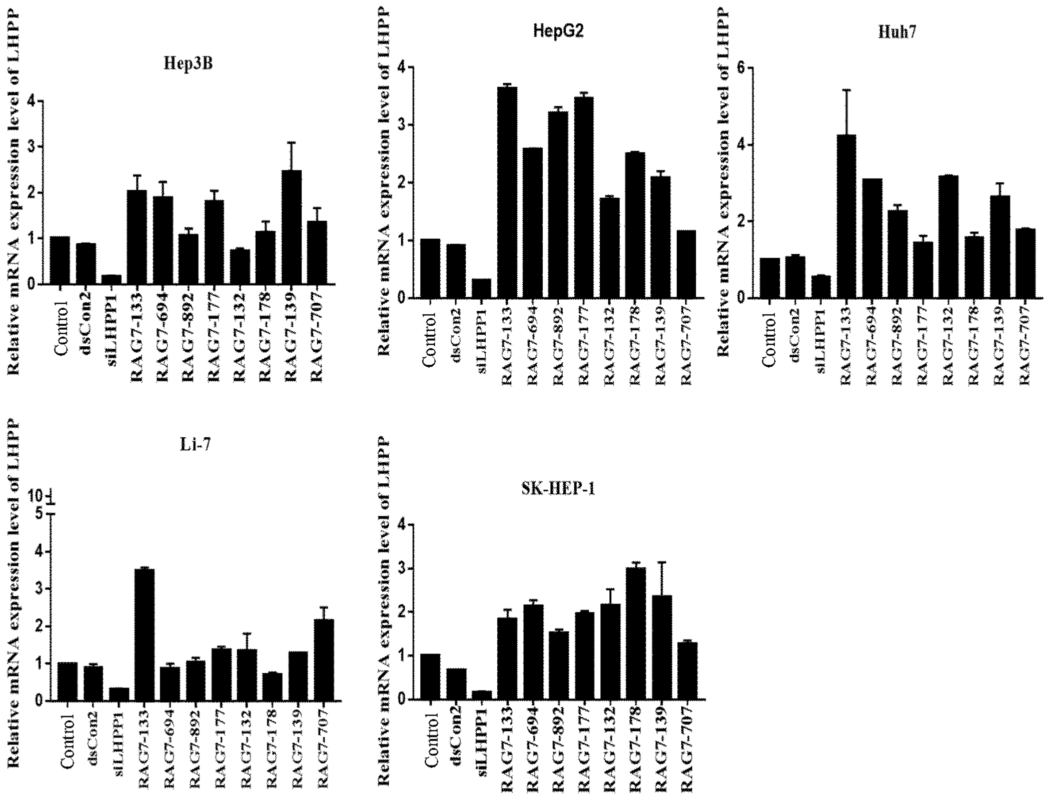
Hep3B
HepG2
Huh7
Li-7
SK-HEP-1
Relative mRNA expression level of LHPP
Control
dsCon2
siLHPP1
RAG7-133
RAG7-694
RAG7-892
RAG7-177
RAG7-132
RAG7-178
RAG7-139
RAG7-707

FIG. 6A

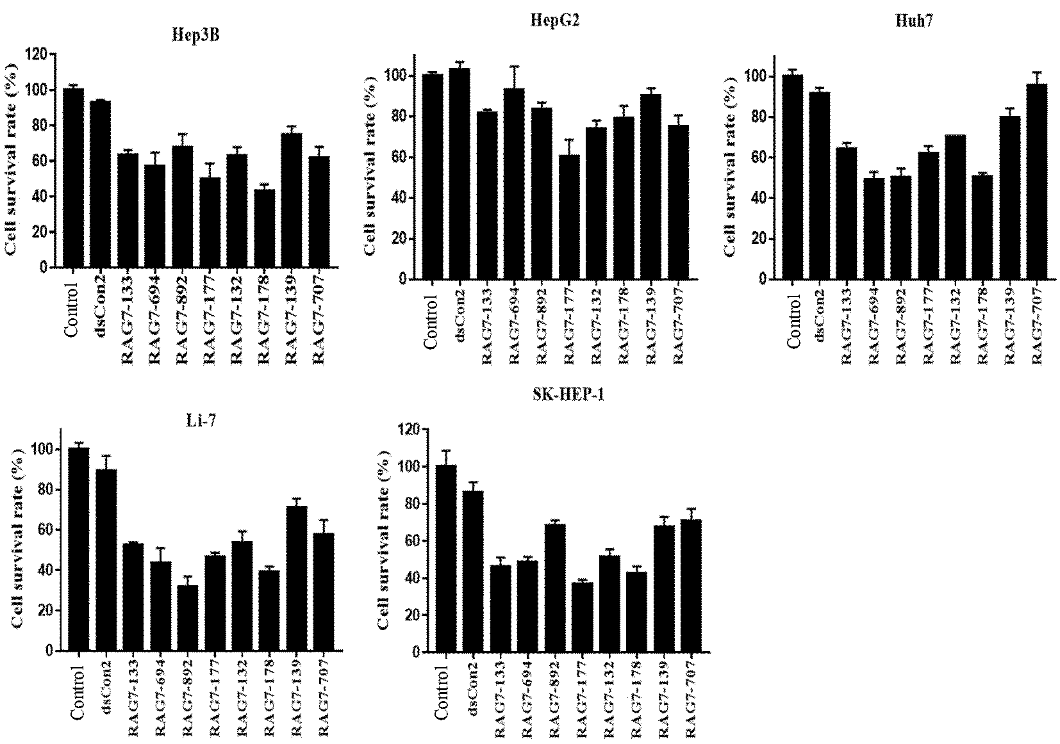
Hep3B
HepG2
Huh7
Li-7
SK-HEP-1
Cell survival rate (%)
Control
dsCon2
RAG7-133
RAG7-694
RAG7-892
RAG7-177
RAG7-132
RAG7-178
RAG7-139
RAG7-707

FIG. 6B

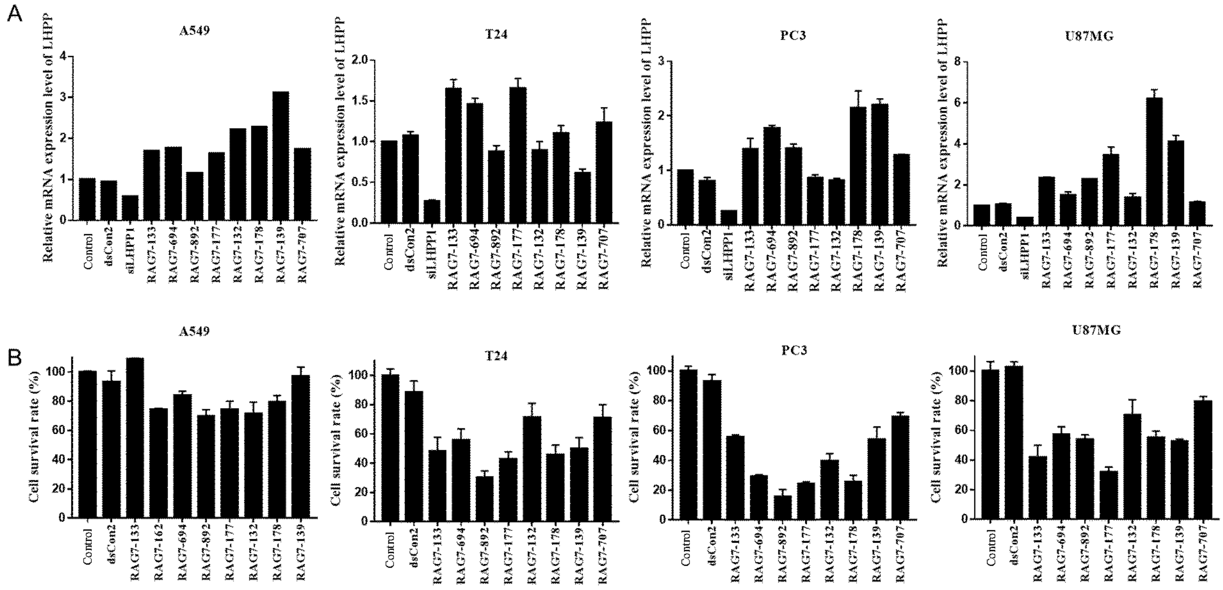


*FIG. 7*

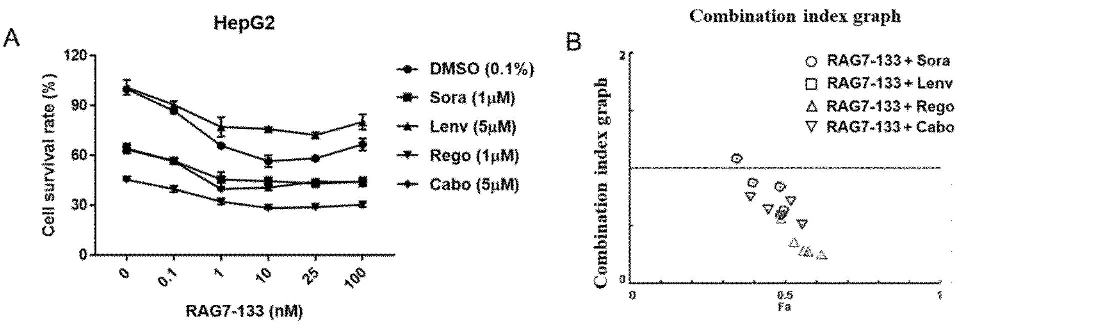


C

| Dose of RAG7-133 (nM) | Dose of Sora (μM) | Inhibition rate | Combination index (CI) |
|---|---|---|---|
| 1.0 | 1.0 | 0.348 | 1.08069 |
| 10.0 | 1.0 | 0.398 | 0.87355 |
| 25.0 | 1.0 | 0.489 | 0.59315 |
| 50.0 | 1.0 | 0.499 | 0.63490 |
| 100.0 | 1.0 | 0.485 | 0.83753 |

| Dose of RAG7-133 (nM) | Dose of Rego (μM) | Inhibition rate | Combination index (CI) |
|---|---|---|---|
| 1.0 | 1.0 | 0.489 | 0.55902 |
| 10.0 | 1.0 | 0.531 | 0.36064 |
| 25.0 | 1.0 | 0.561 | 0.28452 |
| 50.0 | 1.0 | 0.578 | 0.28063 |
| 100.0 | 1.0 | 0.62 | 0.25206 |

| Dose of RAG7-133 (nM) | Dose of Lenv (μM) | Inhibition rate | Combination index (CI) |
|---|---|---|---|
| 10.0 | 5.0 | 0.09 | 49076.7 |
| 25.0 | 5.0 | 0.124 | 323564. |
| 50.0 | 5.0 | 0.116 | 217192. |
| 100.0 | 5.0 | 0.232 | 1.744E7 |

| Dose of RAG7-133 (nM) | Dose of Cabo (μM) | Inhibition rate | Combination index (CI) |
|---|---|---|---|
| 1.0 | 5.0 | 0.388 | 0.74705 |
| 10.0 | 5.0 | 0.446 | 0.63984 |
| 25.0 | 5.0 | 0.488 | 0.59395 |
| 50.0 | 5.0 | 0.556 | 0.50930 |
| 100.0 | 5.0 | 0.52 | 0.71487 |

*FIG. 8*

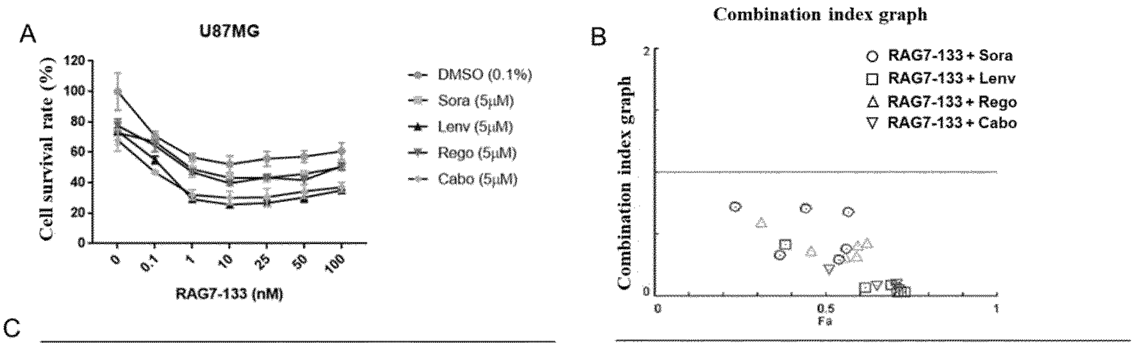


C

| Dose of RAG7-133 (nM) | Dose of Sora (μM) | Inhibition rate | Combination index (CI) |
|---|---|---|---|
| 0.1 | 5.0 | 0.239 | 0.72285 |
| 1.0 | 5.0 | 0.368 | 0.33328 |
| 10.0 | 5.0 | 0.445 | 0.70869 |
| 25.0 | 5.0 | 0.541 | 0.29077 |
| 50.0 | 5.0 | 0.563 | 0.38238 |
| 100.0 | 5.0 | 0.569 | 0.68099 |

| Dose of RAG7-133 (nM) | Dose of Rego (μM) | Inhibition rate | Combination index (CI) |
|---|---|---|---|
| 0.1 | 5.0 | 0.314 | 0.59778 |
| 1.0 | 5.0 | 0.459 | 0.36898 |
| 10.0 | 5.0 | 0.559 | 0.31532 |
| 25.0 | 5.0 | 0.592 | 0.31978 |
| 50.0 | 5.0 | 0.596 | 0.41005 |
| 100.0 | 5.0 | 0.623 | 0.42855 |

| Dose of RAG7-133 (nM) | Dose of Lenv (μM) | Inhibition rate | Combination index (CI) |
|---|---|---|---|
| 0.1 | 5.0 | 0.384 | 0.41790 |
| 1.0 | 5.0 | 0.618 | 0.06939 |
| 10.0 | 5.0 | 0.71 | 0.03461 |
| 25.0 | 5.0 | 0.732 | 0.03079 |
| 50.0 | 5.0 | 0.721 | 0.04252 |
| 100.0 | 5.0 | 0.691 | 0.09414 |

| Dose of RAG7-133 (nM) | Dose of Cabo (μM) | Inhibition rate | Combination index (CI) |
|---|---|---|---|
| 0.1 | 5.0 | 0.512 | 0.21035 |
| 1.0 | 5.0 | 0.649 | 0.08553 |
| 10.0 | 5.0 | 0.713 | 0.05595 |
| 25.0 | 5.0 | 0.707 | 0.06503 |
| 50.0 | 5.0 | 0.706 | 0.07600 |
| 100.0 | 5.0 | 0.707 | 0.09530 |

*FIG. 9*

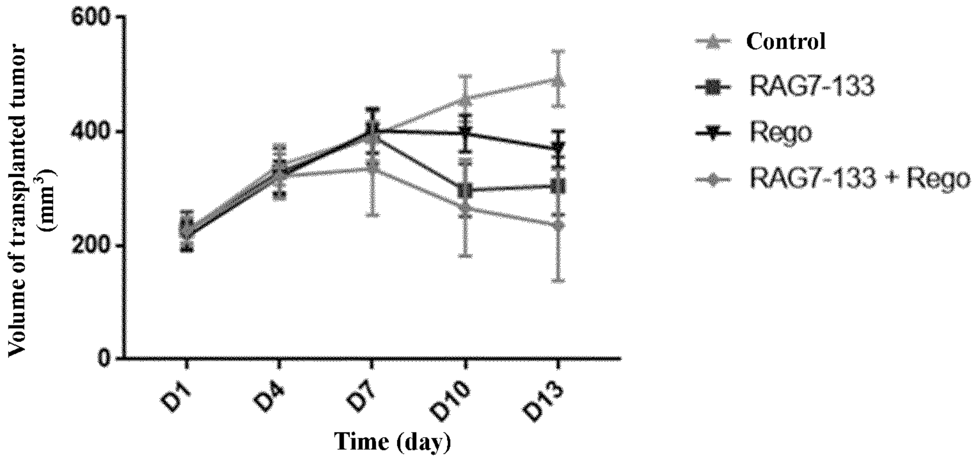


*FIG. 10*

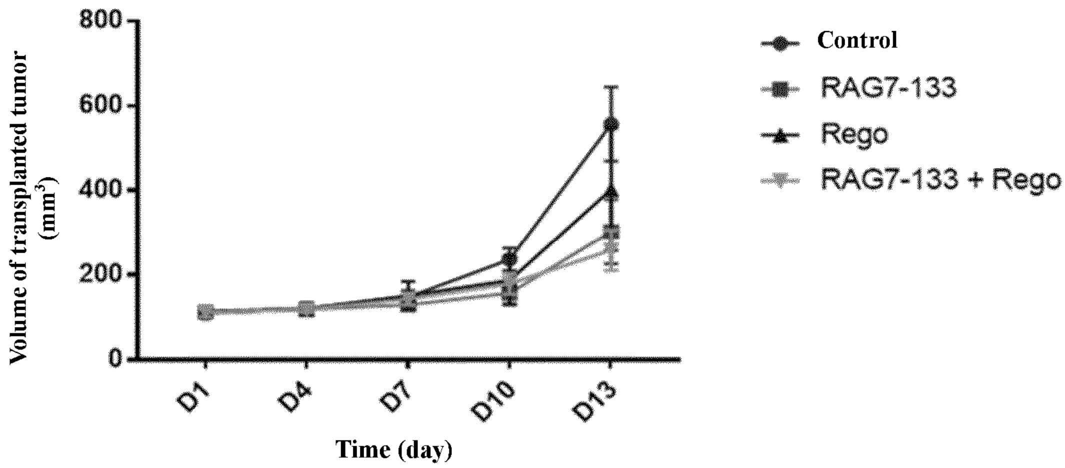
800
600
400
200
0
Volume of transplanted tumor ($mm^3$)
D1
D4
D7
D10
D13
Time (day)
Control
RAG7-133
Rego
RAG7-133 + Rego

*FIG. 11*

## INTERNATIONAL SEARCH REPORT

International application No.
**PCT/CN2019/092720**

**A. CLASSIFICATION OF SUBJECT MATTER**

C12N 15/113(2010.01)i； A61K 48/00(2006.01)i； A61P 35/00(2006.01)i

According to International Patent Classification (IPC) or to both national classification and IPC

**B. FIELDS SEARCHED**

Minimum documentation searched (classification system followed by classification symbols)

C12N; A61K; A61P

Documentation searched other than minimum documentation to the extent that such documents are included in the fields searched

Electronic data base consulted during the international search (name of data base and, where practicable, search terms used)

CNABS; HKABS; TWABS; DWPI; SIPOABS; CNTXT; WOTXT; EPTXT; USTXT; CNKI; 万方数据库; WANFANG; ISI WEB OF SCIENCE: 组氨酸磷酸酶, 磷酸赖氨酸磷酸组氨酸无机焦磷酸磷酸酶, 小激活RNA, Phospholysine phosphohistidine inorganic pyrophosphate phosphatase, histidine phosphatease, LHPP, saRNA, small activating RNA; 中国专利生物序列检索系统+GenBank+EMBL：关于SEQ ID NOs:1, 165, 329的序列检索。China Patents Biological Sequence Search System+GenBank+EMBL: search for SEQ ID NOs: 1, 165, and 329.

**C. DOCUMENTS CONSIDERED TO BE RELEVANT**

| Category* | Citation of document, with indication, where appropriate, of the relevant passages | Relevant to claim No. |
|---|---|---|
| A | US 2018305689 A1 (MINA THERAPEUTICS LTD.) 25 October 2018 (2018-10-25)<br>see entire document | 1-30 and 44-52 (all partially) |
| A | HINDUPUR, S. K. et al. "The protein histidine phosphatase LHPP is a tumour suppressor"<br>*Nature,* Vol. 7698, No. 555, 29 March 2018 (2018-03-29),<br>ISSN: 1476-4687,<br>pp. 678-682, see entire document | 1-30 and 44-52 (all partially) |
| A | ZHENG, Jiangli et al. "Down-regulation of LHPP in cervical cancer influences cell proliferation， metastasis and apoptosis by modulating AKT"<br>*Biochemical and Biophysical Research Communications,*<br>Vol. 503, No. 2, 02 August 2018 (2018-08-02),<br>ISSN: 0006-291X,<br>pp. 1108-1114, see entire document | 1-30 and 44-52 (all partially) |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 October 2019** | **04 November 2019** |
| Name and mailing address of the ISA/CN<br>**China National Intellectual Property Administration (ISA/CN)**<br>**No. 6, Xitucheng Road, Jimenqiao Haidian District, Beijing 100088**<br>**China** | Authorized officer |
| Facsimile No. **(86-10)62019451** | Telephone No. |

Form PCT/ISA/210 (second sheet) (January 2015)

**INTERNATIONAL SEARCH REPORT**

International application No.
**PCT/CN2019/092720**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **31-43**
because they relate to subject matter not required to be searched by this Authority, namely:

[1] The subject matter of claims 31-43 relates to a method of treating a living human or animal body, and therefore does not warrant an international search according to the criteria set out in PCT Rule 39.1(iv).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

[1] Invention 1: claims 1-30 and 44-52 (all partially) relate to active target sequences as shown in SEQ ID NO: 329, and first nucleic acid strand and second nucleic acid strand sequences such as small activating nucleic acid molecules as shown in SEQ ID NO: 1 and SEQ ID NO: 165;

[2] Invention 2: claims 1-30 and 44-52 (all partially) relate to active target sequences as shown in SEQ ID NO: 330, and first nucleic acid strand and second nucleic acid strand sequences such as small activating nucleic acid molecules as shown in SEQ ID NO: 2 and SEQ ID NO: 166;

[3] Invention 3: claims 1-30 and 44-52 (all partially) relate to active target sequences as shown in SEQ ID NO: 331, and first nucleic acid strand and second nucleic acid strand sequences such as small activating nucleic acid molecules as shown in SEQ ID NO: 3 and SEQ ID NO: 167;

[4] ……

[5] Invention 164: claims 1-30 and 44-52 (all partially) relate to active target sequences as shown in SEQ ID NO: 492, and first nucleic acid strand and second nucleic acid strand sequences such as small activating nucleic acid molecules as shown in SEQ ID NO: 164 and SEQ ID NO: 328;

[6] The small activating nucleic acid molecules involved in inventions 1-164 have different structures due to different active targets sequences for different, and their effects on changing the relative expression of LHPP mRNA are also different. Therefore, inventions 1-164 do not share a same or corresponding special technical feature, and are not so linked as to form a single general inventive concept; therefore, claims 1-164 do not comply with comply with the requirement of unity of invention as defined in PCT Rule 13.1 and 13.2.

Form PCT/ISA/210 (continuation of first sheet) (January 2015)

**INTERNATIONAL SEARCH REPORT**

International application No.
**PCT/CN2019/092720**

**Box No. III Observations where unity of invention is lacking (Continuation of item 3 of first sheet)**

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☑ No required additional search fees were timely paid by the applicant. Consequently, this international search report is restricted to the invention first mentioned in the claims; it is covered by claims Nos.: **Claims 1-30 and 44-52 (all partially) relate to active target sequences as shown in SEQ ID NO: 329, and first nucleic acid strand and second nucleic acid strand sequences such as small activating nucleic acid molecules as shown in SEQ ID NO: 1 and SEQ ID NO: 165.**

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet) (January 2015)

**INTERNATIONAL SEARCH REPORT**
**Information on patent family members**

International application No.
**PCT/CN2019/092720**

| Patent document cited in search report | Publication date (day/month/year) | Patent family member(s) | Publication date (day/month/year) |
|---|---|---|---|
| US 2018305689 A1 | 25 October 2018 | WO 2016170348 A2 | 27 October 2016 |
| | | WO 2016170348 A3 | 13 April 2017 |
| | | JP 2018512876 A | 24 May 2018 |
| | | WO 2016170348 A8 | 30 November 2017 |
| | | EP 3286318 A2 | 28 February 2018 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SAMBROOK et al.** Molecular Cloning: A Laboratory Manual. Cold Spring Harbor Laboratory Press, 1989
- **YOKOI F ; HIRAISHI H ; IZUHARA K.** Molecular cloning of a cDNA for the human phospholysine phosphohistidine inorganic pyrophosphate phosphatase. *J Biochem,* 2003, vol. 133, 607-14
- **NEFF CD ; ABKEVICH V ; PACKER JC ; CHEN Y ; POTTER J et al.** Evidence for HTR1A and LHPP as interacting genetic risk factors in major depression. *Mol Psychiatry,* 2009, vol. 14, 621-30
- **GOHLA A.** Do metabolic HAD phosphatases moonlight as protein phosphatases?. *Biochim Biophys Acta Mol Cell Res,* 2019, vol. 1866, 153-66
- **CONVERGE CONSORTIUM.** Sparse whole-genome sequencing identifies two loci for major depressive disorder. *Nature,* 2015, vol. 523, 588-91
- **KNOWLES EE ; KENT JW, JR. ; MCKAY DR ; SPROOTEN E ; MATHIAS SR et al.** Genome-wide linkage on chromosome 10q26 for a dimensional scale of major depression. *J Affect Disord,* 2016, vol. 191, 123-31
- **POLIMANTI R ; WANG Q ; MEDA SA ; PATEL KT ; PEARLSON GD et al.** The Interplay Between Risky Sexual Behaviors and Alcohol Dependence: Genome-Wide Association and Neuroimaging Support for LHPP as a Risk Gene. *Neuropsychopharmacology,* 2017, vol. 42, 598-605
- **CUI L ; GONG X ; TANG Y ; KONG L ; CHANG M et al.** Relationship between the LHPP Gene Polymorphism and Resting-State Brain Activity in Major Depressive Disorder. *Neural Plast,* 2016, vol. 2016, 9162590
- **LESSEUR C ; DIERGAARDE B ; OLSHAN AF ; WUNSCH-FILHO V ; NESS AR et al.** Genome-wide association analyses identify new susceptibility loci for oral cavity and pharyngeal cancer. *Nat Genet,* 2016, vol. 48, 1544-50
- **GUTIERREZ-CAMINO A ; MARTIN-GUERRERO I ; GARCIA-ORAD A.** Genetic susceptibility in childhood acute lymphoblastic leukemia. *Med Oncol,* 2017, vol. 34, 179
- **VIJAYAKRISHNAN J ; KUMAR R ; HENRION MY ; MOORMAN AV ; RACHAKONDA PS et al.** A genome-wide association study identifies risk loci for childhood acute lymphoblastic leukemia at 10q26.13 and 12q23.1. *Leukemia,* 2017, vol. 31, 573-9
- **HINDUPUR SK ; COLOMBI M ; FUHS SR ; MATTER MS ; GURI Y et al.** The protein histidine phosphatase LHPP is a tumour suppressor. *Nature,* 2018, vol. 555, 678-82
- **BRAY F ; FERLAY J ; SOERJOMATARAM I ; SIEGEL RL ; TORRE LA ; JEMAL A.** Global cancer statistics 2018: GLOBOCAN estimates of incidence and mortality worldwide for 36 cancers in 185 countries. *CA Cancer J Clin,* 2018, vol. 68, 394-424